# EUROPEAN PATENT SPECIFICATION

(11) **EP 0 823 428 B1**
(45) Date of publication and mention of the grant of the patent: **02.01.2002**
(21) Application number: 97305159.2
(22) Date of filing: 11.07.1997
(51) Int. Cl.: C07D 309/28, A61K 31/35

(54) **Neuraminic acid derivatives, their preparation and their medical use**
Neuraminsäuredervate, ihre Herstellung und medizinische Verwendung
Dérivés de l'acide neuraminique, leur préparation et utilisation médicale

(30) Priority: 22.07.1996 JP 19186296; 04.04.1997 JP 8688897
(43) Date of publication of application: 11.02.1998
(73) Proprietor: SANKYO COMPANY LIMITED, Chuo-ku, Tokyo 103-0023 (JP)
(72) Inventor: Honda, Takeshi, Shinagawa-ku, Tokyo 140 (JP); Kobayashi, Yoshiyuki, Shinagawa-ku, Tokyo 140 (JP); Masuda, Takeshi, Shinagawa-ku, Tokyo 140 (JP); Yamashita, Makoto, Shinagawa-ku, Tokyo 140 (JP); Arai, Masami, Shinagawa-ku, Tokyo 140 (JP)
(74) Representative: Gibson, Christian John Robert

(56) References cited:
- EP-A- 0 539 204
- WO-A-91/16320
- WO-A-95/16680
- WO-A-95/18800
- WO-A-95/20583
- WO-A-97/06157

## Description

The present invention relates to a series of new neuraminic acid derivatives which have excellent sialidase inhibitory activity and which are therefore useful for the treatment and prevention of influenza and other viral diseases where the replication of the virus is susceptible to sialidase inhibitors. The invention also provides methods and compositions using these compounds for the treatment or prevention of influenza and similar viral infections, as well as processes for the preparation of these compounds.

The compounds of the present invention have a 2-deoxy-2,3-didehydro-N-acylneuraminic acid structure. Sialic acid is N-acetylneuraminic acid.

The influenza virus, as well as a number of other types of virus, have sialidase on the surface of the virus particles. During the replication of such viruses, progeny virus germinates on the surface of an infected cell and then separates from the cell. The progeny virus is bonded to sialic acid on the surface of cells through haemagglutinin on the surface of the progeny virus. The progeny virus separates from the cells by decomposing the sialic acid with sialidase on the surface of the progeny virus, whereupon it then infects other cells.

Accordingly, removal of the progeny virus from the surface of infected cells can be blocked by inhibiting the activity of sialidase and thus the secondary infection can be prevented. It is, therefore, considered that substances having the ability to inhibit the action of sialidase will be useful for the treatment or prevention of influenza.

A number of compounds having a sialidase inhibitory activity and a 2-deoxy-2,3-didehydro-N-acylneuraminic acid backbone are known from WO91/16320 [equivalent to Japanese Patent Publication (Kokoku) No. Hei 5-507068]. Other such compounds are known from WO92/26933, WO95/20583, WO95/18800, WO95/16680 and EP-A-539204.

We have now discovered a series of new compounds having a 2-deoxy-2,3-didehydro-N-acylneuraminic acid backbone which have excellent sialidase inhibitory activity, which is significantly greater than that of the prior art compounds referred to above, and which can thus be used for the treatment and prevention of influenza and other diseases caused by sialidase-bearing viruses.

The compounds of the present invention are those compounds of formula (I): 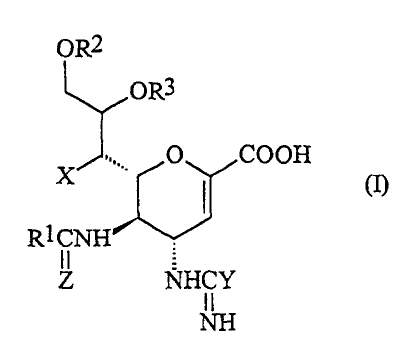 in which:
R¹ represents an alkyl group having from 1 to 4 carbon atoms;
R² and R³ are the same as or different from each other and each represents a hydrogen atom or an aliphatic acyl group having from 2 to 25 carbon atoms;
X represents a halogen atom or an alkoxy group having from 1 to 4 carbon atoms;
Y represents a group of formula R^{b}R^{c}N- or R^{b}R^{c}N-O-, where R^{b} and R^{c} are the same as or different from each other and each represents a hydrogen atom or an alkyl group having from 1 to 4 carbon atoms; and
Z represents an oxygen atom or a sulphur atom;
and pharmaceutically acceptable salts and esters thereof.

The invention also provides a pharmaceutical composition for the treatment or prevention of infections in a mammal, which may be human, caused by sialidase-bearing viruses, such as viruses of the influenza family, which composition comprises a sialidase inhibitory compound in admixture with a pharmaceutically acceptable carrier or diluent, in which the sialidase inhibitory compound is at least one compound of formula (I) or a pharmaceutically acceptable salt or ester thereof.

The invention still further provides the use of at least one compound of formula (I) or a pharmaceutically acceptable salt or ester thereof for the manufacture of a medicament for treating or preventing an infection in a mammal, which may be human, caused by a sialidase-bearing virus, such as a virus of the influenza family.

The invention also provides processes for the preparation of the compounds of the present invention which processes are described in greater detail hereafter.

The compounds of the present invention are named on the basis of saccharide terminology, in which the main positions are numbered as shown in the following formula: 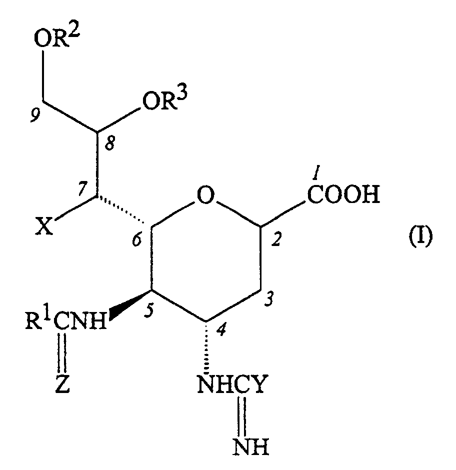 The compounds are named as derivatives of the unsaturated sugar non-2-enopyranosoic acid, of formula: 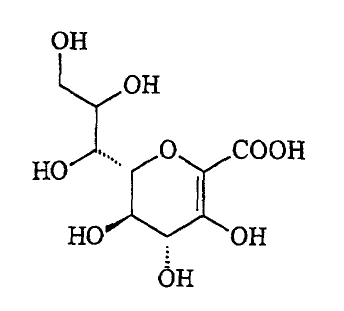 The configuations of the carbon atoms at the 4 to 7 positions are D-galacto, while that of the carbon atom at the 8 position is D-glycero, as can be seen from the following partial formula:

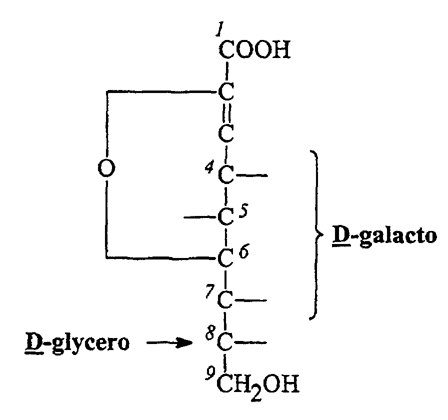

In the compounds of the present invention, where R¹ represents an alkyl group, this may be a straight or branched chain group having from 1 to 4 carbon atoms, and examples of such groups include the methyl, ethyl, propyl, isopropyl, butyl, isobutyl, sec-butyl and t-butyl groups, of which we prefer the methyl group.

More preferably R¹ represents a methyl group.

Where R² or R³ represents an aliphatic carboxylic acyl group having from 2 to 25 carbon atoms, this may be a straight or branched chain group, and is preferably an alkanoyl (alkylcarbonyl) group having from 2 to 25 carbon atoms. Specific examples of such groups include the acetyl, propionyl, butyryl, isobutyryl, pivaloyl, valeryl, isovaleryl, octanoyl, nonylcarbonyl, decylcarbonyl, 3-methylnonylcarbonyl, 8-methylnonylcarbonyl, 3-ethyloctylcarbonyl, 3,7-dimethyloctylcarbonyl, undecylcarbonyl, dodecylcarbonyl, tridecylcarbonyl, tetradecylcarbonyl, pentadecylcarbonyl, hexadecylcarbonyl, 1-methylpentadecylcarbonyl, 14-methylpentadecylcarbonyl, 13, 13-dimethyltetradecylcarbonyl, heptadecylcarbonyl, 15-methylhexadecylcarbonyl, octadecylcarbonyl, 1-methylheptadecylcarbonyl, nonadecylcarbonyl, icosylcarbonyl and tricosylcarbonyl groups. Of these, we prefer those alkylcarbonyl group having from 6 to 25 carbon atoms, more preferably those alkylcarbonyl groups having from 8 to 16 carbon atoms [and particularly the octanoyl, nonylcarbonyl, undecylcarbonyl (i.e. dodecanoyl), tridecylcarbonyl (i.e. myristoyl) and pentadecylcarbonyl (i.e. palmitoyl) groups].

R² preferably represents a hydrogen atom or an aliphatic carboxylic acyl group having from 6 to 25 carbon atoms, more preferably an aliphatic carboxylic acyl group having from 8 to 16 carbon atoms, particularly an octanoyl, nonylcarbonyl, undecylcarbonyl, tridecylcarbonyl or pentadecylcarbonyl group.

R³ preferably represents a hydrogen atom or an aliphatic carboxylic acyl group having from 6 to 25 carbon atoms, more preferably a hydrogen atom or an aliphatic carboxylic acyl group having from 8 to 16 carbon atoms, particularly an octanoyl, nonylcarbonyl, undecylcarbonyl, tridecylcarbonyl or pentadecylcarbonyl group.

Still more preferably, R² represents an aliphatic acyl group having from 8 to 16 carbon atoms (particularly an octanoyl, nonylcarbonyl, undecylcarbonyl, tridecylcarbonyl or pentadecylcarbonyl group), and R³ represents a hydrogen atom.

Where X represents a halogen atom, this may be a fluorine, chlorine, bromine or iodine atom, preferably a fluorine, chlorine or bromine atom, and more preferably a fluorine atom;

Where X represents an alkoxy group having from 1 to 4 carbon atoms, this may be a straight or branched chain group, and examples include the methoxy, ethoxy, propoxy, isopropoxy, butoxy, isobutoxy, sec-butoxy and t-butoxy groups, of which we prefer the methoxy and ethoxy groups.

X preferably represents a fluorine atom, or a methoxy or ethoxy group.

Where Y represents a group of formula R^{b}R^{c}N- or R^{b}R^{c}N-O- and R^{b} and/or R^{c} represents an alkyl group having from 1 to 4 carbon atoms, this may be a straight or branched chain group, and examples include the methyl, ethyl, propyl, isopropyl, butyl, isobutyl, sec-butyl and t-butyl groups, of which we prefer the methyl group. However, we most prefer that at least one, and preferably both, of R^{b} and R^{c} should represent a hydrogen atom. Thus, the preferred group represented by R^{b}R^{c}N-O- is a group of formula NH₂-O-, and the preferred group represented by R^{b}R^{c}N- is NH₂-, i.e. an amino group.

Y preferably represents a group of formula NH₂ or NH₂-O-, more preferably an amino group of formula NH₂.

Z preferably represents an oxygen atom.

The compounds of the present invention contain a carboxy group, and can, therefore, form salts with cations. There is no particular restriction on the nature of these salts, provided that, where the compounds are to be used medically, the salts should be pharmaceutically acceptable, that is they should not be more toxic (or unacceptably more toxic) than the free acid, nor should they be less active (or unacceptably less active) than the free acid. When the compound is to be used for other purposes, for example as intermediates in the preparation of other, and possibly more active compounds, even this restriction does not apply. Examples of such salts include: alkali metal salts, such as the sodium salt, potassium and lithium salts; alkaline earth metal salts, such as the calcium and magnesium salts; other metal salts, such as the aluminium, iron, zinc, copper, nickel and cobalt salts; other inorganic salts, such as the ammonium salt; amine salts, such as the t-octylamine, dibenzylamine, morpholine, glucosamine, phenylglycine alkyl ester, ethylenediamine, methylglucamine, guanidine, diethylamine, triethylamine, dicyclohexylamine, N,N'-dibenzylethylenediamine, chloroprocaine, procaine, diethanolamine, benzyl-phenethylamine, piperazine, tetramethylammonium and tris(hydroxymethyl)aminomethane salts.

Furthermore, when the compound of the present invention contains a guadinino group, it can be also converted to a salt. Such salts, likewise, are not particularly restricted, except that, where they are for medical use, they should be pharmaceutically acceptable. Examples of such salts include: hydrohalides, such as the hydrofluoride, hydrochloride, hydrobromide or hydroiodide; other inorganic acid salts, such as the nitrate, perchlorate, sulphate or phosphate; lower alkanesulphonates, such as the methanesulphonate, trifluoromethanesulphonate or ethanesulphonate; arylsulphonates, such as the benzenesulphonate or p-toluenesulphonate; organic acid salts, especially carboxylic acid salts, such as the acetate, trifluoroacetate, malate, fumarate, succinate, citrate, tartarate, oxalate or maleate; and amino acid salts, such as the glycine, lysine, arginine, ornithine, glutamic acid salt or aspartic acid salt. Of these, we prefer the alkali metal salts, such as the sodium, potassium and lithium salts, organic acid salts, such as the acetate and trifluoroacetate and inorganic acid salts, such as the hydrochloride and sulphate.

Since the compounds of the present invention contain a carboxy group, they can form esters. There is no particular restriction on the nature of these esters, provided that, where the compounds are to be used medically, the esters should be pharmaceutically acceptable, that is they should not be more toxic (or unacceptably more toxic) than the free acid, nor should they be less active (or unacceptably less active) than the free acid. When the compound is to be used for other purposes, for example as intermediates in the preparation of other, and possibly more active compounds, even this restriction does not apply. Examples of groups which can form such esters include:
alkyl groups, preferably having from 1 to 30, more preferably from 1 to 25 carbon atoms, such as the methyl, ethyl, propyl, isopropyl, butyl, isobutyl, sec-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutylheptyl, 1-methylhexyl, 2-methylhexyl, 3-methylhexyl, 4-methylhexyl, 5-methylhexyl, 1-propylbutyl, 4,4-dimethylpentyl, octyl, 1-methylheptyl, 2-methylheptyl, 3-methylheptyl, 4-methylheptyl, 5-methylheptyl, 6-methylheptyl, 1-propylpentyl, 2-ethylhexyl, 5,5-dimethylhexyl, nonyl, 3-methyloctyl, 4-methyloctyl, 5-methyloctyl, 6-methyloctyl, 1-propylhexyl, 2-ethylheptyl, 6,6-dimethylheptyl, decyl, 1-methylnonyl, 3-methylnonyl, 8-methylnonyl, 3-ethyloctyl, 3,7-dimethyloctyl, 7,7-dimethyloctyl, undecyl, 4,8-dimethylnonyl, dodecyl, tridecyl, tetradecyl, pentadecyl, 3,7,11-trimethyldodecyl, hexadecyl, 4,8,12-trimethyltridecyl, 1-methylpentadecyl, 14-methylpentadecyl, 13,13-dimethyltetradecyl, heptadecyl, 15-methylhexadecyl, octadecyl, 1-methylheptadecyl, nonadecyl, icosyl, 3.7,11,15-tetramethylhexadecyl, henicosyl and docosyl groups;
alkenyl groups, preferably having from 2 to 10, and more preferably from 2 to 8 carbon atoms, such as the ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl and 5-hexenyl groups;
alkynyl groups, preferably having from 2 to 10, and more preferably from 2 to 8 carbon atoms, such as the ethynyl, 2-propynyl, 1-methyl-2-propynyl, 2-methyl-2-propynyl, 2-ethyl-2-propynyl, 2-butynyl, 1-methyl-2-butynyl, 2-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 2-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl and 5-hexynyl groups;
haloalkyl groups, preferably having from 1 to 6, more preferably from 1 to 4, carbon atoms, such as the trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl, 3-chloropropyl, 4-fluorobutyl, 6-iodohexyl and 2,2-dibromoethyl groups;
hydroxyalkyl groups, preferably having from 1 to 6, more preferably from 1 to 4, carbon atoms, such as the 2-hydroxyethyl, 2,3-dihydroxypropyl, 3-hydroxypropyl, 3,4-dihydroxybutyl and 4-hydroxybutyl groups;
aliphatic acyl-substituted alkyl groups, in which the alkyl part preferably has from 1 to 6 carbon atoms, and may be any such group of those listed above, and the aliphatic acyl group, such as those exemplified above in relation to R², and especially those having from 2 to 5 carbon atoms, such as the acetylmethyl group;
aralkyl groups in which an alkyl group having from 1 to 6 carbon atoms is substituted with from 1 to 3 unsubstituted carbocyclic aryl groups, such as the benzyl, phenethyl, 3-phenylpropyl, α-naphthylmethyl, β-naphthylmethyl, diphenylmethyl, triphenylmethyl, 6-phenylhexyl, α-naphthyldiphenylmethyl and 9-anthrylmethyl groups;
aralkyl groups in which an alkyl group having from 1 to 6 carbon atoms is substituted with from 1 to 3 substituted carbocyclic aryl groups, the subsituents being, for example, the alkyl, alkoxy, nitro, halogen, cyano oralkoxycarbonyl groups, such as the 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, 4-cyanobenzyldiphenylmethyl, bis(2-nitrophenyl)methyl, piperonyl and 4-methoxycarbonylbenzyl groups;
silyl groups, including trialkylsilyl, dialkylarylsilyl and alkyldiarylsilyl groups (the alkyl groups preferably having from 1 to 6 carbon atoms), such as the trimethylsilyl, triethylsilyl, isopropyldimethylsilyl, t-butyldimethylsilyl, methyldiisopropylsilyl, methyldi-t-butylsilyl, triisopropylsilyl, methyldiphenylsilyl, isopropyldiphenylsilyl, butyldiphenylsilyl and phenyldiisopropylsilyl groups;
alkoxyalkyl groups in which the alkoxy and alkyl parts both have from 1 to 6, preferably from 1 to 4, carbon atoms, such as the methoxymethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, propoxymethyl, isopropoxymethyl, butoxymethyl and t-butoxymethyl groups;
alkoxyalkoxyalkyl groups in which each of the alkoxy parts and the alkyl part all have from 1 to 6, preferably from 1 to 4, carbon atoms, such as the 2-methoxyethoxymethyl group;
aryloxyalkyl groups in which the alkyl part has from 1 to 6, preferably from 1 to 4, carbon atoms, such as the phenoxymethyl group;
halogenated alkoxyalkyl groups in which the alkoxy and alkyl parts both have from 1 to 6, preferably from 1 to 4, carbon atoms, such as the 2,2,2-trichloroethoxymethyl and bis(2-chloroethoxy)methyl groups;
alkoxycarbonylalkyl groups in which the alkoxy and alkyl parts both have from 1 to 6, preferably from 1 to 4, carbon atoms, such as the methoxycarbonylmethyl group;
cyanoalkyl groups in which the alkyl part has from 1 to 6, preferably from 1 to 4, carbon atoms, such as the cyanomethyl and 2-cyanoethyl groups;
alkylthiomethyl groups in which the alkyl part has from 1 to 6, preferably from 1 to 4, carbon atoms, such as the methylthiomethyl and ethylthiomethyl groups;
arylthiomethyl groups, such as the phenylthiomethyl and naphthylthiomethyl groups;
alkylsulphonylalkyl groups in which each of the alkyl parts has from 1 to 6, preferably from 1 to 4, carbon atoms and which may be substituted with one or more halogen atoms, such as the 2-methanesulphonylethyl and 2-trifluoromethanesulphonylethyl groups;
arylsulphonylalkyl groups in which the alkyl part has from 1 to 6, preferably from 1 to 4, carbon atoms, such as the 2-benzenesulphonylethyl and 2-toluenesulphonylethyl groups;
acyloxyalkyl groups, including
   aliphatic carboxylic acyloxyalkyl groups in which the alkyl part has from 1 to 6, preferably from 1 to 4, carbon atoms and the aliphatic acyl part has from 1 to 8, preferably from 2 to 5, carbon atoms, such as the formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl groups;
   cycloalkylcarbonyloxyalkyl groups in which the alkyl part has from 1 to 6, preferably from 1 to 4, carbon atoms and the cycloalkyl part has from 3 to 8, preferably 5 or 6, carbon atoms, such as the cyclopentanecarbonyloxymethyl, cyclohexanecarbonyloxymethyl, 1-cyclopentanecarbonyloxyethyl, 1-cyclohexanecarbonyloxyethyl, 1-cyclopentanecarbonyloxypropyl, 1-cyclohexanecarbonyloxypropyl, 1-cyclopentanecarbonyloxybutyl and 1-cyclohexanecarbonyloxybutyl groups;
   aromatic acyloxyalkyl groups in which the alkyl part has from 1 to 6, preferably from 1 to 4, carbon atoms, such as the benzoyloxymethyl group;
(alkoxycarbonyloxy)alkyl groups in which the alkoxy and alkyl parts both have from 1 to 6, preferably from 1 to 4, carbon atoms, such as the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, 1-(methoxycarbonyloxy)ethyl, 1-(ethoxycarbonyloxy)ethyl, 1-propoxycarbonyloxyethyl, 1-(isopropoxycarbonyloxy)ethyl, 1-butoxycarbonyloxyethyl, 1-isobutoxycarbonyloxyethyl, 1-(tert-butoxycarbonyloxy)ethyl, 1-pentyloxycarbonyloxyethyl, 1-hexyloxycarbonyloxyethyl, 1-(ethoxycarbonyloxy)propyl, 2-methoxycarbonyloxyethyl, 2-ethoxycarbonyloxyethyl, 2-propoxycarbonyloxyethyl, 2-isopropoxycarbonyloxyethyl, 2-butoxycarbonyloxyethyl, 2-isobutoxycarbonyloxyethyl, 2-pentyloxycarbonyloxyethyl, 2-hexyloxycarbonyloxyethyl, 1-methoxycarbonyloxypropyl, 1-ethoxycarbonyloxypropyl, 1-propoxycarbonyloxypropyl, 1-isopropoxycarbonyloxypropyl, 1-butoxycarbonyloxypropyl, 1-isobutoxycarbonyloxypropyl, 1-pentyloxycarbonyloxypropyl, 1-hexyloxycarbonyloxypropyl, 1-methoxycarbonyloxybutyl, 1-ethoxycarbonyloxybutyl, 1-propoxycarbonyloxybutyl, 1-isopropoxycarbonyloxybutyl, 1-butoxycarbonyloxybutyl, 1-isobutoxycarbonyloxybutyl, 1-methoxycarbonyloxypentyl, 1-ethoxycarbonyloxypentyl, 1-methoxycarbonyloxyhexyl and 1-ethoxycarbonyloxyhexyl groups;
(cycloalkyloxycarbonyloxy)alkyl and (cycloalkyloxycarbonyloxy)(cycloalkyl)alkyl groups in which the alkyl part has from 1 to 6, preferably from 1 to 4, carbon atoms and the cycloalkyl part has from 3 to 8, preferably 5 or 6, carbon atoms, such as the cyclohexyloxycarbonyloxymethyl, cyclohexyloxycarbonyloxy(cyclohexyl)methyl, 1-cyclopentyloxycarbonyloxyethyl, 1-cyclopentyloxycarbonyloxypropyl, 1-cyclohexyloxycarbonyloxypropyl, 1-cyclopentyloxycarbonyloxybutyl, 1-cyclohexyloxycarbonyloxybutyl and 1-(cyclohexyloxycarbonyloxy)ethyl groups;
carbonyloxyalkyl groups in which the alkyl part has from 1 to 6, preferably from 1 to 4, carbon atoms, including oxodioxolenylmethyl groups, such as the (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl groups;
phthalidyl and substituted phthalidyl groups, such as the phthalidyl, dimethylphthalidyl and dimethoxyphthalidyl groups;
aryl groups, preferably having from 6 to 14 carbon atoms in one or more carbocyclic rings, such as the phenyl, naphthyl and indanyl groups;
carboxyalkyl groups in which the alkyl part has from 1 to 6, preferably from 1 to 4, carbon atoms, such as the carboxymethyl group; and
amide forming residues of amino acid.

Of the ester groups listed above, the following are cleavable by a biological method such as hydrolysis in a living body;. that is the esters produce free acids or salts by hydrolysis or a similar reaction in a human body, specifically:
alkyl groups, alkoxyalkyl groups, alkoxyalkoxyalkyl groups, aryloxyalkyl groups, halogenated alkoxyalkyl groups, alkoxycarbonylalkyl groups, cyanoalkyl groups, alkylthiomethyl groups, alkylsulphonylalkyl groups, arylsulphonylalkyl groups, acyloxyalkyl groups, (alkoxycarbonyloxy)alkyl groups, (cycloalkyloxycarbonyloxy)alkyl groups, (cycloalkyloxycarbonyloxy)(cycloalkyl)alkyl groups, carbonyloxyalkyl groups, phthalidyl and substituted phthalidyl groups, aryl groups, carboxyalkyl groups, and amide forming residues of amino acid.

Of these, we prefer the straight or branched alkyl groups having from 6 to 25 carbon atoms, more preferably an alkyl group having 16 to 25 carbon atoms.

The compounds of the present invention, when they are allowed to stand in the atmosphere, may absorb some moisture, and they may, as a result, be associated with adsorption water or they may be converted to a corresponding hydrate. Such hydrates also form part of the present invention.

Preferred classes of compounds of the present invention are those compounds of formula (I) and pharmaceutically acceptable salts and esters thereof in which:
(A) R¹ represents a methyl group.
(B) R² represents a hydrogen atom or an aliphatic carboxylic acyl group having from 6 to 25 carbon atoms.
(C) R³ represents a hydrogen atom or an aliphatic carboxylic acyl group having from 6 to 25 carbon atoms.
(D) Y represents an amino group or a group of formula R^{b}R^{c}N-O-, where R^{b} and R^{c} are as defined above.
Of the above compounds, we particularly prefer those compounds of formula (I) and pharmaceutically acceptable salts and esters thereof in which R¹ is as defined in (A) above, R² is as defined in (B) above, R³ is as defined in (C) above, and Y is as defined in (D) above, especially those in which R¹ is as defined in (A) above, R² is as defined in (B) above, R³ is as defined in (C) above, Y is as defined in (D) above, and Z represents an oxygen atom.

More preferred classes of compounds of the present invention are those compounds of formula (I) and pharmaceutically acceptable salts and esters thereof in which:
(E) R¹ represents a methyl group;
(F) R² represents a hydrogen atom or an aliphatic carboxylic acyl group having from 8 to 16 carbon atoms.
(G) R³ represents a hydrogen atom or an aliphatic carboxylic acyl group having from 8 to 16 carbon atoms.
(H) X represents a fluorine atom, a methoxy group or an ethoxy group.
(I) Y represents an amino group or an aminooxy group.
(J) Z represents an oxygen atom.

Of the above compounds, we particularly prefer those compounds of formula (I) and pharmaceutically acceptable salts and esters thereof in which R¹ is as defined in (E) above, R² is as defined in (F) above, R³ is as defined in (G) above, X is as defined in (H) above, Y is as defined in (I) above, and Z is as defined in (J) above.

A still more preferred class of compounds of the present invention are those compounds of formula (I) and pharmaceutically acceptable salts and esters thereof in which:
(K) R² represents a hydrogen atom or an aliphatic carboxylic acyl group having from 8 to 16 carbon atoms and R³ represents a hydrogen atom.
Of the above compounds, we particularly prefer those compounds of formula (I) and pharmaceutically acceptable salts and esters thereof in which R¹ is as defined in (E) above, R² and R³ are as defined in (K) above, X is as defined in (H) above, Y is as defined in (I) above, and Z is as defined in (J) above.

Further more preferred classes of compounds of the present invention are those compounds of formula (I) and pharmaceutically acceptable salts and esters thereof in which:
(L) R¹ represents a methyl group.
(M) R² represents an octanoyl, decanoyl, dodecanoyl, myristoyl or palmitoyl group.
(N) R³ represents a hydrogen atom, or an octanoyl, decanoyl, dodecanoyl, myristoyl or palmitoyl group.
(O) Y represents an amino group.
Of the above compounds, we particularly prefer those compounds of formula (I) and pharmaceutically acceptable salts and esters thereof in which R¹ is as defined in (L) above, R² is as defined in (M) above, R³ is as defined in (N) above, X is as defined in (H) above, Y is as defined in (O) above, and Z is as defined in (J) above.

Further more preferred classes of compounds of the present invention are those compounds of formula (I) and pharmaceutically acceptable salts and esters thereof in which:
(P) R¹ represents a methyl group.
(Q) R² represents an octanoyl, decanoyl, dodecanoyl, myristoyl or palmitoyl group and R³ represents a hydrogen atom.
Of the above compounds, we particularly prefer those compounds of formula (I) and pharmaceutically acceptable salts and esters thereof in which R¹ is as defined in (P) above, R² and R³ are as defined in (Q) above, X is as defined in (H) above, Y is as defined in (O) above, and Z is as defined in (J) above.

Specific compounds of the present invention are exemplified below by the following formula (I'), in which the substituent groups are as defined in the following Tables 1 and 2. Any individual compound may be defined by taking the combination of definitions of R¹, X, Y and Z in any row of Table 1 and combining them with the combination of definitions of R², R³ and W in any row of Table 2. The compound may then be identified by a number in the form α-β, where α is the number of the respective row of Table 1 and β is the number of the respective row of Table 2. Thus, for example, the compound of formula (I"), given below, which is a compound of formula (I') in which R¹ represents a methyl group, X represents a fluorine atom, Y represents an amino group, Z represents an oxygen atom (row 1 of Table 1), R² represents a hydrogen atom, R³ represents a hydrogen atom and W represents an undecyl group (row 4 of Table 2) is Compound No. 1-4.

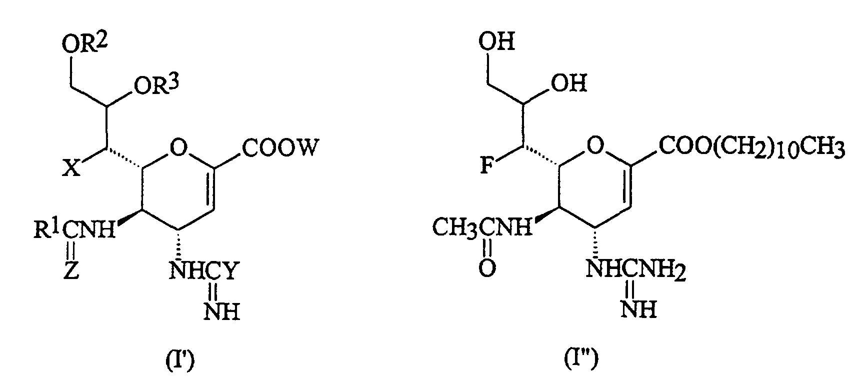

In the Tables, the following abbreviations are used:
- Bu: butyl
- iBu: isobutyl
- Et: ethyl
- Me: methyl
- Pr: propyl
- iPr: isopropyl

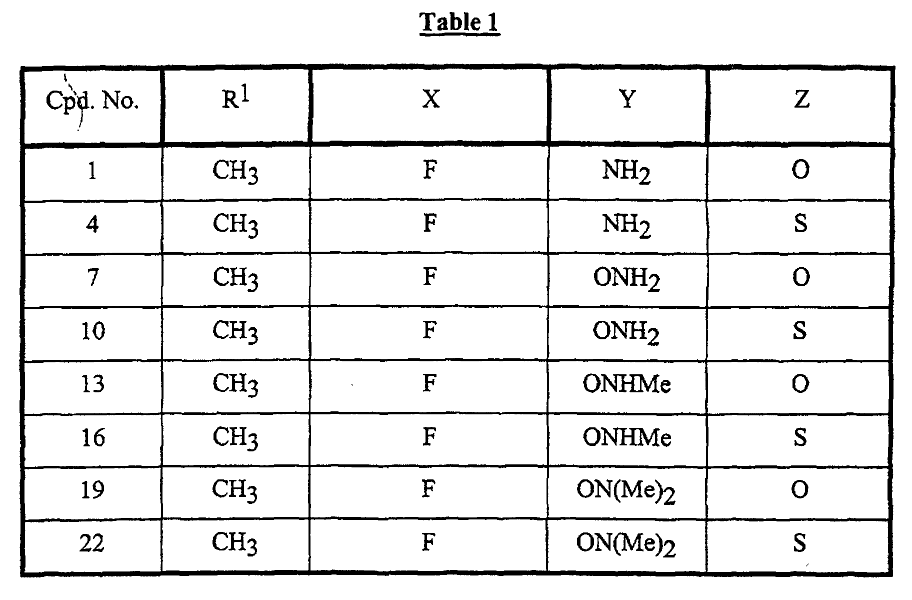

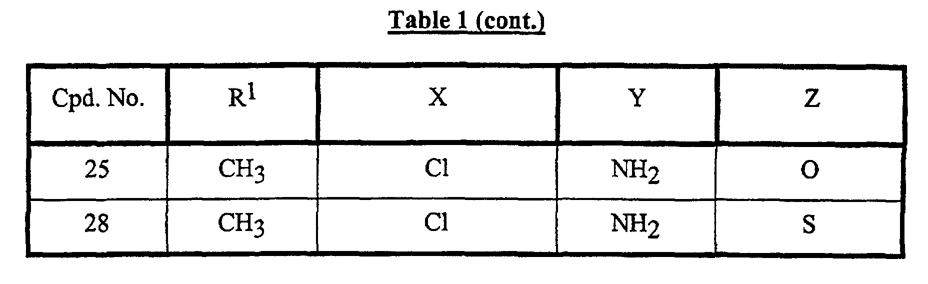

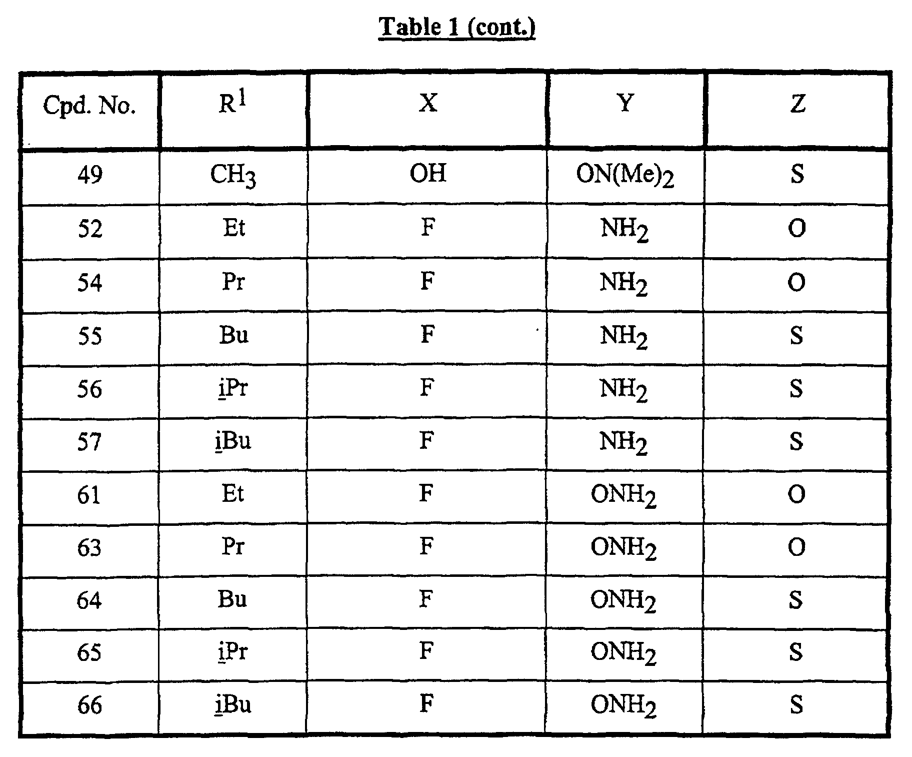

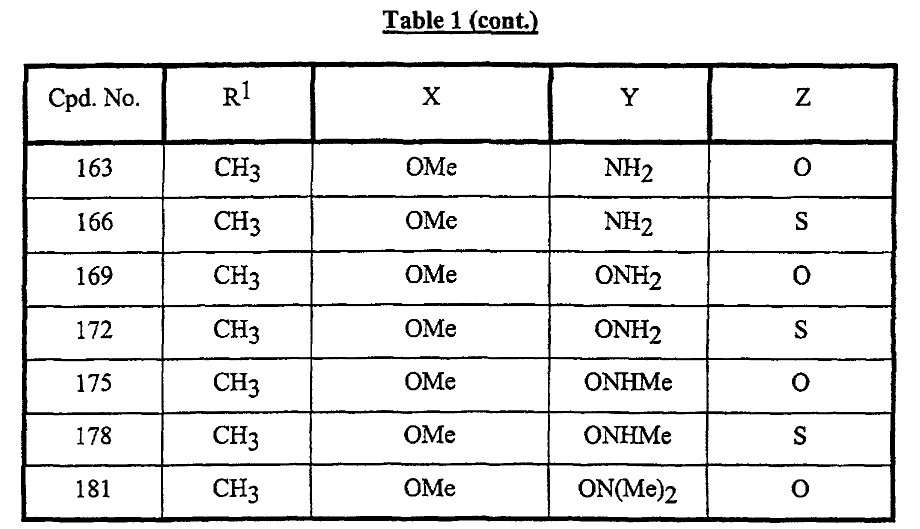

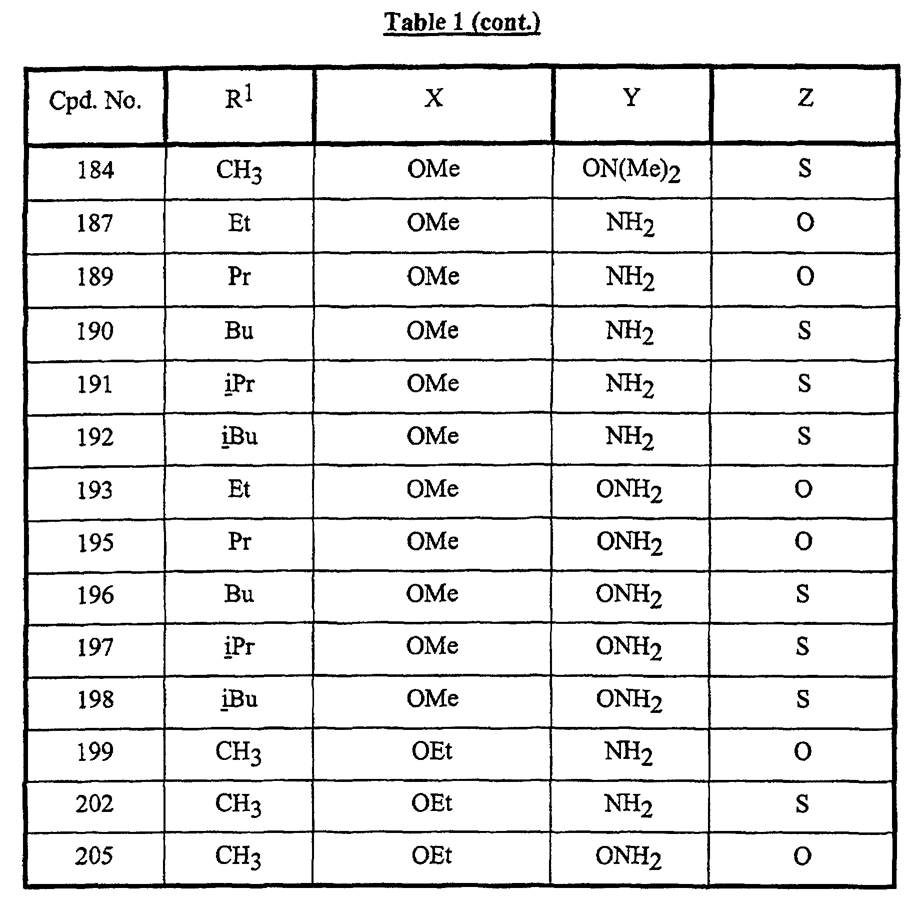

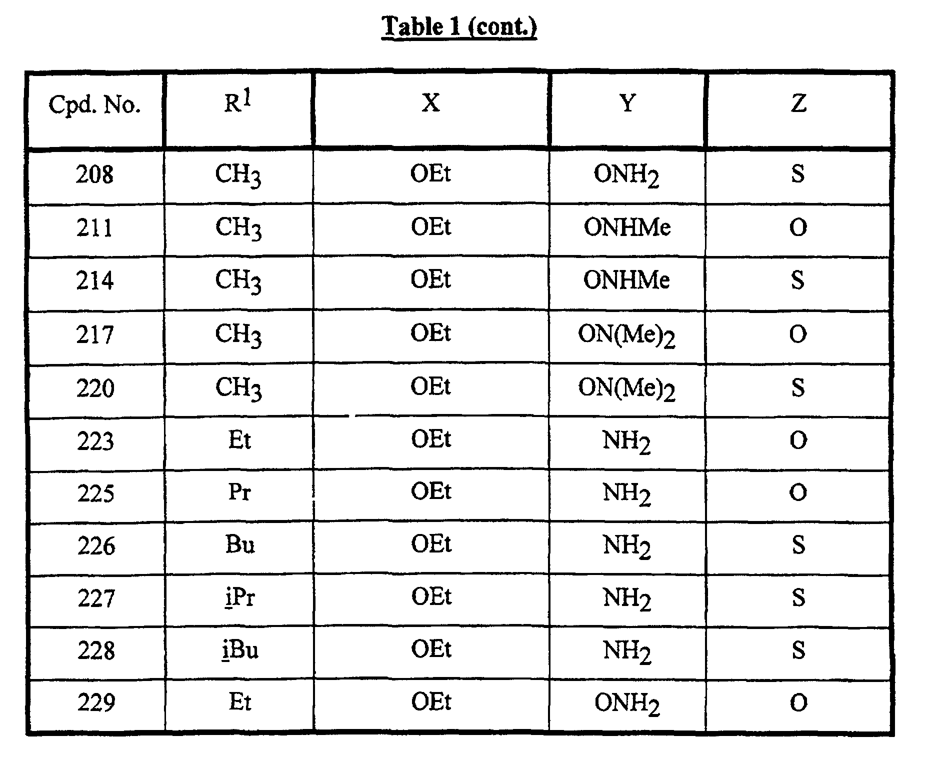

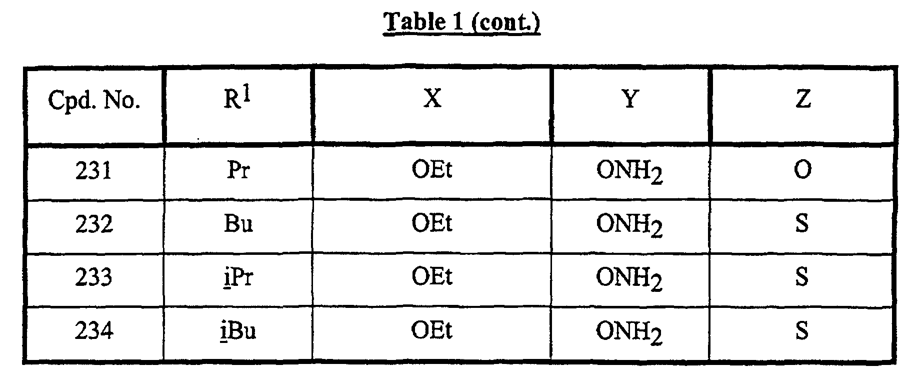

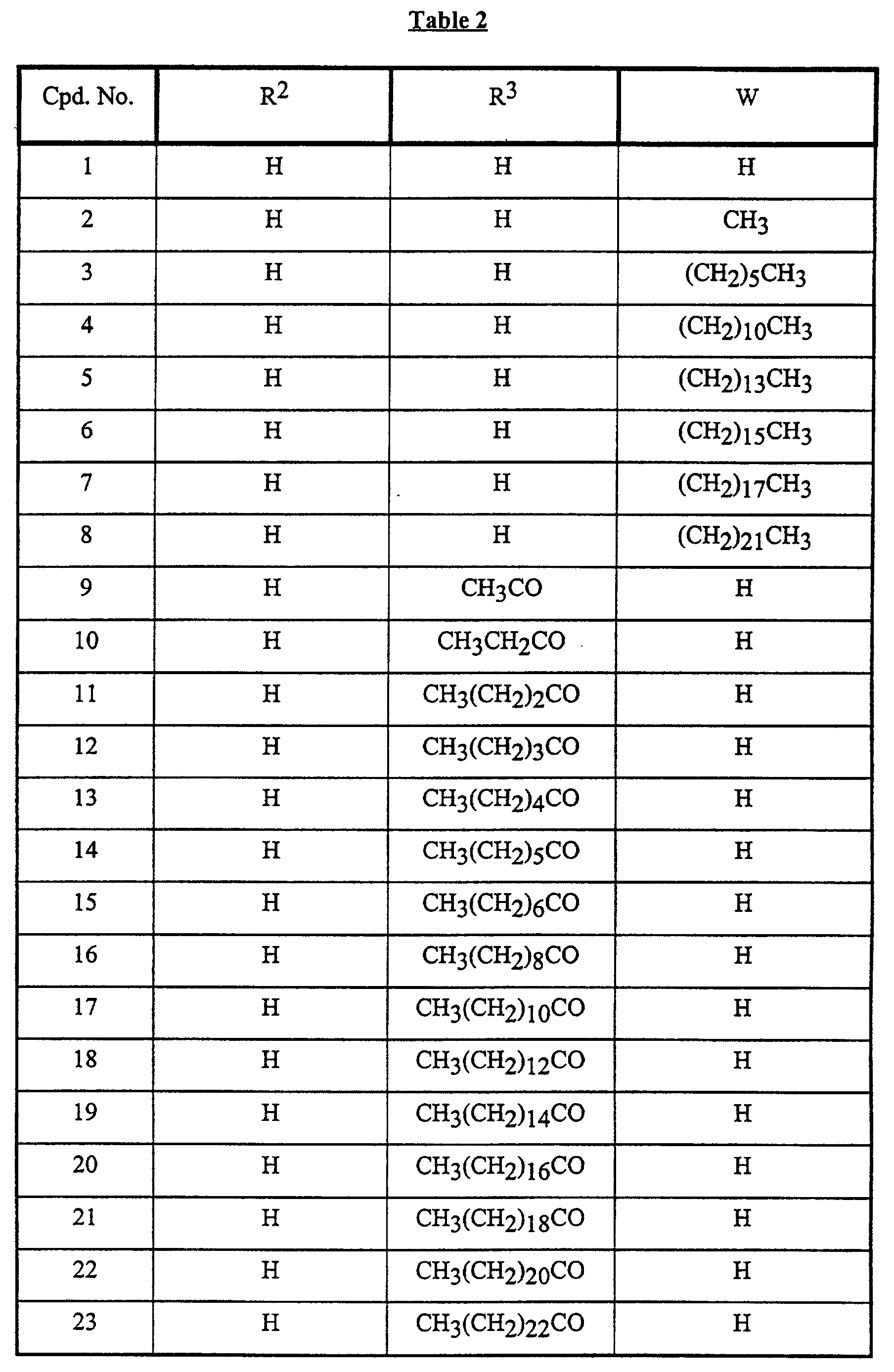

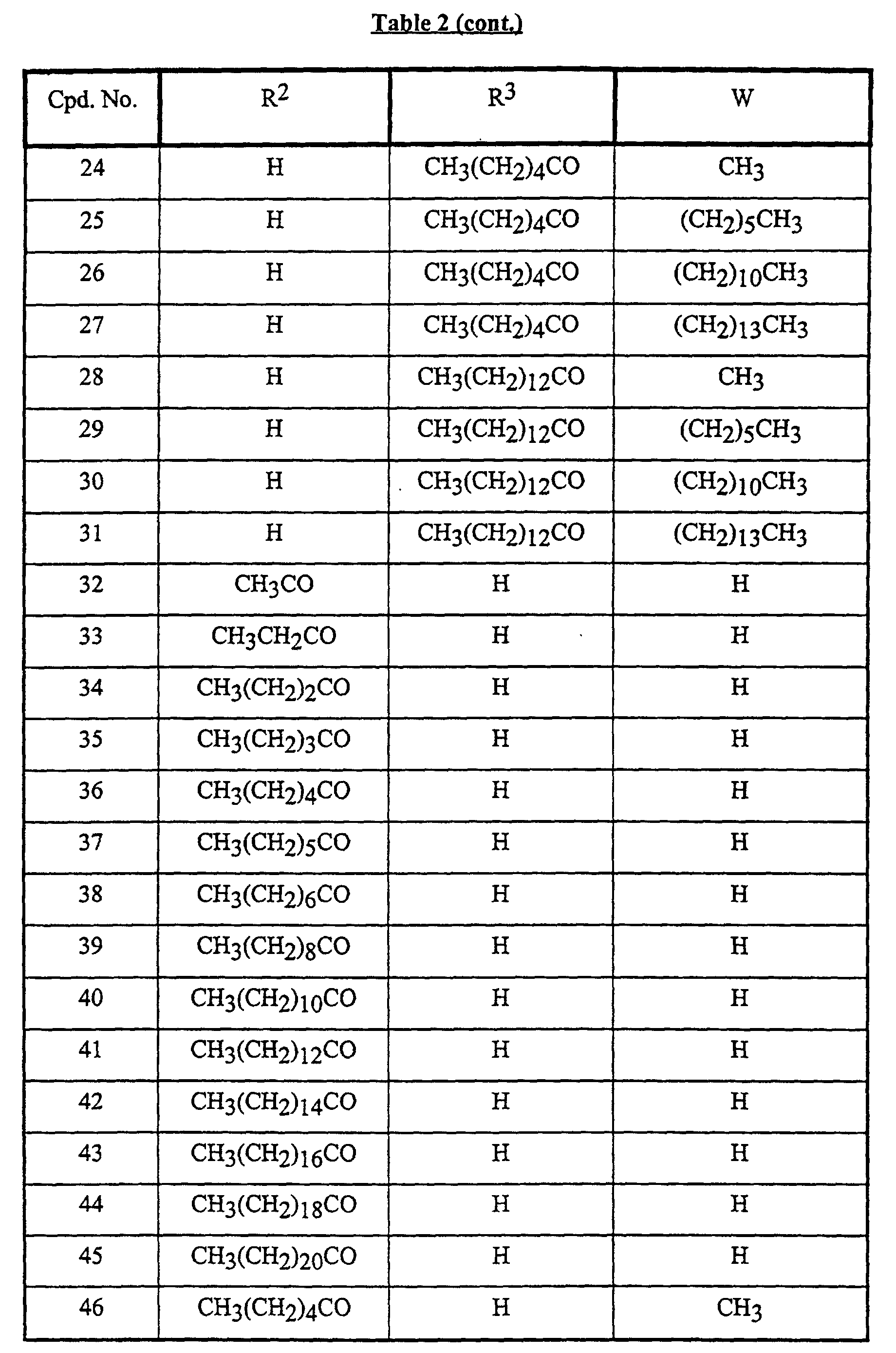

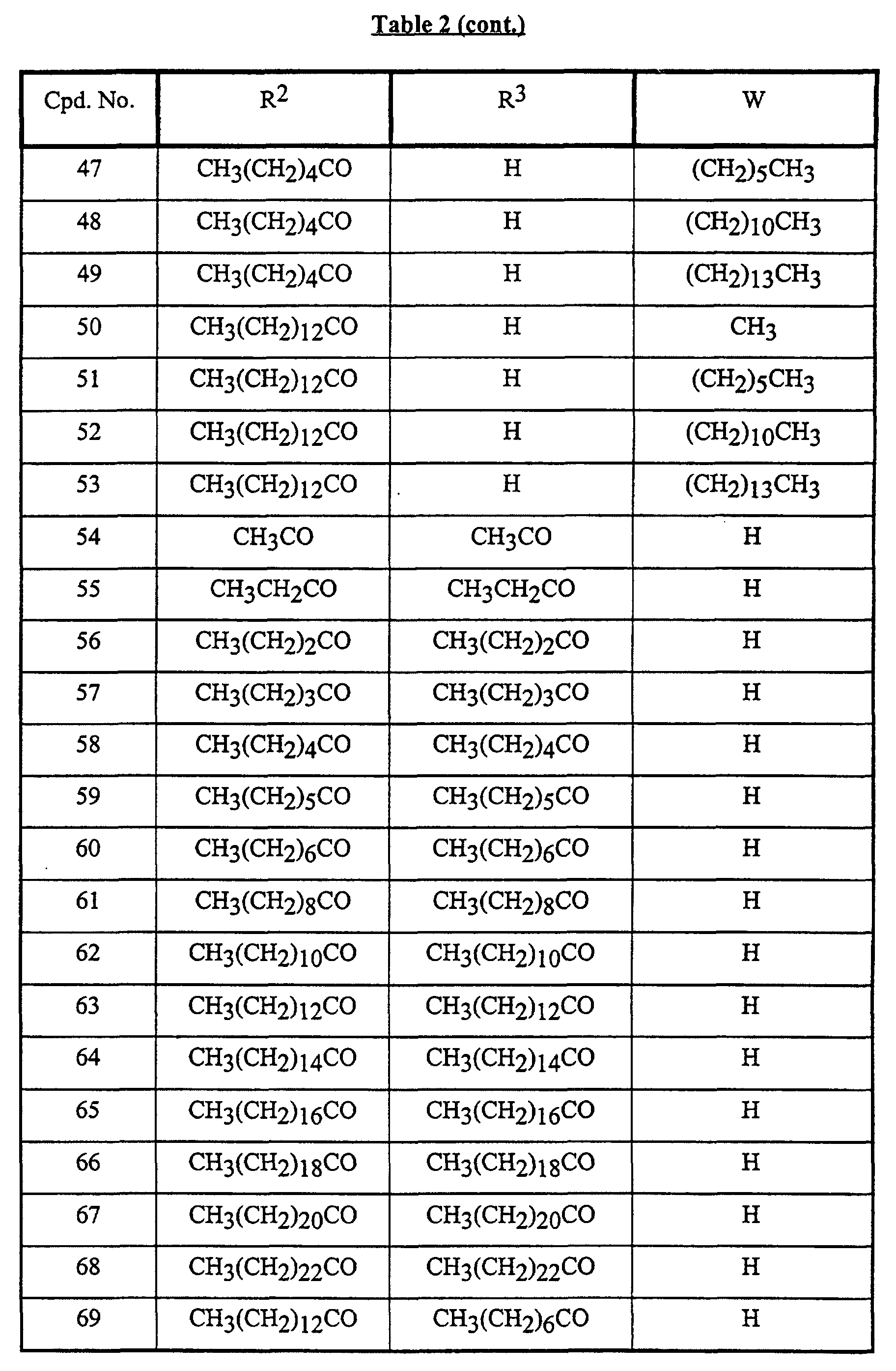

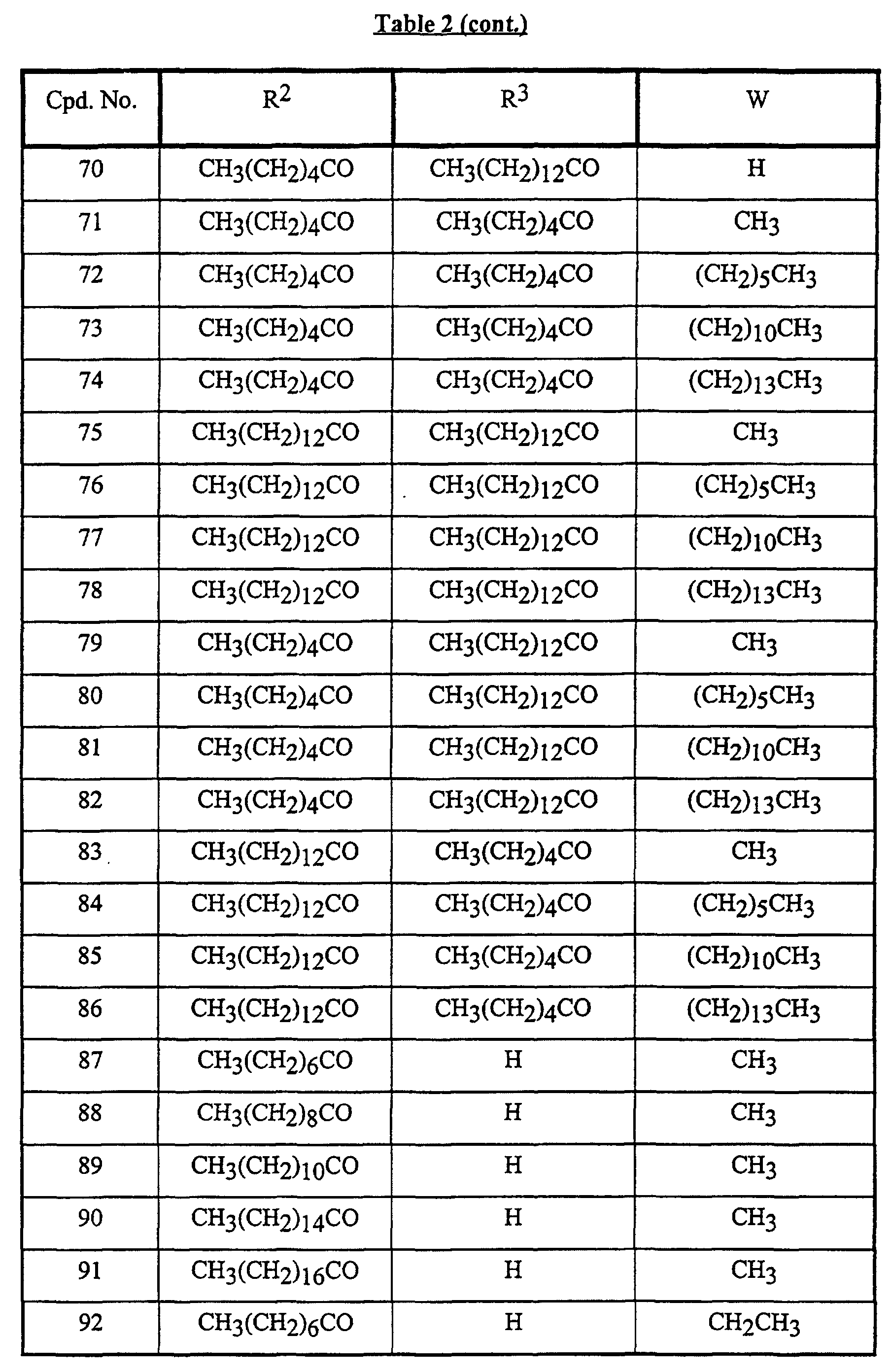

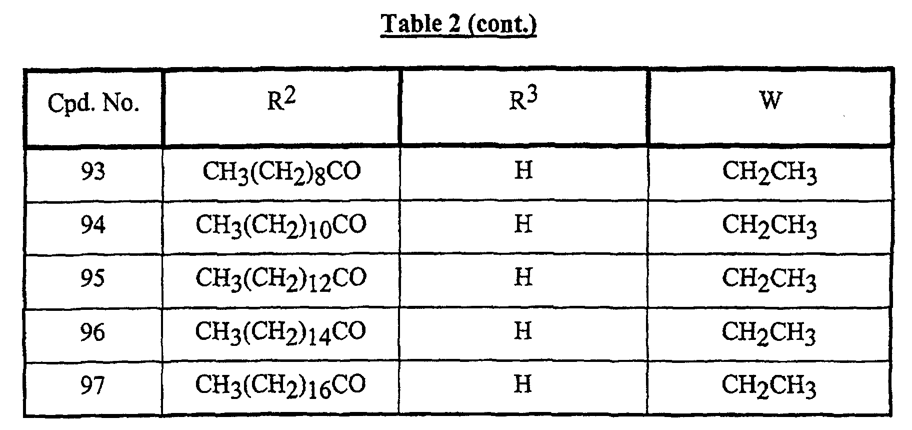

Thus, the following compounds are disclosed: Compounds No., 1-1, 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, 1-25, 1-26, 1-27, 1-28, 1-27, 1-30, 1-31, 1-32, 1-33, 1-34, 1-35, 1-36, 1-37, 1-38, 1-39, 1-40, 1-41, 1-42, 1-43, 1-44, 1-45, 1-46, 1-47, 1-48, 1-49, 1-50, 1-51, 1-52, 1-53, 1-54, 1-55, 1-56, 1-57, 1-58, 1-59, 1-60, 1-61, 1-62, 1-63, 1-64, 1-65, 1-66, 1-67, 1-68, 1-69, 1-70, 1-71, 1-72, 1-73, 1-74, 1-75, 1-76, 1-77, 1-78, 1-79, 1-80, 1-81, 1-82, 1-83, 1-84, 1-85, 1-86, 1-87, 1-88, 1-89, 1-90, 1-91, 1-92, 1-93, 1-94, 1-95, 1-96, 1-97,
4-1, 4-2, 4-3, 4-4, 4-5, 4-6, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 4-30, 4-31, 4-32, 4-33, 4-34, 4-35, 4-36, 4-37, 4-38, 4-39, 4-40, 4-41, 4-42, 4-43, 4-44, 4-45, 4-46, 4-47, 4-48, 4-49, 4-50, 4-51, 4-52, 4-53, 4-54, 4-55, 4-56, 4-57, 4-58, 4-59, 4-60, 4-61, 4-62, 4-63, 4-64, 4-65, 4-66, 4-67, 4-68, 4-69, 4-70, 4-71, 4-72, 4-73, 4-74, 4-75, 4-76, 4-77, 4-78, 4-79, 4-80, 4-81, 4-82, 4-83, 4-84, 4-85, 4-86, 4-87, 4-88, 4-89, 4-90, 4-91, 4-92, 4-93, 4-94, 4-95, 4-96, 4-97,
7-1, 7-2, 7-3, 7-4, 7-5, 7-6, 7-7, 7-8, 7-9, 7-10, 7-11, 7-12, 7-13, 7-14, 7-15, 7-16, 7-17, 7-18, 7-19, 7-20, 7-21, 7-22, 7-23, 7-24, 7-25, 7-26, 7-27, 7-28, 7-29, 7-30, 7-31, 7-32, 7-33, 7-34, 7-35, 7-36, 7-37, 7-38, 7-39, 7-40, 7-41, 7-42, 7-43, 7-44, 7-45, 7-46, 7-47, 7-48, 7-49, 7-50, 7-51, 7-52, 7-53, 7-54, 7-55, 7-56, 7-57, 7-58, 7-59, 7-60, 7-61, 7-62, 7-63, 7-64, 7-65, 7-66, 7-67, 7-68, 7-69, 7-70, 7-71, 7-72, 7-73, 7-74, 7-75, 7-76, 7-77, 7-78, 7-79, 7-80, 7-81, 7-82, 7-83, 7-84, 7-85, 7-86, 7-87, 7-88, 7-89, 7-90, 7-91, 7-92, 7-93, 7-94, 7-95, 7-96, 7-97,
10-1, 10-2, 10-3, 10-4, 10-5, 10-6, 10-7, 10-8, 10-9, 10-10, 10-11, 10-12, 10-13, 10-14, 10-15, 10-16, 10-17, 10-18, 10-19, 10-20, 10-21, 10-22, 10-23, 10-24, 10-25, 10-26, 10-27, 10-28, 10-29, 10-30, 10-31, 10-32, 10-33, 10-34, 10-35, 10-36, 10-37, 10-38, 10-39, 10-40, 10-41, 10-42, 10-43, 10-44, 10-45, 10-46, 10-47, 10-48, 10-49, 10-50, 10-51, 10-52, 10-53, 10-54, 10-55, 10-56, 10-57, 10-58, 10-59, 10-60, 10-61, 10-62, 10-63, 10-64, 10-65, 10-66, 10-67, 10-68, 10-69, 10-70, 10-71, 10-72, 10-73, 10-74, 10-75, 10-76, 10-77, 10-78, 10-79, 10-80, 10-81, 10-82, 10-83, 10-84, 10-85, 10-86, 10-87, 10-88, 10-89, 10-90, 10-91, 10-92, 10-93, 10-94, 10-95, 10-96, 10-97,
13-1, 13-2, 13-3, 13-4, 13-5, 13-6, 13-7, 13-8, 13-9, 13-10, 13-11, 13-12, 13-13, 13-14, 13-15, 13-16, 13-17, 13-18, 13-19, 13-20, 13-21, 13-22, 13-23, 13-24, 13-25, 13-26, 13-27, 13-28, 13-29, 13-30, 13-31, 13-32, 13-33, 13-34, 13-35, 13-36, 13-37, 13-38, 13-39, 13-40, 13-41, 13-42, 13-43, 13-44, 13-45, 13-46, 13-47, 13-48, 13-49, 13-50, 13-51, 13-52, 13-53, 13-54, 13-55, 13-56, 13-57, 13-58, 13-59, 13-60, 13-61, 13-62, 13-63, 13-64, 13-65, 13-66, 13-67, 13-68, 13-69, 13-70, 13-71, 13-72, 13-73, 13-74, 13-75, 13-76, 13-77, 13-78, 13-79, 13-80, 13-81, 13-82, 13-83, 13-84, 13-85, 13-86, 13-87, 13-88, 13-89, 13-90, 13-91, 13-92, 13-93, 13-94, 13-95, 13-96, 13-97,
16-1, 16-2,16-3, 16-4, 16-5, 16-6, 16-7, 16-8, 16-9, 16-10, 16-11, 16-12, 16-13, 16-14, 16-15, 16-16, 16-17, 16-18, 16-19, 16-20, 16-21, 16-22, 16-23, 16-24, 16-25, 16-26, 16-27, 16-28, 16-29, 16-30, 16-31, 16-32, 16-33, 16-34, 16-35, 16-36, 16-37, 16-38, 16-39, 16-40, 16-41, 16-42, 16-43, 16-44, 16-45, 16-46, 16-47, 16-48, 16-49, 16-50, 16-51, 16-52, 16-53, 16-54, 16-55, 16-56, 16-57, 16-58, 16-59, 16-60, 16-61, 16-62, 16-63, 16-64, 16-65, 16-66, 16-67, 16-68, 16-69, 16-70, 16-71, 16-72, 16-73, 16-74, 16-75, 16-76, 16-77, 16-78, 16-79, 16-80, 16-81, 16-82, 16-83, 16-84, 16-85, 16-86, 16-87, 16-88, 16-89, 16-90, 16-91, 16-92, 16-93, 16-94, 16-95, 16-96, 16-97,
19-1, 19-2, 19-3, 19-4, 19-5, 19-6, 19-7, 19-8, 19-9, 19-10, 19-11, 19-12, 19-13, 19-14, 19-15, 19-16, 19-17, 19-18, 19-19, 19-20, 19-21, 19-22, 19-23, 19-24, 19-25, 19-26, 19-27, 19-28, 19-29, 19-30, 19-31, 19-32, 19-33, 19-34, 19-35, 19-36, 19-37, 19-38, 19-39, 19-40, 19-41, 19-42, 19-43, 19-44, 19-45, 19-46, 19-47, 19-48, 19-49, 19-50, 19-51, 19-52, 19-53, 19-54, 19-55, 19-56, 19-57, 19-58, 19-59, 19-60, 19-61, 19-62, 19-63, 19-64, 19-65, 19-66, 19-67, 19-68, 19-69, 19-70, 19-71, 19-72, 19-73, 19-74, 19-75, 19-76, 19-77, 19-78, 19-79, 19-80, 19-81, 19-82, 19-83, 19-84, 19-85, 19-86, 19-87, 19-88, 19-89, 19-90, 19-91, 19-92, 19-93, 19-94, 19-95, 19-96, 19-97,
22-1, 22-2, 22-3, 22-4, 22-5, 22-6, 22-7, 22-8, 22-9, 22-10, 22-11, 22-12, 22-13, 22-14, 22-15, 22-16, 22-17, 22-18, 22-19, 22-20, 22-21, 22-22, 22-23, 22-24, 22-25, 22-26, 22-27, 22-28, 22-29, 22-30, 22-31, 22-32, 22-33, 22-34, 22-35, 22-36, 22-37, 22-38, 22-39, 22-40, 22-41, 22-42, 22-43, 22-44, 22-45, 22-46, 22-47, 22-48, 22-49, 22-50, 22-51, 22-52, 22-53, 22-54, 22-55, 22-56, 22-57, 22-58, 22-59, 22-60, 22-61, 22-62, 22-63, 22-64, 22-65, 22-66, 22-67, 22-68, 22-69, 22-70, 22-71, 22-72, 22-73, 22-74, 22-75, 22-76, 22-77, 22-78, 22-79, 22-80, 22-81, 22-82, 22-83, 22-84, 22-85, 22-86, 22-87, 22-88, 22-89, 22-90, 22-91, 22-92, 22-93, 22-94, 22-95, 22-96, 22-97,
25-1, 25-2, 25-3, 25-4, 25-5, 25-6, 25-7, 25-8, 25-9, 25-10, 25-11, 25-12, 25-13, 25-14, 25-15, 25-16, 25-17, 25-18, 25-19, 25-20, 25-21, 25-22, 25-23, 25-24, 25-25, 25-26, 25-27, 25-28, 25-29, 25-30, 25-31, 25-32, 25-33, 25-34, 25-35, 25-36, 25-37, 25-38, 25-39, 25-40, 25-41, 25-42, 25-43, 25-44, 25-45, 25-46, 25-47, 25-48, 25-49, 25-50, 25-51, 25-52, 25-53, 25-54, 25-55, 25-56, 25-57, 25-58, 25-59, 25-60, 25-61, 25-62, 25-63, 25-64, 25-65, 25-66, 25-67, 25-68, 25-69, 25-70, 25-71, 25-72, 25-73, 25-74, 25-75, 25-76, 25-77, 25-78, 25-79, 25-80, 25-81, 25-82, 25-83, 25-84, 25-85, 25-86, 25-87, 25-88, 25-89, 25-90, 25-91, 25-92, 25-93, 25-94, 25-95, 25-96, 25-97,
28-1, 28-2, 28-3, 28-4, 28-5, 28-6, 28-7, 28-8, 28-9, 28-10, 28-11, 28-12, 28-13, 28-14, 28-15, 28-16, 28-17, 28-18, 28-19, 28-20, 28-21, 28-22, 28-23, 28-24, 28-25, 28-26, 28-27, 28-28, 28-29, 28-30, 28-31, 28-32, 28-33, 28-34, 28-35, 28-36, 28-37, 28-38, 28-39, 28-40, 28-41, 28-42, 28-43, 28-44, 28-45, 28-46, 28-47, 28-48, 28-49, 28-50, 28-51, 28-52, 28-53, 28-54, 28-55, 28-56, 28-57, 28-58, 28-59, 28-60, 28-61, 28-62, 28-63, 28-64, 28-65, 28-66, 28-67, 28-68, 28-69, 28-70, 28-71, 28-72, 28-73, 28-74, 28-75, 28-76, 28-77, 28-78, 28-79, 28-80, 28-81, 28-82, 28-83, 28-84, 28-85, 28-86, 28-87, 28-88, 28-89, 28-90, 28-91, 28-92, 28-93, 28-94, 28-95, 28-96, 28-97,
49-1, 49-2, 49-3, 49-4, 49-5, 49-6, 49-7, 49-8, 49-9, 49-10, 49-11, 49-12, 49-13, 49-14, 49-15, 49-16, 49-17, 49-18, 49-19, 49-20, 49-21, 49-22, 49-23, 49-24, 49-25, 49-26, 49-27, 49-28, 49-29, 49-30, 49-31, 49-32, 49-33, 49-34, 49-35, 49-36, 49-37, 49-38, 49-39, 49-40, 49-41, 49-42, 49-43, 49-44, 49-45, 49-46, 49-47, 49-48, 49-49, 49-50, 49-51, 49-52, 49-53, 49-54, 49-55, 49-56, 49-57, 49-58, 49-59, 49-60, 49-61, 49-62, 49-63, 49-64, 49-65, 49-66, 49-67, 49-68, 49-69, 49-70, 49-71, 49-72, 49-73, 49-74, 49-75, 49-76, 49-77, 49-78, 49-79, 49-80, 49-81, 49-82, 49-83, 49-84, 49-85, 49-86, 49-87, 49-88, 49-89, 49-90, 49-91, 49-92, 49-93, 49-94, 49-95, 49-96, 49-97,
52-1, 52-2, 52-3, 52-4, 52-5, 52-6, 52-7, 52-8, 52-9, 52-10, 52-11, 52-12, 52-13, 52-14, 52-15, 52-16, 52-17, 52-18, 52-19, 52-20, 52-21, 52-22, 52-23, 52-24, 52-25, 52-26, 52-27, 52-28, 52-29, 52-30, 52-31, 52-32, 52-33, 52-34, 52-35, 52-36, 52-37, 52-38, 52-39, 52-40, 52-41, 52-42, 52-43, 52-44, 52-45, 52-46, 52-47, 52-48, 52-49, 52-50, 52-51, 52-52, 52-53, 52-54, 52-55, 52-56, 52-57, 52-58, 52-59, 52-60, 52-61, 52-62, 52-63, 52-64, 52-65, 52-66, 52-67, 52-68, 52-69, 52-70, 52-71, 52-72, 52-73, 52-74, 52-75, 52-76, 52-77, 52-78, 52-79, 52-80, 52-81, 52-82, 52-83, 52-84, 52-85, 52-86, 52-87, 52-88, 52-89, 52-90, 52-91, 52-92, 52-93, 52-94, 52-95, 52-96, 52-97,
54-1, 54-2, 54-3, 54-4, 54-5, 54-6, 54-7, 54-8, 54-9, 54-10, 54-11, 54-12, 54-13, 54-14, 54-15, 54-16, 54-17, 54-18, 54-19, 54-20, 54-21, 54-22, 54-23, 54-24, 54-25, 54-26, 54-27, 54-28, 54-29, 54-30, 54-31, 54-32, 54-33, 54-34, 54-35, 54-36, 54-37, 54-38, 54-39, 54-40, 54-41, 54-42, 54-43, 54-44, 54-45, 54-46, 54-47, 54-48, 54-49, 54-50, 54-51, 54-52, 54-53, 54-54, 54-55, 54-56, 54-57, 54-58, 54-59, 54-60, 54-61, 54-62, 54-63, 54-64, 54-65, 54-66, 54-67, 54-68, 54-69, 54-70, 54-71, 54-72, 54-73, 54-74, 54-75, 54-76, 54-77, 54-78, 54-79, 54-80, 54-81, 54-82, 54-83, 54-84, 54-85, 54-86, 54-87, 54-88, 54-89, 54-90, 54-91, 54-92, 54-93, 54-94, 54-95, 54-96, 54-97,
55-1, 55-2, 55-3, 55-4, 55-5, 55-6, 55-7, 55-8, 55-9, 55-10, 55-11, 55-12, 55-13, 55-14, 55-15, 55-16, 55-17, 55-18, 55-19, 55-20, 55-21, 55-22, 55-23, 55-24, 55-25, 55-26, 55-27, 55-28, 55-29, 55-30, 55-31, 55-32, 55-33, 55-34, 55-35, 55-36, 55-37, 55-38, 55-39, 55-40, 55-41, 55-42, 55-43, 55-44, 55-45, 55-46, 55-47, 55-48, 55-49, 55-50, 55-51, 55-52, 55-53, 55-54, 55-55, 55-56, 55-57, 55-58, 55-59, 55-60, 55-61, 55-62, 55-63, 55-64, 55-65, 55-66, 55-67, 55-68, 55-69, 55-70, 55-71, 55-72, 55-73, 55-74, 55-75, 55-76, 55-77, 55-78, 55-79, 55-80, 55-81, 55-82, 55-83, 55-84, 55-85, 55-86, 55-87, 55-88, 55-89, 55-90, 55-91, 55-92, 55-93, 55-94, 55-95, 55-96, 55-97,
56-1, 56-2, 56-3, 56-4, 56-5, 56-6, 56-7, 56-8, 56-9, 56-10, 56-11, 56-12, 56-13, 56-14, 56-15, 56-16, 56-17, 56-18, 56-19, 56-20, 56-21, 56-22, 56-23, 56-24, 56-25, 56-26, 56-27, 56-28, 56-29, 56-30, 56-31, 56-32, 56-33, 56-34, 56-35, 56-36, 56-37, 56-38, 56-39, 56-40, 56-41, 56-42, 56-43, 56-44, 56-45, 56-46, 56-47, 56-48, 56-49, 56-50, 56-51, 56-52, 56-53, 56-54, 56-55, 56-56, 56-57, 56-58, 56-59, 56-60, 56-61, 56-62, 56-63, 56-64, 56-65, 56-66, 56-67, 56-68, 56-69, 56-70, 56-71, 56-72, 56-73, 56-74, 56-75, 56-76, 56-77, 56-78, 56-79, 56-80, 56-81, 56-82, 56-83, 56-84, 56-85, 56-86, 56-87, 56-88, 56-89, 56-90, 56-91, 56-92, 56-93, 56-94, 56-95, 56-96, 56-97,
57-1, 57-2, 57-3, 57-4, 57-5, 57-6, 57-7, 57-8, 57-9, 57-10, 57-11, 57-12, 57-13, 57-14, 57-15, 57-16, 57-17, 57-18, 57-19, 57-20, 57-21, 57-22, 57-23, 57-24, 57-25, 57-26, 57-27, 57-28, 57-29, 57-30, 57-31, 57-32, 57-33, 57-34, 57-35, 57-36, 57-37, 57-38, 57-39, 57-40, 57-41, 57-42, 57-43, 57-44, 57-45, 57-46, 57-47, 57-48, 57-49, 57-50, 57-51, 57-52, 57-53, 57-54, 57-55, 57-56, 57-57, 57-58, 57-59, 57-60, 57-61, 57-62, 57-63, 57-64, 57-65, 57-66, 57-67, 57-68, 57-69, 57-70, 57-71, 57-72, 57-73, 57-74, 57-75, 57-76, 57-77, 57-78, 57-79, 57-80, 57-81, 57-82, 57-83, 57-84, 57-85, 57-86, 57-87, 57-88, 57-89, 57-90, 57-91, 57-92, 57-93, 57-94, 57-95, 57-96, 57-97,
61-1, 61-2, 61-3, 61-4, 61-5, 61-6, 61-7, 61-8, 61-9, 61-10, 61-11, 61-12, 61-13, 61-14, 61-15, 61-16, 61-17, 61-18, 61-19, 61-20, 61-21, 61-22, 61-23, 61-24, 61-25, 61-26, 61-27, 61-28, 61-29, 61-30, 61-31, 61-32, 61-33, 61-34, 61-35, 61-36, 61-37, 61-38, 61-39, 61-40, 61-41, 61-42, 61-43, 61-44, 61-45, 61-46, 61-47, 61-48, 61-49, 61-50, 61-51, 61-52, 61-53, 61-54, 61-55, 61-56, 61-57, 61-58, 61-59, 61-60, 61-61, 61-62, 61-63, 61-64, 61-65, 61-66, 61-67, 61-68, 61-69, 61-70, 61-71, 61-72, 61-73, 61-74, 61-75, 61-76, 61-77, 61-78, 61-79, 61-80, 61-81, 61-82, 61-83, 61-84, 61-85, 61-86, 61-87, 61-88, 61-89, 61-90, 61-91, 61-92, 61-93, 61-94, 61-95, 61-96, 61-97,
63-1, 63-2, 63-3, 63-4, 63-5, 63-6, 63-7, 63-8, 63-9, 63-10, 63-11, 63-12, 63-13, 63-14, 63-15, 63-16, 63-17, 63-18, 63-19, 63-20, 63-21, 63-22, 63-23, 63-24, 63-25, 63-26, 63-27, 63-28, 63-29, 63-30, 63-31, 63-32, 63-33, 63-34, 63-35, 63-36, 63-37, 63-38, 63-39, 63-40, 63-41, 63-42, 63-43, 63-44, 63-45, 63-46, 63-47, 63-48, 63-49, 63-50, 63-51, 63-52, 63-53, 63-54, 63-55, 63-56, 63-57, 63-58, 63-59, 63-60, 63-61, 63-62, 63-63, 63-64, 63-65, 63-66, 63-67, 63-68, 63-69, 63-70, 63-71, 63-72, 63-73, 63-74, 63-75, 63-76, 63-77, 63-78, 63-79, 63-80, 63-81, 63-82, 63-83, 63-84, 63-85, 63-86, 63-87, 63-88, 63-89, 63-90, 63-91, 63-92, 63-93, 63-94, 63-95, 63-96, 63-97,
64-1, 64-2, 64-3, 64-4, 64-5, 64-6, 64-7, 64-8, 64-9, 64-10, 64-11, 64-12, 64-13, 64-14, 64-15, 64-16, 64-17, 64-18, 64-19, 64-20, 64-21, 64-22, 64-23, 64-24, 64-25, 64-26, 64-27, 64-28, 64-29, 64-30, 64-31, 64-32, 64-33, 64-34, 64-35, 64-36, 64-37, 64-38, 64-39, 64-40, 64-41, 64-42, 64-43, 64-44, 64-45, 64-46, 64-47, 64-48, 64-49, 64-50, 64-51, 64-52, 64-53, 64-54, 64-55, 64-56, 64-57, 64-58, 64-59, 64-60, 64-61, 64-62, 64-63, 64-64, 64-65, 64-66, 64-67, 64-68, 64-69, 64-70, 64-71, 64-72, 64-73, 64-74, 64-75, 64-76, 64-77, 64-78, 64-79, 64-80, 64-81, 64-82, 64-83, 64-84, 64-85, 64-86, 64-87, 64-88, 64-89, 64-90, 64-91, 64-92, 64-93, 64-94, 64-95, 64-96, 64-97,
65-1, 65-2, 65-3, 65-4, 65-5, 65-6, 65-7, 65-8, 65-9, 65-10, 65-11, 65-12, 65-13, 65-14, 65-15, 65-16, 65-17, 65-18, 65-19, 65-20, 65-21, 65-22, 65-23, 65-24, 65-25, 65-26, 65-27, 65-28, 65-29, 65-30, 65-31, 65-32, 65-33, 65-34, 65-35, 65-36, 65-37, 65-38, 65-39, 65-40, 65-41, 65-42, 65-43, 65-44, 65-45, 65-46, 65-47, 65-48, 65-49, 65-50, 65-51, 65-52, 65-53, 65-54, 65-55, 65-56, 65-57, 65-58, 65-59, 65-60, 65-61, 65-62, 65-63, 65-64, 65-65, 65-66, 65-67, 65-68,
65-69, 65-70, 65-71, 65-72, 65-73, 65-74, 65-75, 65-76, 65-77, 65-78, 65-79, 65-80, 65-81, 65-82, 65-83, 65-84, 65-85, 65-86, 65-87, 65-88, 65-89, 65-90, 65-91, 65-92, 65-93, 65-94, 65-95, 65-96, 65-97,
66-1, 66-2, 66-3, 66-4, 66-5, 66-6, 66-7, 66-8, 66-9, 66-10, 66-11, 66-12, 66-13, 66-14, 66-15, 66-16, 66-17, 66-18, 66-19, 66-20, 66-21, 66-22, 66-23, 66-24, 66-25, 66-26, 66-27, 66-28, 66-29, 66-30, 66-31, 66-32, 66-33, 66-34, 66-35, 66-36, 66-37, 66-38, 66-39, 66-40, 66-41, 66-42, 66-43, 66-44, 66-45, 66-46, 66-47, 66-48, 66-49, 66-50, 66-51, 66-52, 66-53, 66-54, 66-55, 66-56, 66-57, 66-58, 66-59, 66-60, 66-61, 66-62, 66-63, 66-64, 66-65, 66-66, 66-67, 66-68, 66-69, 66-70, 66-71, 66-72, 66-73, 66-74, 66-75, 66-76, 66-77, 66-78, 66-79, 66-80, 66-81, 66-82, 66-83, 66-84, 66-85, 66-86, 66-87, 66-88, 66-89, 66-90, 66-91, 66-92, 66-93, 66-94, 66-95, 66-96, 66-97,
163-1, 163-2, 163-3, 163-4, 163-5, 163-6, 163-7, 163-8, 163-9, 163-10, 163-11, 163-12, 163-13, 163-14, 163-15, 163-16, 163-17, 163-18, 163-19, 163-20, 163-21, 163-22, 163-23, 163-24, 163-25, 163-26, 163-27, 163-28, 163-29, 163-30, 163-31, 163-32, 163-33, 163-34, 163-35, 163-36, 163-37, 163-38, 163-39, 163-40, 163-41, 163-42, 163-43, 163-44, 163-45, 163-46, 163-47, 163-48, 163-49, 163-50, 163-51, 163-52, 163-53, 163-54, 163-55, 163-56, 163-57, 163-58, 163-59, 163-60, 163-61, 163-62, 163-63, 163-64, 163-65, 163-66, 163-67, 163-68, 163-69, 163-70, 163-71, 163-72, 163-73, 163-74, 163-75, 163-76, 163-77, 163-78, 163-79, 163-80, 163-81, 163-82, 163-83, 163-84, 163-85, 163-86, 163-87, 163-88, 163-89, 163-90, 163-91, 163-92, 163-93, 163-94, 163-95, 163-96, 163-97,
166-1, 166-2, 166-3, 166-4, 166-5, 166-6, 166-7, 166-8, 166-9, 166-10, 166-11, 166-12, 166-13, 166-14, 166-15, 166-16, 166-17, 166-18, 166-19, 166-20, 166-21, 166-22, 166-23, 166-24, 166-25, 166-26, 166-27, 166-28, 166-29, 166-30, 166-31, 166-32, 166-33, 166-34, 166-35, 166-36, 166-37, 166-38, 166-39, 166-40, 166-41, 166-42, 166-43, 166-44, 166-45, 166-46, 166-47, 166-48, 166-49, 166-50, 166-51, 166-52, 166-53, 166-54, 166-55, 166-56, 166-57, 166-58, 166-59, 166-60, 166-61, 166-62, 166-63, 166-64, 166-65, 166-66, 166-67, 166-68, 166-69, 166-70, 166-71, 166-72, 166-73, 166-74, 166-75, 166-76, 166-77, 166-78, 166-79, 166-80, 166-81, 166-82, 166-83, 166-84, 166-85, 166-86, 166-87, 166-88, 166-89, 166-90, 166-91, 166-92, 166-93, 166-94, 166-95, 166-96, 166-97,
169-1, 169-2, 169-3, 169-4, 169-5, 169-6, 169-7, 169-8, 169-9, 169-10, 169-11, 169-12, 169-13, 169-14, 169-15, 169-16, 169-17, 169-18, 169-19, 169-20, 169-21, 169-22, 169-23, 169-24, 169-25, 169-26, 169-27, 169-28, 169-29, 169-30, 169-31, 169-32, 169-33, 169-34, 169-35, 169-36, 169-37, 169-38, 169-39, 169-40, 169-41, 169-42, 169-43, 169-44, 169-45, 169-46, 169-47, 169-48, 169-49, 169-50, 169-51, 169-52, 169-53, 169-54, 169-55, 169-56, 169-57, 169-58, 169-59, 169-60, 169-61, 169-62, 169-63, 169-64, 169-65, 169-66, 169-67, 169-68, 169-69, 169-70, 169-71, 169-72, 169-73, 169-74, 169-75, 169-76, 169-77, 169-78, 169-79, 169-80, 169-81, 169-82, 169-83, 169-84, 169-85, 169-86, 169-87, 169-88, 169-89, 169-90, 169-91, 169-92, 169-93, 169-94, 169-95, 169-96, 169-97,
172-1, 172-2, 172-3, 172-4, 172-5, 172-6, 172-7, 172-8, 172-9, 172-10, 172-11, 172-12, 172-13, 172-14, 172-15, 172-16, 172-17, 172-18, 172-19, 172-20, 172-21, 172-22, 172-23, 172-24, 172-25, 172-26, 172-27, 172-28, 172-29, 172-30, 172-31, 172-32, 172-33, 172-34, 172-35, 172-36, 172-37, 172-38, 172-39, 172-40, 172-41, 172-42, 172-43, 172-44, 172-45, 172-46, 172-47, 172-48, 172-49, 172-50, 172-51, 172-52, 172-53, 172-54, 172-55, 172-56, 172-57, 172-58, 172-59, 172-60, 172-61, 172-62, 172-63, 172-64, 172-65, 172-66, 172-67, 172-68, 172-69, 172-70, 172-71, 172-72, 172-73, 172-74, 172-75, 172-76, 172-77, 172-78, 172-79, 172-80, 172-81, 172-82, 172-83, 172-84, 172-85, 172-86, 172-87, 172-88, 172-89, 172-90, 172-91, 172-92, 172-93, 172-94, 172-95, 172-96, 172-97,
175-1, 175-2, 175-3, 175-4, 175-5, 175-6, 175-7, 175-8, 175-9, 175-10, 175-11, 175-12, 175-13, 175-14, 175-15, 175-16, 175-17, 175-18, 175-19, 175-20, 175-21, 175-22, 175-23, 175-24, 175-25, 175-26, 175-27, 175-28, 175-29, 175-30, 175-31, 175-32, 175-33, 175-34, 175-35, 175-36, 175-37, 175-38, 175-39, 175-40, 175-41, 175-42, 175-43, 175-44, 175-45, 175-46, 175-47, 175-48, 175-49, 175-50, 175-51, 175-52, 175-53, 175-54, 175-55, 175-56, 175-57, 175-58, 175-59, 175-60, 175-61, 175-62, 175-63, 175-64, 175-65, 175-66, 175-67, 175-68, 175-69, 175-70, 175-71, 175-72, 175-73, 175-74, 175-75, 175-76, 175-77, 175-78, 175-79, 175-80, 175-81, 175-82, 175-83, 175-84, 175-85, 175-86, 175-87, 175-88, 175-89, 175-90, 175-91, 175-92, 175-93, 175-94, 175-95, 175-96, 175-97,
178-1, 178-2, 178-3, 178-4, 178-5, 178-6, 178-7, 178-8, 178-9, 178-10, 178-11, 178-12, 178-13, 178-14, 178-15, 178-16, 178-17, 178-18, 178-19, 178-20, 178-21, 178-22, 178-23, 178-24, 178-25, 178-26, 178-27, 178-28, 178-29, 178-30, 178-31, 178-32, 178-33, 178-34, 178-35, 178-36, 178-37, 178-38, 178-39, 178-40, 178-41, 178-42, 178-43, 178-44, 178-45, 178-46, 178-47, 178-48, 178-49, 178-50, 178-51, 178-52, 178-53, 178-54, 178-55, 178-56, 178-57, 178-58, 178-59, 178-60, 178-61, 178-62, 178-63, 178-64, 178-65, 178-66, 178-67, 178-68, 178-69, 178-70, 178-71, 178-72, 178-73, 178-74, 178-75, 178-76, 178-77, 178-78, 178-79, 178-80, 178-81, 178-82, 178-83, 178-84, 178-85, 178-86, 178-87, 178-88, 178-89, 178-90, 178-91, 178-92, 178-93, 178-94, 178-95, 178-96, 178-97,
181-1, 181-2, 181-3, 181-4, 181-5, 181-6, 181-7, 181-8, 181-9, 181-10, 181-11, 181-12, 181-13, 181-14, 181-15, 181-16, 181-17, 181-18, 181-19, 181-20, 181-21, 181-22, 181-23, 181-24, 181-25, 181-26, 181-27, 181-28, 181-29, 181-30, 181-31, 181-32, 181-33, 181-34, 181-35, 181-36, 181-37, 181-38, 181-39, 181-40, 181-41, 181-42, 181-43, 181-44, 181-45, 181-46, 181-47, 181-48, 181-49, 181-50, 181-51, 181-52, 181-53, 181-54, 181-55, 181-56, 181-57, 181-58, 181-59, 181-60, 181-61, 181-62, 181-63, 181-64, 181-65, 181-66, 181-67, 181-68, 181-69, 181-70, 181-71, 181-72, 181-73, 181-74, 181-75, 181-76, 181-77, 181-78, 181-79, 181-80, 181-81, 181-82, 181-83, 181-84, 181-85, 181-86, 181-87, 181-88, 181-89, 181-90, 181-91, 181-92, 181-93, 181-94, 181-95, 181-96, 181-97,
184-1, 184-2, 184-3, 184-4, 184-5, 184-6, 184-7, 184-8, 184-9, 184-10, 184-11, 184-12, 184-13, 184-14, 184-15, 184-16, 184-17, 184-18, 184-19, 184-20, 184-21, 184-22, 184-23, 184-24, 184-25, 184-26, 184-27, 184-28, 184-29, 184-30, 184-31, 184-32, 184-33, 184-34, 184-35, 184-36, 184-37, 184-38, 184-39, 184-40, 184-41, 184-42, 184-43, 184-44, 184-45, 184-46, 184-47, 184-48, 184-49, 184-50, 184-51, 184-52, 184-53, 184-54, 184-55, 184-56, 184-57, 184-58, 184-59, 184-60, 184-61, 184-62, 184-63, 184-64, 184-65, 184-66, 184-67, 184-68, 184-69, 184-70, 184-71, 184-72, 184-73, 184-74, 184-75, 184-76, 184-77, 184-78, 184-79, 184-80, 184-81, 184-82, 184-83, 184-84, 184-85, 184-86, 184-87, 184-88, 184-89, 184-90, 184-91, 184-92, 184-93, 184-94, 184-95, 184-96, 184-97,
187-1, 187-2, 187-3, 187-4, 187-5, 187-6, 187-7, 187-8, 187-9, 187-10, 187-11, 187-12, 187-13, 187-14, 187-15, 187-16, 187-17, 187-18, 187-19, 187-20, 187-21, 187-22, 187-23, 187-24, 187-25, 187-26, 187-27, 187-28, 187-29, 187-30, 187-31, 187-32, 187-33, 187-34, 187-35, 187-36, 187-37, 187-38, 187-39, 187-40, 187-41, 187-42, 187-43, 187-44, 187-45, 187-46, 187-47, 187-48, 187-49, 187-50, 187-51, 187-52, 187-53, 187-54, 187-55, 187-56, 187-57, 187-58, 187-59, 187-60, 187-61, 187-62, 187-63, 187-64, 187-65, 187-66, 187-67, 187-68, 187-69, 187-70, 187-71, 187-72, 187-73, 187-74, 187-75, 187-76, 187-77, 187-78, 187-79, 187-80, 187-81, 187-82, 187-83, 187-84, 187-85, 187-86, 187-87, 187-88, 187-89, 187-90, 187-91, 187-92, 187-93, 187-94, 187-95, 187-96, 187-97,
189-1, 189-2, 189-3, 189-4, 189-5, 189-6, 189-7, 189-8, 189-9, 189-10, 189-11, 189-12, 189-13, 189-14, 189-15, 189-16, 189-17, 189-18, 189-19, 189-20, 189-21, 189-22, 189-23, 189-24, 189-25, 189-26, 189-27, 189-28, 189-29, 189-30, 189-31, 189-32, 189-33, 189-34, 189-35, 189-36, 189-37, 189-38, 189-39, 189-40, 189-41, 189-42, 189-43, 189-44, 189-45, 189-46, 189-47, 189-48, 189-49, 189-50, 189-51, 189-52, 189-53, 189-54, 189-55, 189-56, 189-57, 189-58, 189-59, 189-60, 189-61, 189-62, 189-63, 189-64, 189-65, 189-66, 189-67, 189-68, 189-69, 189-70, 189-71, 189-72, 189-73, 189-74, 189-75, 189-76, 189-77, 189-78, 189-79, 189-80, 189-81, 189-82, 189-83, 189-84, 189-85, 189-86, 189-87, 189-88, 189-89, 189-90, 189-91, 189-92, 189-93, 189-94, 189-95, 189-96, 189-97,
190-1, 190-2, 190-3, 190-4, 190-5, 190-6, 190-7, 190-8, 190-9, 190-10, 190-11, 190-12, 190-13, 190-14, 190-15, 190-16, 190-17, 190-18, 190-19, 190-20, 190-21, 190-22, 190-23, 190-24, 190-25, 190-26, 190-27, 190-28, 190-29, 190-30, 190-31, 190-32, 190-33, 190-34, 190-35, 190-36, 190-37, 190-38, 190-39, 190-40, 190-41, 190-42, 190-43, 190-44, 190-45, 190-46, 190-47, 190-48, 190-49, 190-50, 190-51, 190-52, 190-53, 190-54, 190-55, 190-56, 190-57, 190-58, 190-59, 190-60, 190-61, 190-62, 190-63, 190-64, 190-65, 190-66, 190-67, 190-68, 190-69, 190-70, 190-71, 190-72, 190-73, 190-74, 190-75, 190-76, 190-77, 190-78, 190-79, 190-80, 190-81, 190-82, 190-83, 190-84, 190-85, 190-86, 190-87, 190-88, 190-89, 190-90, 190-91, 190-92, 190-93, 190-94, 190-95, 190-96, 190-97,
191-1, 191-2, 191-3, 191-4, 191-5, 191-6, 191-7, 191-8, 191-9, 191-10, 191-11, 191-12, 191-13, 191-14, 191-15, 191-16, 191-17, 191-18, 191-19, 191-20, 191-21, 191-22, 191-23, 191-24, 191-25, 191-26, 191-27, 191-28, 191-29, 191-30, 191-31, 191-32, 191-33, 191-34, 191-35, 191-36, 191-37, 191-38, 191-39, 191-40, 191-41, 191-42, 191-43, 191-44, 191-45, 191-46, 191-47, 191-48, 191-49, 191-50, 191-51, 191-52, 191-53, 191-54, 191-55, 191-56, 191-57, 191-58, 191-59, 191-60, 191-61, 191-62, 191-63, 191-64, 191-65, 191-66, 191-67, 191-68, 191-69, 191-70, 191-71, 191-72, 191-73, 191-74, 191-75, 191-76, 191-77, 191-78, 191-79, 191-80, 191-81, 191-82, 191-83, 191-84, 191-85, 191-86, 191-87, 191-88, 191-89, 191-90, 191-91, 191-92, 191-93, 191-94, 191-95, 191-96, 191-97,
192-1, 192-2, 192-3, 192-4, 192-5, 192-6, 192-7, 192-8, 192-9, 192-10, 192-11, 192-12, 192-13, 192-14, 192-15, 192-16, 192-17, 192-18, 192-19, 192-20, 192-21, 192-22, 192-23, 192-24, 192-25, 192-26, 192-27, 192-28, 192-29, 192-30, 192-31, 192-32, 192-33, 192-34, 192-35, 192-36, 192-37, 192-38, 192-39, 192-40, 192-41, 192-42, 192-43, 192-44, 192-45, 192-46, 192-47, 192-48, 192-49, 192-50, 192-51, 192-52, 192-53, 192-54, 192-55, 192-56, 192-57, 192-58, 192-59, 192-60, 192-61, 192-62, 192-63, 192-64, 192-65, 192-66, 192-67, 192-68, 192-69, 192-70, 192-71, 192-72, 192-73, 192-74, 192-75, 192-76, 192-77, 192-78, 192-79, 192-80, 192-81, 192-82, 192-83, 192-84, 192-85, 192-86, 192-87, 192-88, 192-89, 192-90, 192-91, 192-92, 192-93, 192-94, 192-95, 192-96, 192-97,
193-1, 193-2, 193-3, 193-4, 193-5, 193-6, 193-7, 193-8, 193-9, 193-10, 193-11, 193-12, 193-13, 193-14, 193-15, 193-16, 193-17, 193-18, 193-19, 193-20, 193-21, 193-22, 193-23, 193-24, 193-25, 193-26, 193-27, 193-28, 193-29, 193-30, 193-31, 193-32, 193-33, 193-34, 193-35, 193-36, 193-37, 193-38, 193-39, 193-40, 193-41, 193-42, 193-43, 193-44, 193-45, 193-46, 193-47, 193-48, 193-49, 193-50, 193-51, 193-52, 193-53, 193-54, 193-55, 193-56, 193-57, 193-58, 193-59, 193-60, 193-61, 193-62, 193-63, 193-64, 193-65, 193-66, 193-67, 193-68, 193-69, 193-70, 193-71, 193-72, 193-73, 193-74, 193-75, 193-76, 193-77, 193-78, 193-79, 193-80, 193-81, 193-82, 193-83, 193-84, 193-85, 193-86, 193-87, 193-88, 193-89, 193-90, 193-91, 193-92, 193-93, 193-94, 193-95, 193-96, 193-97,
195-1, 195-2, 195-3, 195-4, 195-5, 195-6, 195-7, 195-8, 195-9, 195-10, 195-11, 195-12, 195-13, 195-14, 195-15, 195-16, 195-17, 195-18, 195-19, 195-20, 195-21, 195-22, 195-23, 195-24, 195-25, 195-26, 195-27, 195-28, 195-29, 195-30, 195-31, 195-32, 195-33, 195-34, 195-35, 195-36, 195-37, 195-38, 195-39, 195-40, 195-41, 195-42, 195-43, 195-44, 195-45, 195-46, 195-47, 195-48, 195-49, 195-50, 195-51, 195-52, 195-53, 195-54, 195-55, 195-56, 195-57, 195-58, 195-59, 195-60, 195-61, 195-62, 195-63, 195-64, 195-65, 195-66, 195-67, 195-68, 195-69, 195-70, 195-71, 195-72, 195-73, 195-74, 195-75, 195-76, 195-77, 195-78, 195-79, 195-80, 195-81, 195-82, 195-83, 195-84, 195-85, 195-86, 195-87, 195-88, 195-89, 195-90, 195-91, 195-92, 195-93, 195-94, 195-95, 195-96, 195-97,
196-1, 196-2, 196-3, 196-4, 196-5, 196-6, 196-7, 196-8, 196-9, 196-10, 196-11, 196-12, 196-13, 196-14, 196-15, 196-16, 196-17, 196-18, 196-19, 196-20, 196-21, 196-22, 196-23, 196-24, 196-25, 196-26, 196-27, 196-28, 196-29, 196-30, 196-31, 196-32, 196-33, 196-34, 196-35, 196-36, 196-37, 196-38, 196-39, 196-40, 196-41, 196-42, 196-43, 196-44, 196-45, 196-46, 196-47, 196-48, 196-49, 196-50, 196-51, 196-52, 196-53, 196-54, 196-55, 196-56, 196-57, 196-58, 196-59, 196-60, 196-61, 196-62, 196-63, 196-64, 196-65, 196-66, 196-67, 196-68, 196-69, 196-70, 196-71, 196-72, 196-73, 196-74, 196-75, 196-76, 196-77, 196-78, 196-79, 196-80, 196-81, 196-82, 196-83, 196-84, 196-85, 196-86, 196-87, 196-88, 196-89, 196-90, 196-91, 196-92, 196-93, 196-94, 196-95, 196-96, 196-97,
197-1, 197-2, 197-3, 197-4, 197-5, 197-6, 197-7, 197-8, 197-9, 197-10, 197-11, 197-12, 197-13, 197-14, 197-15, 197-16, 197-17, 197-18, 197-19, 197-20, 197-21, 197-22, 197-23, 197-24, 197-25, 197-26, 197-27, 197-28, 197-29, 197-30, 197-31, 197-32, 197-33, 197-34, 197-35, 197-36, 197-37, 197-38, 197-39, 197-40, 191-41, 197-42, 197-43, 197-44, 197-45, 197-46, 197-47, 197-48, 197-49, 197-50, 197-51, 197-52, 197-53, 197-54, 197-55, 197-56, 197-57, 197-58, 197-59, 197-60, 197-61, 197-62, 197-63, 197-64, 197-65, 197-66, 197-67, 197-68, 197-69, 197-70, 197-71, 197-72, 197-73, 197-74, 197-75, 197-76, 197-77, 197-78, 197-79, 197-80, 197-81, 197-82, 197-83, 197-84, 197-85, 197-86, 197-87, 197-88, 197-89, 197-90, 197-91, 197-92, 197-93, 197-94, 197-95, 197-96, 197-97,
198-1, 198-2, 198-3, 198-4, 198-5, 198-6, 198-7, 198-8, 198-9, 198-10, 198-11, 198-12, 198-13, 198-14, 198-15, 198-16, 198-17, 198-18, 198-19, 198-20, 198-21, 198-22, 198-23, 198-24, 198-25, 198-26, 198-27, 198-28, 198-29, 198-30, 198-31, 198-32, 198-33, 198-34, 198-35, 198-36, 198-37, 198-38, 198-39, 198-40, 198-41, 198-42, 198-43, 198-44, 198-45, 198-46, 198-47, 198-48, 198-49, 198-50, 198-51, 198-52, 198-53, 198-54, 198-55, 198-56, 198-57, 198-58, 198-59, 198-60, 198-61, 198-62, 198-63, 198-64, 198-65, 198-66, 198-67, 198-68, 198-69, 198-70, 198-71, 198-72, 198-73, 198-74, 198-75, 198-76, 198-77, 198-78, 198-79, 198-80, 198-81, 198-82, 198-83, 198-84, 198-85, 198-86, 198-87, 198-88, 198-89, 198-90, 198-91, 198-92, 198-93, 198-94, 198-95, 198-96, 198-97,
199-1, 199-2, 199-3, 199-4, 199-5, 199-6, 199-7, 199-8, 199-9, 199-10, 199-11, 199-12, 199-13, 199-14, 199-15, 199-16, 199-17, 199-18, 199-19, 199-20, 199-21, 199-22, 199-23, 199-24, 199-25, 199-26, 199-27, 199-28, 199-29, 199-30, 199-31, 199-32, 199-33, 199-34, 199-35, 199-36, 199-37, 199-38, 199-39, 199-40, 199-41, 199-42, 199-43, 199-44, 199-45, 199-46, 199-47, 199-48, 199-49, 199-50, 199-51, 199-52, 199-53, 199-54, 199-55, 199-56, 199-57, 199-58, 199-59, 199-60, 199-61, 199-62, 199-63, 199-64, 199-65, 199-66, 199-67, 199-68, 199-69, 199-70, 199-71, 199-72, 199-73, 199-74, 199-75, 199-76, 199-77, 199-78, 199-79, 199-80, 199-81, 199-82, 199-83, 199-84, 199-85, 199-86, 199-87, 199-88, 199-89, 199-90, 199-91, 199-92, 199-93, 199-94, 199-95, 199-96, 199-97,
202-1, 202-2, 202-3, 202-4, 202-5, 202-6, 202-7, 202-8, 202-9, 202-10, 202-11, 202-12, 202-13, 202-14, 202-15, 202-16, 202-17, 202-18, 202-19, 202-20, 202-21, 202-22, 202-23, 202-24, 202-25, 202-26, 202-27, 202-28, 202-29, 202-30, 202-31, 202-32, 202-33, 202-34, 202-35, 202-36, 202-37, 202-38, 202-39, 202-40, 202-41, 202-42, 202-43, 202-44, 202-45, 202-46, 202-47, 202-48, 202-49, 202-50, 202-51, 202-52, 202-53, 202-54, 202-55, 202-56, 202-57, 202-58, 202-59, 202-60, 202-61, 202-62, 202-63, 202-64, 202-65, 202-66, 202-67, 202-68, 202-69, 202-70, 202-71, 202-72, 202-73, 202-74, 202-75, 202-76, 202-77, 202-78, 202-79, 202-80, 202-81, 202-82, 202-83, 202-84, 202-85, 202-86, 202-87, 202-88, 202-89, 202-90, 202-91, 202-92, 202-93, 202-94, 202-95, 202-96, 202-97,
205-1, 205-2, 205-3, 205-4, 205-5, 205-6, 205-7, 205-8, 205-9, 205-10, 205-11, 205-12, 205-13, 205-14, 205-15, 205-16, 205-17, 205-18, 205-19, 205-20, 205-21, 205-22, 205-23, 205-24, 205-25, 205-26, 205-27, 205-28, 205-29, 205-30, 205-31, 205-32, 205-33, 205-34, 205-35, 205-36, 205-37, 205-38, 205-39, 205-40, 205-41, 205-42, 205-43, 205-44, 205-45, 205-46, 205-47, 205-48, 205-49, 205-50, 205-51, 205-52, 205-53, 205-54, 205-55, 205-56; 205-57, 205-58, 205-59, 205-60, 205-61, 205-62, 205-63, 205-64, 205-65, 205-66, 205-67, 205-68, 205-69, 205-70, 205-71, 205-72, 205-73, 205-74, 205-75, 205-76, 205-77, 205-78, 205-79, 205-80, 205-81, 205-82, 205-83, 205-84, 205-85, 205-86, 205-87, 205-88, 205-89, 205-90, 205-91, 205-92, 205-93, 205-94, 205-95, 205-96, 205-97,
208-1, 208-2, 208-3, 208-4, 208-5, 208-6, 208-7, 208-8, 208-9, 208-10, 208-11, 208-12, 208-13, 208-14, 208-15, 208-16, 208-17, 208-18, 208-19, 208-20, 208-21, 208-22, 208-23, 208-24, 208-25, 208-26, 208-27, 208-28, 208-29, 208-30, 208-31, 208-32, 208-33, 208-34, 208-35, 208-36, 208-37, 208-38, 208-39, 208-40, 208-41, 208-42, 208-43, 208-44, 208-45, 208-46, 208-47, 208-48, 208-49, 208-50, 208-51, 208-52, 208-53, 208-54, 208-55, 208-56, 208-57, 208-58, 208-59, 208-60, 208-61, 208-62, 208-63, 208-64, 208-65, 208-66, 208-67, 208-68, 208-69, 208-70, 208-71, 208-72, 208-73, 208-74, 208-75, 208-76, 208-77, 208-78, 208-79, 208-80, 208-81, 208-82, 208-83, 208-84, 208-85, 208-86, 208-87, 208-88, 208-89, 208-90, 208-91, 208-92, 208-93, 208-94, 208-95, 208-96, 208-97,
211-1, 211-2, 211-3, 211-4, 211-5, 211-6, 211-7, 211-8, 211-9,211-10, 211-11, 211-12, 211-13, 211-14, 211-15, 211-16, 211-17, 211-18, 211-19, 211-20, 211-21, 211-22, 211-23, 211-24, 211-25, 211-26, 211-27, 211-28, 211-29, 211-30, 211-31, 211-32, 211-33, 211-34, 211-35, 211-36, 211-37, 211-38, 211-39, 211-40, 211-41, 211-42, 211-43, 211-44, 211-45, 211-46, 211-47, 211-48, 211-49, 211-50, 211-51, 211-52, 211-53, 211-54, 211-55, 211-56, 211-57, 211-58, 211-59, 211-60, 211-61, 211-62, 211-63, 211-64, 211-65, 211-66, 211-67, 211-68, 211-69, 211-70, 211-71, 211-72, 211-73, 211-74, 211-75, 211-76, 211-77, 211-78, 211-79, 211-80, 211-81, 211-82, 211-83, 211-84, 211-85, 211-86, 211-87, 211-88, 211-89, 211-90, 211-91, 211-92, 211-93, 211-94, 211-95, 211-96, 211-97,
214-1, 214-2, 214-3, 214-4, 214-5, 214-6, 214-7, 214-8, 214-9, 214-10, 214-11, 214-12, 214-13, 214-14, 214-15, 214-16, 214-17, 214-18, 214-19, 214-20, 214-21, 214-22, 214-23, 214-24, 214-25, 214-26, 214-27, 214-28, 214-29, 214-30, 214-31, 214-32, 214-33, 214-34, 214-35, 214-36, 214-37, 214-38, 214-39, 214-40, 214-41, 214-42, 214-43, 214-44, 214-45, 214-46, 214-47, 214-48, 214-49, 214-50, 214-51, 214-52, 214-53, 214-54, 214-55, 214-56, 214-57, 214-58, 214-59, 214-60, 214-61, 214-62, 214-63, 214-64, 214-65, 214-66, 214-67, 214-68, 214-69, 214-70, 214-71, 214-72, 214-73, 214-74, 214-75, 214-76, 214-77, 214-78, 214-79, 214-80, 214-81, 214-82, 214-83, 214-84, 214-85, 214-86, 214-87, 214-88, 214-89, 214-90, 214-91, 214-92, 214-93, 214-94, 214-95, 214-96, 214-97,
217-1, 217-2, 217-3, 217-4, 217-5, 217-6, 217-7, 217-8, 217-9, 217-10, 217-11, 217-12, 217-13, 217-14, 217-15, 217-16, 217-17, 217-18, 217-19, 217-20, 217-21, 217-22, 217-23, 217-24, 217-25, 217-26, 217-27, 217-28, 217-29, 217-30, 217-31, 217-32, 217-33, 217-34, 217-35, 217-36, 217-37, 217-38, 217-39, 217-40, 217-41, 217-42, 217-43, 217-44, 217-45, 217-46, 217-47, 217-48, 217-49, 217-50, 217-51, 217-52, 217-53, 217-54, 217-55, 217-56, 217-57, 217-58, 217-59, 217-60, 217-61, 217-62, 217-63, 217-64, 217-65, 217-66, 217-67, 217-68, 217-69, 217-70, 217-71, 217-72, 217-73, 217-74, 217-75, 217-76, 217-77, 217-78, 217-79, 217-80, 217-81, 217-82, 217-83, 217-84, 217-85, 217-86, 217-87, 217-88, 217-89, 217-90, 217-91, 217-92, 217-93, 217-94, 217-95, 217-96, 217-97,
220-1, 220-2, 220-3, 220-4, 220-5, 220-6, 220-7, 220-8, 220-9, 220-10, 220-11, 220-12, 220-13, 220-14, 220-15, 220-16, 220-17, 220-18, 220-19, 220-20, 220-21, 220-22, 220-23, 220-24, 220-25, 220-26, 220-27, 220-28, 220-29, 220-30, 220-31, 220-32, 220-33, 220-34, 220-35, 220-36, 220-37, 220-38, 220-39, 220-40, 220-41, 220-42, 220-43, 220-44, 220-45, 220-46, 220-47, 220-48, 220-49, 220-50, 220-51, 220-52, 220-53, 220-54, 220-55, 220-56, 220-57, 220-58, 220-59, 220-60, 220-61, 220-62, 220-63, 220-64, 220-65, 220-66, 220-67, 220-68, 220-69, 220-70, 220-71, 220-72, 220-73, 220-74, 220-75, 220-76, 220-77, 220-78, 220-79, 220-80, 220-81, 220-82, 220-83, 220-84, 220-85, 220-86, 220-87, 220-88, 220-89, 220-90, 220-91, 220-92, 220-93, 220-94, 220-95, 220-96, 220-97,
223-1, 223-2, 223-3, 223-4, 223-5, 223-6, 223-7, 223-8, 223-9, 223-10, 223-11, 223-12, 223-13, 223-14, 223-15, 223-16, 223-17, 223-18, 223-19, 223-20, 223-21, 223-22, 223-23, 223-24, 223-25, 223-26, 223-27, 223-28, 223-29, 223-30, 223-31, 223-32, 223-33, 223-34, 223-35, 223-36, 223-37, 223-38, 223-39, 223-40, 223-41, 223-42, 223-43, 223-44, 223-45, 223-46, 223-47, 223-48, 223-49, 223-50, 223-51, 223-52, 223-53, 223-54, 223-55, 223-56, 223-57, 223-58, 223-59, 223-60, 223-61, 223-62, 223-63, 223-64, 223-65, 223-66, 223-67, 223-68, 223-69, 223-70, 223-71, 223-72, 223-73, 223-74, 223-75, 223-76, 223-77, 223-78, 223-79, 223-80, 223-81, 223-82, 223-83, 223-84, 223-85, 223-86, 223-87, 223-88, 223-89, 223-90, 223-91, 223-92, 223-93, 223-94, 223-95, 223-96, 223-97,
225-1, 225-2, 225-3, 225-4, 225-5, 225-6, 225-7, 225-8, 225-9, 225-10, 225-11, 225-12, 225-13, 225-14, 225-15, 225-16, 225-17, 225-18, 225-19, 225-20, 225-21, 225-22, 225-23, 225-24, 225-25, 225-26, 225-27, 225-28, 225-29, 225-30, 225-31, 225-32, 225-33, 225-34, 225-35, 225-36, 225-37, 225-38, 225-39, 225-40, 225-41, 225-42, 225-43, 225-44, 225-45, 225-46, 225-47, 225-48, 225-49, 225-50, 225-51, 225-52, 225-53, 225-54, 225-55, 225-56, 225-57, 225-58, 225-59, 225-60, 225-61, 225-62, 225-63, 225-64, 225-65, 225-66, 225-67, 225-68, 225-69, 225-70, 225-71, 225-72, 225-73, 225-74, 225-75, 225-76, 225-77, 225-78, 225-79, 225-80, 225-81, 225-82, 225-83, 225-84, 225-85, 225-86, 225-87, 225-88, 225-89, 225-90, 225-91, 225-92, 225-93, 225-94, 225-95, 225-96, 225-97,
226-1, 226-2, 226-3, 226-4, 226-5, 226-6, 226-7, 226-8, 226-9, 226-10, 226-11, 226-12, 226-13, 226-14, 226-15, 226-16, 226-17, 226-18, 226-19, 226-20, 226-21, 226-22, 226-23, 226-24, 226-25, 226-26, 226-27, 226-28, 226-29, 226-30, 226-31, 226-32, 226-33, 226-34, 226-35, 226-36, 226-37, 226-38, 226-39, 226-40, 226-41, 226-42, 226-43, 226-44, 226-45, 226-46, 226-47, 226-48, 226-49, 226-50, 226-51, 226-52, 226-53, 226-54, 226-55, 226-56, 226-57, 226-58, 226-59, 226-60, 226-61, 226-62, 226-63, 226-64, 226-65, 226-66, 226-67, 226-68, 226-69, 226-70, 226-71, 226-72, 226-73, 226-74, 226-75, 226-76, 226-77, 226-78, 226-79, 226-80, 226-81, 226-82, 226-83, 226-84, 226-85, 226-86, 226-87, 226-88, 226-89, 226-90, 226-91, 226-92, 226-93, 226-94, 226-95, 226-96, 226-97,
227-1, 227-2, 227-3, 227-4, 227-5, 227-6, 227-7, 227-8, 227-9, 227-10, 227-11, 227-12, 227-13, 227-14, 227-15, 227-16, 227-17, 227-18, 227-19, 227-20, 227-21, 227-22, 227-23, 227-24, 227-25, 227-26, 227-27, 227-28, 227-29, 227-30, 227-31, 227-32, 227-33, 227-34, 227-35, 227-36, 227-37, 227-38, 227-39, 227-40, 227-41, 227-42, 227-43, 227-44, 227-45, 227-46, 227-47, 227-48, 227-49, 227-50, 227-51, 227-52, 227-53, 227-54, 227-55, 227-56, 227-57, 227-58, 227-59, 227-60, 227-61, 227-62, 227-63, 227-64, 227-65, 227-66, 227-67, 227-68, 227-69, 227-70, 227-71, 227-72, 227-73, 227-74, 227-75, 227-76, 227-77, 227-78, 227-79, 227-80, 227-81, 227-82, 227-83, 227-84, 227-85, 227-86, 227-87, 227-88, 227-89, 227-90, 227-91, 227-92, 227-93, 227-94, 227-95, 227-96, 227-97,
228-1, 228-2, 228-3, 228-4, 228-5, 228-6, 228-7, 228-8, 228-9, 228-10, 228-11, 228-12, 228-13, 228-14, 228-15, 228-16, 228-17, 228-18, 228-19, 228-20, 228-21, 228-22, 228-23, 228-24, 228-25, 228-26, 228-27, 228-28, 228-29, 228-30, 228-31, 228-32, 228-33, 228-34, 228-35, 228-36, 228-37, 228-38, 228-39, 228-40, 228-41, 228-42, 228-43, 228-44, 228-45, 228-46, 228-47, 228-48, 228-49, 228-50, 228-51, 228-52, 228-53, 228-54, 228-55, 228-56, 228-57, 228-58, 228-59, 228-60, 228-61, 228-62, 228-63, 228-64, 228-65, 228-66, 228-67, 228-68, 228-69, 228-70, 228-71, 228-72, 228-73, 228-74, 228-75, 228-76, 228-77, 228-78, 228-79, 228-80, 228-81, 228-82, 228-83, 228-84, 228-85, 228-86, 228-87, 228-88, 228-89, 228-90, 228-91, 228-92, 228-93, 228-94, 228-95, 228-96, 228-97,
229-1, 229-2, 229-3, 229-4, 229-5, 229-6, 229-7, 229-8, 229-9, 229-10, 229-11, 229-12, 229-13, 229-14, 229-15, 229-16, 229-17, 229-18, 229-19, 229-20, 229-21, 229-22, 229-23, 229-24, 229-25, 229-26, 229-27, 229-28, 229-29, 229-30, 229-31, 229-32, 229-33, 229-34, 229-35, 229-36, 229-37, 229-38, 229-39, 229-40, 229-41, 229-42, 229-43, 229-44, 229-45, 229-46, 229-47, 229-48, 229-49, 229-50, 229-51, 229-52, 229-53, 229-54, 229-55, 229-56, 229-57, 229-58, 229-59, 229-60, 229-61, 229-62, 229-63, 229-64, 229-65, 229-66, 229-67, 229-68, 229-69, 229-70, 229-71, 229-72, 229-73, 229-74, 229-75, 229-76, 229-77, 229-78, 229-79, 229-80, 229-81, 229-82, 229-83, 229-84, 229-85, 229-86, 229-87, 229-88, 229-89, 229-90, 229-91, 229-92, 229-93, 229-94, 229-95, 229-96, 229-97,
231-1, 231-2, 231-3, 231-4, 231-5, 231-6, 231-7, 231-8, 231-9, 231-10, 231-11, 231-12, 231-13, 231-14, 231-15, 231-16, 231-17, 231-18, 231-19, 231-20, 231-21, 231-22, 231-23, 231-24, 231-25, 231 -26, 231 -27, 231-28, 231-29, 231-30, 231-31, 231-32, 231-33, 231-34, 231-35, 231-36, 231-37, 231-38, 231-39, 231-40, 231-41, 231-42, 231-43, 231-44, 231-45, 231-46, 231-47, 231-48, 231-49, 231-50, 231-51, 231-52, 231-53, 231-54, 231-55, 231-56, 231-57, 231-58, 231-59, 231-60, 231-61, 231-62, 231-63, 231-64, 231-65, 231-66, 231-67, 231-68, 231-69, 231-70, 231-71, 231-72, 231-73, 231-74, 231-75, 231-76, 231-77, 231-78, 231-79, 231-80, 231-81, 231-82, 231-83, 231-84, 231-85, 231-86, 231-87, 231-88, 231-89, 231-90, 231-91, 231-92, 231-93, 231-94, 231-95, 231-96, 231-97,
232-1, 232-2, 232-3, 232-4, 232-5, 232-6, 232-7, 232-8, 232-9, 232-10, 232-11, 232-12, 232-13, 232-14, 232-15, 232-16, 232-17, 232-18, 232-19, 232-20, 232-21, 232-22, 232-23, 232-24, 232-25, 232-26, 232-27, 232-28, 232-29, 232-30, 232-31, 232-32, 232-33, 232-34, 232-35, 232-36, 232-37, 232-38, 232-39, 232-40, 232-41, 232-42, 232-43, 232-44, 232-45, 232-46, 232-47, 232-48, 232-49, 232-50, 232-51, 232-52, 232-53, 232-54, 232-55, 232-56, 232-57, 232-58, 232-59, 232-60, 232-61, 232-62, 232-63, 232-64, 232-65, 232-66, 232-67, 232-68, 232-69, 232-70, 232-71, 232-72, 232-73, 232-74, 232-75, 232-76, 232-77, 232-78, 232-79, 232-80, 232-81, 232-82, 232-83, 232-84, 232-85, 232-86, 232-87, 232-88, 232-89, 232-90, 232-91, 232-92, 232-93, 232-94, 232-95, 232-96, 232-97,
233-1, 233-2, 233-3, 233-4, 233-5, 233-6, 233-7, 233-8, 233-9, 233-10, 233-11, 233-12, 233-13, 233-14, 233-15, 233-16, 233-17, 233-18, 233-19, 233-20, 233-21, 233-22, 233-23, 233-24, 233-25, 233-26, 233-27, 233-28, 233-29, 233-30, 233-31, 233-32, 233-33, 233-34, 233-35, 233-36, 233-37, 233-38, 233-39, 233-40, 233-41, 233-42, 233-43, 233-44, 233-45, 233-46, 233-47, 233-48, 233-49, 233-50, 233-51, 233-52, 233-53, 233-54, 233-55, 233-56, 233-57, 233-58, 233-59, 233-60, 233-61, 233-62, 233-63, 233-64, 233-65, 233-66, 233-67, 233-68, 233-69, 233-70, 233-71, 233-72, 233-73, 233-74, 233-75, 233-76, 233-77, 233-78, 233-79, 233-80, 233-81, 233-82, 233-83, 233-84, 233-85, 233-86, 233-87, 233-88, 233-89, 233-90, 233-91, 233-92, 233-93, 233-94, 233-95, 233-96, 233-97,
234-1, 234-2, 234-3, 234-4, 234-5, 234-6, 234-7, 234-8, 234-9, 234-10, 234-11, 234-12, 234-13, 234-14, 234-15, 234-16, 234-17, 234-18, 234-19, 234-20, 234-21, 234-22, 234-23, 234-24, 234-25, 234-26, 234-27, 234-28, 234-29, 234-30, 234-31, 234-32, 234-33, 234-34, 234-35, 234-36, 234-37, 234-38, 234-39, 234-40, 234-41, 234-42, 234-43, 234-44, 234-45, 234-46, 234-47, 234-48, 234-49, 234-50, 234-51, 234-52, 234-53, 234-54, 234-55, 234-56, 234-57, 234-58, 234-59, 234-60, 234-61, 234-62, 234-63, 234-64, 234-65, 234-66, 234-67, 234-68, 234-69, 234-70, 234-71, 234-72, 234-73, 234-74, 234-75, 234-76, 234-77, 234-78, 234-79, 234-80, 234-81, 234-82, 234-83, 234-84, 234-85, 234-86, 234-87, 234-88, 234-89, 234-90, 234-91, 234-92, 234-93, 234-94, 234-95, 234-96, 234-97

Among the combinations of groups given in Table 1 above, we prefer those identified as Nos. 1, 4, 7, 163 and 199, and more prefer those identified as Nos. 1, 163 and 199.

Among the combinations of groups in Table 2 above, we prefer those identified as Nos. 1 to 8, 32 to 53, and 87 to 97, and more prefer those identified as Nos. 1, 38, 40, 41 and 42.

Among the compounds represented by the combinations of groups selected from Table 1 and Table 2, we prefer Compounds Nos. 1-1, 1-2, 1-3, 1-4, 1-5, 1-6, 1-7, 1-8, 1-32, 1-33, 1-34, 1-35, 1-36, 1-37, 1-38, 1-39, 1-40, 1-41, 1-42, 1-43, 1-44, 1-45, 1-48, 1-49, 1-50, 1-51, 1-52, 1-53, 7-1, 7-2, 7-3, 7-4, 7-5, 7-6, 7-7, 7-8, 7-32, 7-33, 7-34, 7-35, 7-36, 7-37, 7-38, 7-39, 7-40, 7-41, 7-42, 7-43, 7-44, 7-45, 7-48, 7-49, 7-50, 7-51, 7-52, 7-53, 163-1, 163-38, 163-39, 16340, 163-41, 163-42, 199-1, 199-38, 199-39, 199-40, 199-41 and 199-42.

The most preferred compounds are Compounds No.:
1-1. 5-acetamido-4-guanidino-2,3,4,5,7-pentadeoxy-7-fluoro-D-glycero-D-galacto-non-2-enopyranosoic acid; IUPAC name: 5-acetamido-6-(1-fluoro-2,3-dihydroxypropyl)-4-(R)-guanidino-2-(S)-3-(R)-dihydropyran-2-carboxylic acid;
1-40. 5-acetamido-4-guanidino-9-O-dodecanoyl-2,3,4,5,7-pentadeoxy-7-fluoro-D-glycero-D-galacto-non-2-enopyranosoic acid; IUPAC name: 5-acetamido-6-(1-fluoro-2-hydroxy-3-dodecanoyloxypropyl)-4-(R)-guanidino-2-(S)-3-(R)-dihydropyran-2-carboxylic acid;
1-41. 5-acetamido-4-guanidino-9-O-myristoyl-2,3,4,5,7-pentadeoxy-7-fluoro-D-glycero-D-galacto-non-2-enopyranosoic acid; IUPAC name: 5-acetamido-6-(1-fluoro-2-hydroxy-3-myristoyloxypropyl)-4-(R)-guanidino-2-(S)-3-(R)-dihydropyran-2-carboxylic acid;
1-42. 5-acetamido-4-guanidino-9-O-palmitoyl-2,3,4,5,7-pentadeoxy-7-fluoro-D-glycero-D-galacto-non-2-enopyranosoic acid; IUPAC name: 5-acetamido-6-(1-fluoro-2-hydroxy-3-palmitoyloxypropyl)-4-(R)-guanidino-2-(S)-3-(R)-dihydropyran-2-carboxylic acid;
163-1. 5-acetamido-4-guanidino-2,3,4,5,7-pentadeoxy-7-methoxy-D-glycero-D-galacto-non-2-enopyranosoic acid; IUPAC name: 5-acetamido-6-(2,3-dihydroxy-1-methoxypropyl)-4-(R)-guanidino-2-(S)-3-(R)-dihydropyran-2-carboxylic acid;
163-41. 5-acetamido-4-guanidino-9-O-myristoyl-2,3,4,5,7-pentadeoxy-7-methoxy-D-glycero-D-galacto-non-2-enopyranosoic acid: 5-acetamido-6-(2-hydroxy-1-methoxy-3-myristoyloxypropyl)-4-(R)-guanidino-2-(S)-3-(R)-dihydropyran-2-carboxylic acid;
199-1. 5-acetamido-4-guanidino-2,3,4,5,7-pentadeoxy-7-ethoxy-D-glycero-D-galacto-non-2-enopyranosoic acid; IUPAC name: 5-acetamido-6-(1-ethoxy-2,3-dihydroxypropyl)-4-(R)-guanidino-2-(S)-3-(R)-dihydropyran-2-carboxylic acid;
199-38. 5-acetamido-4-guanidine-9-O-octanoyl-2,3,4,5,7-pentadeoxy-7-ethoxy-D-glycero-D-galacto-non-2-enopyranosoic acid; IUPAC name: 5-acetamido-6-(1-ethoxy-2-hydroxy-3-octanoyloxypropyl)-4-(R)-guanidino-2-(S)-3-(R)-dihydropyran-2-carboxylic acid;
199-40. 5-acetamido-4-guanidino-9-O-dodecanoyl-2,3,4,5,7-pentadeoxy-7-ethoxy-D-glycero-D-galacto-non-2-enopyranosoic acid; IUPAC name: 5-acetamido-6-(1-ethoxy-2-hydroxy-3-dodecanoyloxypropyl)-4-(R)-guanidino-2-(S)-3-(R)-dihydropyran-2-carboxylic acid;
199-41. 5-acetamido-4-guanidino-9-O-myristoyl-2,3,4,5,7-pentadeoxy-7-ethoxy-D-glycero-D-galacto-non-2-enopyranosoic acid; IUPAC name: 5-acetamido-6-(1-ethoxy-2-hydroxy-3-myristoyloxypropyl)-4-(R)-guanidino-2-(S)-3-(R)-dihydropyran-2-carboxylic acid; and
199-42. 5-acetamido-4-guanidino-9-O-palmitoyl-2,3,4,5,7-pentadeoxy-7-ethoxy-D-glycero-D-galacto-non-2-enopyranosoic acid; IUPAC name: 5-acetamido-6-(1-ethoxy-2-hydroxy-3-palmitoyloxypropyl)-4-(R)-guanidino-2-(S)-3-(R)-dihydropyran-2-carboxylic acid;.

The compounds of the present invention may be prepared by a variety of processes well known in the art for the preparation of compounds of this type, for example as illustrated by the following Methods A, B and C.

The compound of formula (2), which is used as a starting material in Method A or B, can be prepared by the following Method G.

The compound of formula (5), which is used as a starting material in Method C, can be prepared by the following Method H.

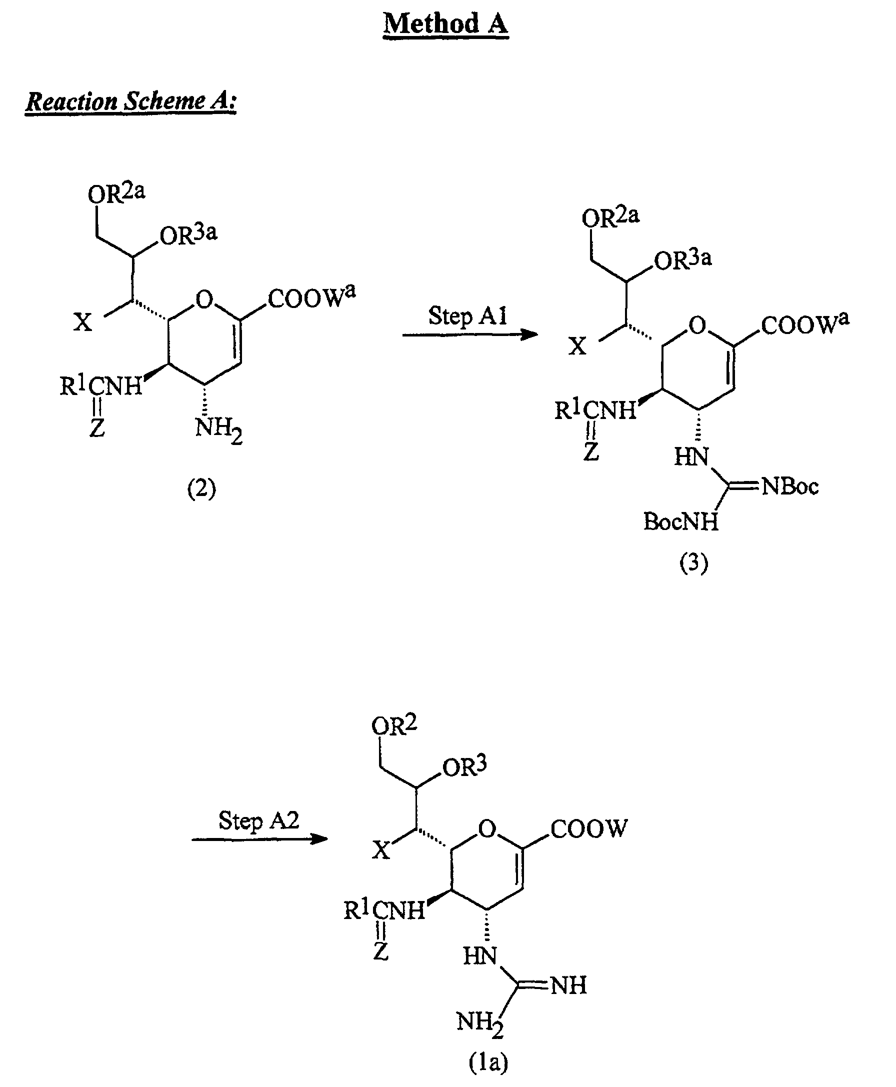

In the above formulae:
R¹, R², R³, X and Z are as defined above;
R^{2a} represents any of the groups or atoms represented by R² or a hydroxy-protecting group, preferably a t-butyldimethylsilyl group;
R^{3a} represents any of the groups or atoms represented by R³ or a hydroxy-protecting group, preferably a t-butyldimethylsilyl group;
W represents a hydrogen atom or an ester residue;
W^{a} represents any of the groups or atoms represented by W or a carboxy-protecting group, preferably an allyl group, a methoxymethyl group, a methylthiomethyl group, a [2-(trimethylsilyl)ethoxy]methoxy group or a diphenylmethyl group, more preferably a diphenylmethyl group; and
Boc represents a t-butoxycarbonyl group.

In this Method, a compound of formula (1a) is prepared by reacting a compound of formula (2) with N,N'-di-t-butoxycarbonylthiourea to give a compound of formula (3), which is then deprotected.

### Step A1

In this Step, a compound of formula (3) is prepared by reacting the compound of formula (2) with N,N'-di-t-butoxycarbonylthiourea in the presence of a base and mercuric chloride and in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene and xylene; ethers, such as diethyl ether, diisopropyl ether, tetrahydrofuran, dioxane, dimethoxyethane and diethylene glycol dimethyl ether; and amides, such as N,N-dimethylacetamide, and dimethylformamide. Of these, we prefer the amides, particularly N,N-dimethylacetamide or dimethylformamide.

There is likewise no particular restriction on the nature of the bases used, and any base commonly used in reactions of this type may equally be used here. Examples of such bases include: organic bases, such as triethylamine or dimethylaminopyridine.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents, base and solvent employed. However, provided that the reaction is effected under the preferred conditions outlined above, a period of from 1 hour to 24 hours, more preferably from 5 to 10 hours, will usually suffice.

After completion of the reaction, the desired compound can be obtained by conventional means. For example, one suitable recovery procedure comprises: filtering the reaction solution under reduced pressure to remove the insolubles; adding a water-immiscible organic solvent, such as ethyl acetate, thereto, separating the organic layer containing the desired compound after washing with water; and distilling off the solvent after the organic layer is dried, for example over anhydrous magnesium sulphate.

The desired compound can, if desired, be further purified by recrystallization or by the various forms of chromatography, such as column chromatography or preparative thin layer chromatography.

### Step A2

In this Step, a compound of formula (1a), which is a compound of the present invention, is prepared by treating the compound of formula (3) with a reagent which can remove a t-butoxycarbonyl group, in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; water; or a mixture of any two or more thereof.

There is likewise no particular restriction on the nature of the reagents used to remove the t-butoxycarbonyl group, and any reagent commonly used in reactions of this type may equally be used here. Examples of such reagents include acids, and any acid commonly used as an acid catalyst may be used, for example a Brønsted acid, such as an inorganic acid (e.g. hydrochloric acid, hydrobromic acid, sulphuric acid, perchloric acid or phosphoric acid) or an organic acid (e.g. acetic acid, formic acid, oxalic acid, methanesulphonic acid, p-toluenesulphonic acid, trifluoroacetic acid or trifluoromethanesulphonic acid), preferably an organic acid (particularly acetic acid or trifluoroacetic acid).

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: neutralising the reaction solution and purifying the residue obtained by distilling off the solvent under reduced pressure by means of silica gel chromatography.

Where R^{2a} or R^{3a} represents a hydroxy-protecting group or W^{a} represents a carboxy-protecting group , a compound of the present invention can be prepared by deprotecting these protecting groups further.

The reaction employed for the removal of the protecting group will vary, depending on the nature of the protecting group. However it can be carried out by conventional means, for example, by the method described in "Protective Groups in Organic Synthesis", Second Edition (written by Greene and Wuts, John Wiley & Sons, Inc., published in 1991).

Where the hydroxy-protecting group is a trialkylsilyl group, such as a t-butyldimethylsilyl group, acetic acid in a mixture of water and tetrahydrofuran or tetrabutylammonium fluoride in tetrahydrofuran can preferably be used. Where the carboxy-protecting group is a diphenylmethyl group, catalytic reduction using hydrogen gas and a catalyst such as palladium black in a mixture of methanol and tetrahydrofuran, trifluoroacetic acid in phenol, acetic acid as an acid and a solvent or trifluoroboran-diethyl ether complex in acetic acid can be used.

### Method B

In this Method, a compound of formula (1) is prepared by reacting the compound of formula (2) with ammonia or a hydroxylamine which may be substituted by an alkyl group having from 1 to 4 carbon atoms at the amino moiety, if desired, followed by deprotection. 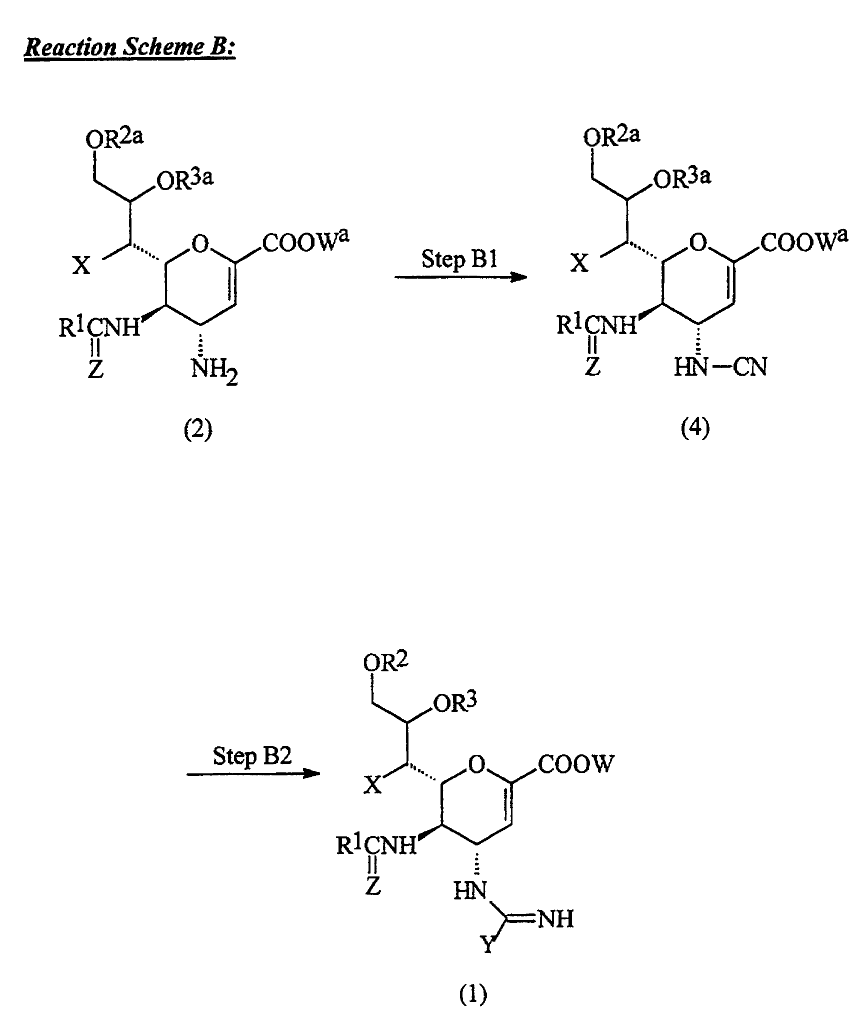 In the above formulae, R¹, R², R³, R^{2a}, R^{3a}, X, Y, W, Z and W^{a} are as defined above.

### Step B1

In this Step, a compound of formula (4) is prepared by reacting the compound of formula (2) with a cyanating agent in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol, ethanol, propanol, isopropanol, butanol, isobutanol, t-butanol, isoamyl alcohol, diethylene glycol, glycerin, octanol, cyclohexanol and ethylene glycol monomethyl ether (methyl Cellosolve - "Cellosolve" is a trade mark); amides, such as formamide, dimethylformamide, N,N-dimethylacetamide, N-methyl-2-pyrrolidone, N-methylpyrrolidinone and hexamethylphosphoric triamide and sulphoxides, such as dimethyl sulphoxide and sulpholane. Of these, we prefer the alcohols (particularly methanol).

There is likewise no particular restriction on the nature of the cyanating agents used, and any cyanating agent commonly used in reactions of this type may equally be used here. Examples of such cyanating agents include cyanogen bromide, in which case, sodium acetate is simultaneously used as a base.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 40°C. The time required for the reaction may also vary widely, depending on many factors, notably the reaction temperature and the nature of the reagents, base and solvent employed. However, provided that the reaction is effected under the preferred conditions outlined above, a period of from 15 minutes to 10 hours, more preferably from 1 to 5 hours, will usually suffice.

After completion of the reaction the desired compound can, for example, be obtained by using silica gel chromatography to purify the residue obtained by distilling off the solvent.

### Step B2

In this Step, a compound of formula (1), which is a compound of the present invention, is prepared by reacting the compound of formula (4) with ammonia or a hydroxylamine which may be substituted by an alkyl group having from 1 to 4 carbon atoms at the amino moiety, if desired, followed by deprotection.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include alcohols (particularly methanol).

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 40°C. The time required for the reaction may also vary widely, depending on many factors, notably the reaction temperature and the nature of the reagents and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises using silica gel chromatography to purify the residue obtained by distilling off the solvent.

Where R^{2a} or R^{3a} represents a hydroxy-protecting group or W^{a} represents a carboxy-protecting group , a compound of the present invention can be prepared by the subsequent removal of these protecting groups as described in Method A.

### Method C

In this Method, a compound of formula (1c) is prepared by reacting the compound of formula (5) with an acylating agent, followed by deprotection.

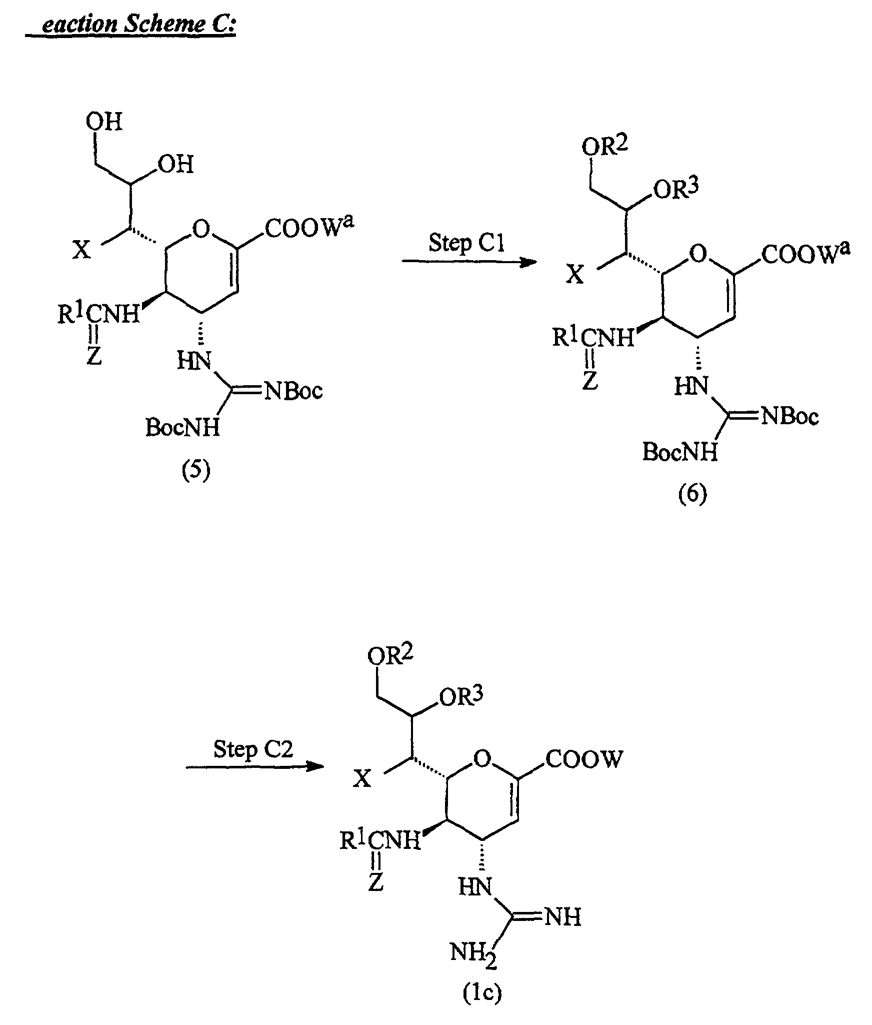

In the above formulae:
R¹, R², R³, W, X, Z and W^{a} are as defined above.

### Step C1

In this Step, a compound of formula (6) is prepared by introducing a desired acyl group into the compound of formula (5) in an inert solvent.

The acylation method may be carried out by any of the following Processes 1, 2 and 3.

### Process 1

In this Process, a compound of formula (5) is reacted with a compound of formula RCO-L or with a compound of formula RCO-O-COR, in which R represents an alkyl group having from 1 to 24 carbon atoms; and L represents a leaving group. There is no particular restriction on the nature of the leaving groups used, and any nucleophilic leaving group commonly used in reactions of this type may equally be used here. Examples of such leaving groups include: halogen atoms, such as the chlorine, bromine and iodine atoms; alkoxycarbonyloxy groups having from 1 to 6 carbon atoms in the alkoxy part, such as the methoxycarbonyloxy and ethoxycarbonyloxy groups; halogenated alkanoyloxy groups, such as the chloroacetoxy, dichloroacetoxy, trichloroacetoxy and trifluoroacetoxy groups; alkanesulphonyloxy groups having from 1 to 6 carbon atoms in the alkyl part, such as the methanesulphonyloxy and ethanesulphonyloxy groups; halogenated alkanesulphonyloxy groups having from 1 to 6 carbon atoms in the alkyl part, such as the trifluoromethanesulphonyloxy and pentafluoroethanesulphonyloxy groups; and arylsulphonyloxy groups, such as the benzenesulphonyloxy, p-toluenesulphonyloxy and p-nitrobenzenesulphonyloxy groups. Of these, we prefer the halogen atoms, halogenated alkanesulphonyloxy groups and arylsulphonyloxy groups.

The reaction may take place in the presence or absence of a base and in a solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane and heptane; aromatic hydrocarbons, such as benzene, toluene and xylene; halogenated hydrocarbons, such as methylene chloride, chloroform, carbon tetrachloride, dichloroethane, chlorobenzene and dichlorobenzene; esters, such as ethyl formate, ethyl acetate, propyl acetate, butyl acetate and diethyl carbonate; ethers, such as diethyl ether, diisopropyl ether, tetrahydrofuran, dioxane, dimethoxyethane and diethylene glycol dimethyl ether; nitriles, such as acetonitrile and isobutyronitrile; and amides, such as formamide, dimethylformamide, N,N-dimethylacetamide, N-methyl-2-pyrrolidone, N-methylpyrrolidinone and hexamethylphosphoric triamide.

There is likewise no particular restriction on the nature of the bases used, and any base commonly used in reactions of this type may equally be used here. Examples of such bases include: organic bases, such as N-methylmorpholine, triethylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline and N,N-diethylaniline.

4-(N,N-Dimethylamino)pyridine and 4-pyrrolidinopyridine can be used in a catalytic amount in combination with one or more other bases. In addition, it may facilitate the reaction to carry it out in the presence of one or more of: quaternary ammonium salts, such as benzyltriethylammonium chloride and tetrabutylammonium chloride; and crown ethers, such as dibenzo-18-crown-6.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C to the reflux temperature of the solvent used, more preferably from 0°C to the reflux temperature of the solvent used. The time required for the reaction may also vary widely, depending on many factors, notably the reaction temperature and the nature of the reagents, base and solvent employed. However, provided that the reaction is effected under the preferred conditions outlined above, a period of from 10 minutes to 3 days, more preferably from 1 hour to 6 hours, will usually suffice.

### Process 2

In this Process, a compound of formula: RCOOH [in which R is as defined above] is reacted with the compound of formula (5) in the presence of an esterifying agent and in the presence or absence of a catalytic amount of a base in a solvent. The reaction may also be effected in the presence of a condensing agent.

There is no particular restriction on the nature of the esterifying agents used, and any esterifying agent commonly used in reactions of this type may equally be used here. Examples of such esterifying agents include active esters, especially: alkyl haloformates, such as methyl chloroformate and ethyl chloroformate; and cyanophosphoric acid diesters, such as diethyl cyanophosphate. Esterification with such active esters preferably takes place in the presence of at least one condensing agent. Specific examples of such condensing agents include: N-hydroxy derivatives, such as N-hydroxysuccinimide, 1-hydroxybenzotriazole and N-hydroxy-5-norbornene-2,3-dicarboximide; disulphide compounds, such as 2,2'-dipyridyl disulphide; succinic acid compounds, such as N,N'-disuccinimidyl carbonate; phosphinic chloride compounds, such as N,N'-bis(2-oxo-3-oxazolidinyl)phosphinic chloride; oxalate derivatives, such as N,N'-disuccinimidyl oxalate (DSO), N,N'-diphthalimidoxalate (DPO), N,N'-bis(norbornenylsuccinimidyl) oxalate (BNO), 1,1'-bis(benzotriazolyl)oxalate (BBTO), 1,1'-bis(6-chlorobenzotriazolyl)oxalate (BCTO) and 1,1'-bis(6-trifluoromethylbenzotriazolyl)oxalate (BTBO); triarylphosphines including triarylphosphines, such as triphenylphosphine, dialkyl azodicarboxylate-triarylphosphines in which the alkyl part has from 1 to 6 carbon atoms, such as diethyl azodicarboxylatetriphenylphosphine; N-alkyl-5-arylisoxazolium-3'-sulphonates in which the alkyl part has from 1 to 6 carbon atoms, such as N-ethyl-5-phenylisoxazolium-3'-sulphonate; carbodiimide derivatives including N',N'-dicycloalkylcarbodiimides, such as N',N"-dicyclohexylcarbodiimide (DCC) and 1-ethyl-3-(3-dimethylaminopropyl)carbodiimide (EDAPC); diheteroaryldiselenides, such as di-2-pyridyldiselenide; arylsulphonyltriazolides, such as p-nitrobenzenesulphonyltriazolide; 2-halo-1-alkylpyridinium halides in which the alkyl part has from 1 to 6 carbon atoms, such as 2-chloro-1-methylpyridinium iodide; diarylphosphorylazides, such as diphenylphosphorylazide (DPPA); imidazole derivatives, such as 1,1'-oxalyldiimidazole and N,N'-carbonyldiimidazole; benzotriazole derivatives, such as 1-hydroxybenzotriazole (HOBT); and dicarboximide derivatives, such as N-hydroxy-5-norbomene-2,3-dicarboximide (HONB), preferably the diarylphosphorylazides.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane and heptane; aromatic hydrocarbons, such as benzene, toluene and xylene; halogenated hydrocarbons, such as methylene chloride, chloroform, carbon tetrachloride, dichloroethane, chlorobenzene and dichlorobenzene; esters, such as ethyl formate, ethyl acetate, propyl acetate, butyl acetate and diethyl carbonate; ethers, such as diethyl ether, diisopropyl ether, tetrahydrofuran, dioxane, dimethoxyethane and diethylene glycol dimethyl ether; nitriles, such as acetonitrile and isobutyronitrile; amides, such as formamide, dimethylformamide, N,N-dimethylacetamide, N-methyl-2-pyrrolidone, N-methylpyrrolidinone and hexamethylphosphoric triamide.

There is likewise no particular restriction on the nature of the bases used, and any base commonly used in reactions of this type may equally be used here. Examples of such bases include those described in relation to Process 1, above.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C to 80°C, more preferably from 0°C to room temperature. The time required for the reaction may also vary widely, depending on many factors, notably the reaction temperature and the nature of the reagents and solvent employed. However, provided that the reaction is effected under the preferred conditions outlined above, a period of from 10 minutes to 3 days, more preferably from 30 minutes to 1 day, will usually suffice.

### Process 3

In this Process, a compound of formula: RCOOH [in which R is as defined above] is reacted with the compound of formula (5) in the presence of a halogenated phosphoric acid dialkyl ester, such as diethyl chlorophosphate, and a base in a solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane and heptane; aromatic hydrocarbons, such as benzene, toluene and xylene; halogenated hydrocarbons, such as methylene chloride, chloroform, carbon tetrachloride, dichloroethane, chlorobenzene and dichlorobenzene; esters, such as ethyl formate, ethyl acetate, propyl acetate, butyl acetate and diethyl carbonate; ethers, such as diethyl ether, diisopropyl ether, tetrahydrofuran, dioxane, dimethoxyethane and diethylene glycol dimethyl ether; nitriles, such as acetonitrile and isobutyronitrile; amides, such as formamide, N,N-dimethylformamide, N,N-dimethylacetamide, N-methyl-2-pyrrolidone, N-methylpyrrolidinone and hexamethylphosphoric triamide.

There is likewise no particular restriction on the nature of the bases used, and any base commonly used in reactions of this type may equally be used here. Examples of such bases include those described in relation to Process 1, above.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to the reflux temperature of the solvent used, more preferably from room temperature to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 3 days, more preferably from 30 minutes to 1 day, will usually suffice.

The number of acyl groups introduced varies depending on the amount of acylating agent employed.

After completion of the reaction of any of the above Processes, the desired compound of formula (6) may be collected from the reaction mixture by conventional means. For example, the desired compound can be obtained by the following procedure: appropriately neutralising the reaction mixture; adding a water-immiscible organic solvent, such as ethyl acetate, thereto after the insolubles have, if necessary, been removed by filtration; washing with water; separating the organic layer containing the desired compound; drying the organic layer over anhydrous magnesium sulphate; and distilling off the solvent.

The compound thus obtained can be separated and purified, if necessary, by any suitable combination conventional methods, for example, recrystallization, reprecipitation or other methods commonly used for the separation and purification of organic compounds, for example: adsorption column chromatography in which a carrier such as silica gel, alumina or a magnesium silicate gel (such as that available under the trade mark "Florisil") is used; a method in which a synthesis adsorbing agent is used such as partition chromatography, using a carrier such as Sephadex LH-20 (a trade mark for a materiel produced by Pharmacia Co., Ltd.), Amberlite XAD-11 (a trade mark for a material produced by Rohm & Haas Co., Ltd.) or Diaion HP-20 (a trade mark for a material produced by Mitsubishi Kasei Co., Ltd.); or normal phase or reverse phase column chromatography (preferably high performance liquid chromatography) using silica gel or an alkylated silica gel and elution with a suitable eluent.

### Step C2

In this Step, a compound of formula (1c), which is a compound of the present invention is prepared by treating the compound of formula (6) with a reagent which can remove a t-butoxycarbonyl group. The reaction is normally and preferably effected in the presence of an inert solvent.

This reaction is essentially the same as and may be carried out in the same manner as and using the same reagents and reaction conditions as in Step A2 of Method A.

Where W^{a} represents a carboxy-protecting group, a compound of the present invention can be prepared by the subsequent removal of these protecting groups as mentioned in Step A2.

### Method D

This Method illustrates the preparation of a compound of formula (2a), which is one of starting materials used in Methods A and B.

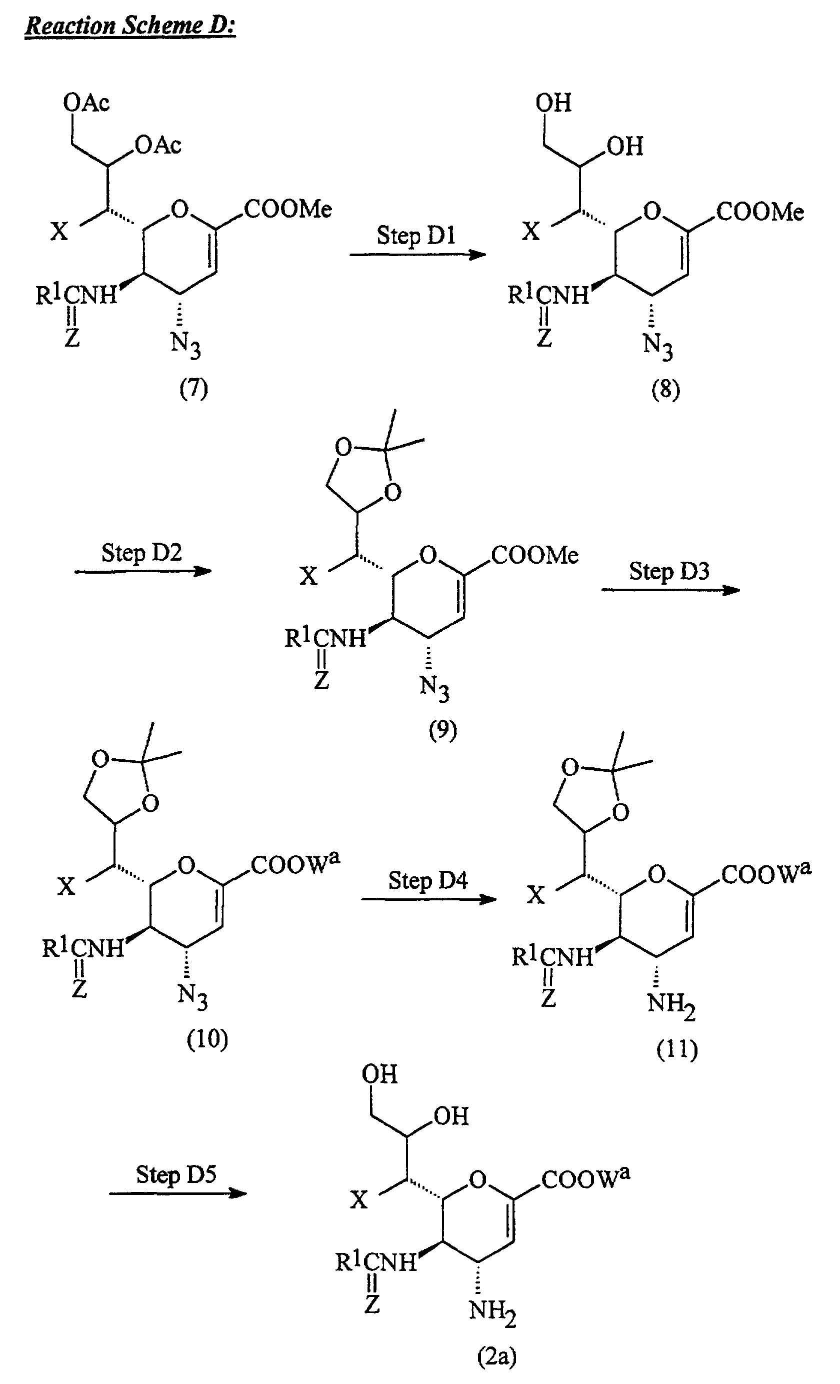

In the above formulae:
R¹, Z, X and W^{a} are as defined above;
Ac represents an acetyl group;
Boc represents a t-butoxycarbonyl group; and
Me represents a methyl group.

### Step D1

In this Step, a compound of formula (8) is prepared by reacting the compound of formula (7) with a base in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether, aromatic hydrocarbons, such as benzene, toluene and xylene; halogenated hydrocarbons, such as methylene chloride, chloroform, carbon tetrachloride, dichloroethane, chlorobenzene and dichlorobenzene; ethers, such as diethyl ether, diisopropyl ether, tetrahydrofuran, dioxane, dimethoxyethane and diethylene glycol dimethyl ether; and alcohols, such as methanol. Of these, we prefer the halogenated hydrocarbons and methanol.

There is likewise no particular restriction on the nature of the bases used, provided that it does not affect other functional groups (for example methyl ester groups), and any base commonly used in reactions of this type may equally be used here. Examples of such bases include the alkali metal methoxides, such as sodium methoxide and potassium methoxide.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents, base and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: neutralising the reaction solution with a solution of hydrogen chloride in dioxane; distilling off the solvent under reduced pressure; and purifying the residue thus obtained by silica gel chromatography.

### Step D2

In this Step, a compound of formula (9) is prepared by reacting the compound of formula (8) with a reagent for introducing an isopropylidene group.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; ethers, such as diethyl ether, diisopropyl ether, tetrahydrofuran, dioxane, dimethoxyethane and diethylene glycol dimethyl ether, and ketones, such as acetone, methyl ethyl ketone, methyl isobutyl ketone, isophorone and cyclohexanone. Of these, we prefer the ketones (particularly acetone).

The reagent used to introduce an isopropylidene group is preferably 2,2-dimethoxypropane. The reaction is usually effected in the presence of an acid, such as p-toluenesulphonic acid, as a catalyst.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents, base and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: adding a water-immiscible solvent, such as ethyl acetate, and an aqueous solution of sodium hydrogencarbonate to the reaction solution; extracting the desired compound with a solvent such as ethyl acetate; and distilling off the solvent. The desired compound can be further purified by recrystallization or by the various types of chromatography, such as column chromatography or preparative thin layer chromatography, if necessary.

### Step D3

This Step may be carried out, if desired, by:
1) converting the methyl group of the carboxylic acid ester into another substituent;
2) hydrolysing the carboxylic acid ester; or
3) carrying out 2) above and then protecting the resulting free carboxylic acid.

### 1. Ester conversion

In this Step, a compound of formula (10) is prepared by reacting the compound of formula (9) with an alcohol which is capable of giving the desired ester group, in the presence of a base and in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether, aromatic hydrocarbons, such as benzene, toluene and xylene; halogenated hydrocarbons, such as methylene chloride, chloroform, carbon tetrachloride, dichloroethane, chlorobenzene and dichlorobenzene; ethers, such as diethyl ether, diisopropyl ether, tetrahydrofuran, dioxane, dimethoxyethane and diethylene glycol dimethyl ether; alcohols, such as methanol, ethanol, propanol, isopropanol, butanol, isobutanol, t-butanol, isoamyl alcohol, diethylene glycol, glycerin, octanol, cyclohexanol and ethylene glycol monomethyl ether (such as that available under the trade mark "methyl Cellosolve"). Of these, we prefer those alcohols which form the desired ester group.

There is likewise no particular restriction on the nature of the bases used, and any base commonly used in reactions of this type may equally be used here. Examples of such bases include organic bases, such as pyridine, triethylamine, diethylamine and 4-N,N-dimethylaminopyridine.

After completion of the reaction, the desired compound can be recovered from the reaction mixture by conventional means. For example, one suitable recovery procedure comprises: neutralising the reaction mixture with an acid; adding a water-immiscible organic solvent such as ethyl acetate; extracting the desired compound with a solvent such as ethyl acetate; washing the extract; and distilling off the solvent. The desired compound can be further purified by recrystallization or the various types of chromatography, such as column chromatography or preparative thin layer chromatography, if necessary.

The same compound of formula (10) can be also obtained by reacting a carboxylic acid [obtained by removing a methyl group of the compound of formula (9)] with an alcohol of formula W^{d}-OH or an alkyl halide of formula W^{d}-Hal (where W^{d} represents an alkyl group and Hal represents a halogen atom).

### 2. Hydrolysis

In this Step, the compound of formula (10) is prepared by reacting the compound of formula (9) with a base in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include water or a mixture of water and one or more organic solvents. Suitable such organic solvents include: alcohols, such as methanol and ethanol; and ethers, such as diethyl ether, tetrahydrofuran and dioxane. Of these solvents, we prefer water or a mixture of water and one or more alcohols.

There is likewise no particular restriction on the nature of the bases used, and any base commonly used in reactions of this type may equally be used here. Examples of such bases include alkali metal hydroxides, such as sodium hydroxide and potassium hydroxide.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents, base and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: adding Dowex-50x8 (H⁺ type) (Dowex is a trade mark) to the reaction solution to neutralize it; distilling off the solvent under reduced pressure; and purifying the residue thus obtained by silica gel chromatography.

### 3. Diphenylmethylation

In this Step, a compound of formula (10) is prepared by reacting diphenyldiazomethane with the compound of formula (9) in the presence of a Lewis acid and in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; halogenated hydrocarbons, such as methylene chloride, chloroform, carbon tetrachloride, dichloroethane, chlorobenzene and dichlorobenzene; ethers, such as diethyl ether, diisopropyl ether, tetrahydrofuran, dioxane, dimethoxyethane and diethylene glycol dimethyl ether, alcohols, such as methanol, ethanol, propanol, isopropanol, butanol, isobutanol, t-butanol, isoamyl alcohol, diethylene glycol, glycerin, octanol, cyclohexanol and ethylene glycol monomethyl ether (such as that available under the trade mark "methyl Cellosolve"). Of these, we prefer the alcohols (particularly methanol), the halogenated hydrocarbons (particularly dichloromethane) and mixtures thereof.

The Lewis acid which may be employed in this reaction is preferably boron trifluoride-diethyl ether complex.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for the reaction may also vary widely, depending on many factors, notably the reaction temperature and the nature of the reagents and solvent employed. However, provided that the reaction is effected under the preferred conditions outlined above, a period of from 10 minutes to 5 hours, more preferably from 1 to 3 hours, will usually suffice.

After completion of the reaction, the desired compound can be recovered from the reaction mixture by conventional means. For example, one suitable recovery procedure comprises: adding an acid, such as acetic acid, to the reaction mixture; distilling off the solvent; and purifying the residue by recrystallization or chromatography.

### Step D4

In this Step, a compound of formula (11) is prepared from the compound of formula (10), using a reducing agent in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether, aromatic hydrocarbons, such as benzene, toluene and xylene; ethers, such as diethyl ether, diisopropyl ether, tetrahydrofuran, dioxane, dimethoxyethane and diethylene glycol dimethyl ether; alcohols, such as methanol, ethanol, propanol, isopropanol, butanol, isobutanol, t-butanol, isoamyl alcohol, diethylene glycol, glycerin, octanol, cyclohexanol and ethylene glycol monomethyl ether (such as that available under the trade mark "methyl Cellosolve"); ketones, such as acetone, methyl ethyl ketone, methyl isobutyl ketone, isophorone and cyclohexanone; nitriles, such as acetonitrile and isobutyronitrile; amides, such as formamide, dimethylformamide, N,N-dimethylacetamide, N-methyl-2-pyrrolidone, N-methylpyrrolidinone and hexamethylphosphoric triamide; sulphoxides, such as dimethyl sulphoxide and sulpholane; fatty acids, such as acetic acid; and mixtures of water and any one or more of these organic solvents. Of these, we prefer the alcohols (particularly methanol), the ethers, such as tetrahydrofuran and dioxane, the fatty acids, such as acetic acid, and mixtures of one or more of these organic solvents and water.

There is likewise no particular restriction on the nature of the reducing agents used, and any reducing agent commonly used in reactions of this type may equally be used here. Examples of such reducing agents include a hydrogenation catalyst, such as palladium-on-carbon, platinum or Raney nickel, in the presence of hydrogen gas. We particularly prefer to use a Lindlar catalyst (Pd-BaSO₄ or Pd-CaCO₃ and quinoline or lead acetate in combination).

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents, reducing agent and solvent employed. However, provided that the reaction is effected under the preferred conditions outlined above, a period of from 15 minutes to 10 hours, more preferably from 1 to 5 hours, will usually suffice.

After completion of the reaction, the desired compound can be obtained, for example, by filtering the reaction solution under reduced pressure to remove the catalyst and distilling off the solvent under reduced pressure. The desired compound can be further purified by recrystallization or the various types of chromatography, such as column chromatography or preparative thin layer chromatography, if necessary.

### Step D5

In this Step, a compound of formula (2a) is prepared by deprotecting the isopropylidene group of a compound of formula (11) in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, such as methylene chloride or chloroform.

The reagent employed for deprotecting the isopropylidene group is preferably an acid. There is likewise no particular restriction on the nature of the acids used, and any acid commonly used as an acid catalyst in reactions of this type may equally be used here. Examples of such acids include: Bronsted acids, such as inorganic acids (e.g. hydrochloric acid, hydrobromic acid, sulphuric acid, perchloric acid or phosphoric acid) or organic acids (e.g. formic acid, oxalic acid, methanesulphonic acid, p-toluenesulphonic acid, trifluoroacetic acid or trifluoromethanesulphonic acid); Lewis acids, such as zinc chloride, tin tetrachloride, boron trichloride, boron trifluoride or boron tribromide; and acidic ion exchange resins. Of these, we prefer the organic acids (particularly trifluoroacetic acid).

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: neutralising the reaction solution; and distilling off the solvent under reduced pressure. The desired compound can, if desired, be further purified by recrystallization or the various types of chromatography, such as column chromatography or preparative thin layer chromatography.

### Method H

In Method H, a compound of formula (5), which is among the starting materials used in Method C is prepared.

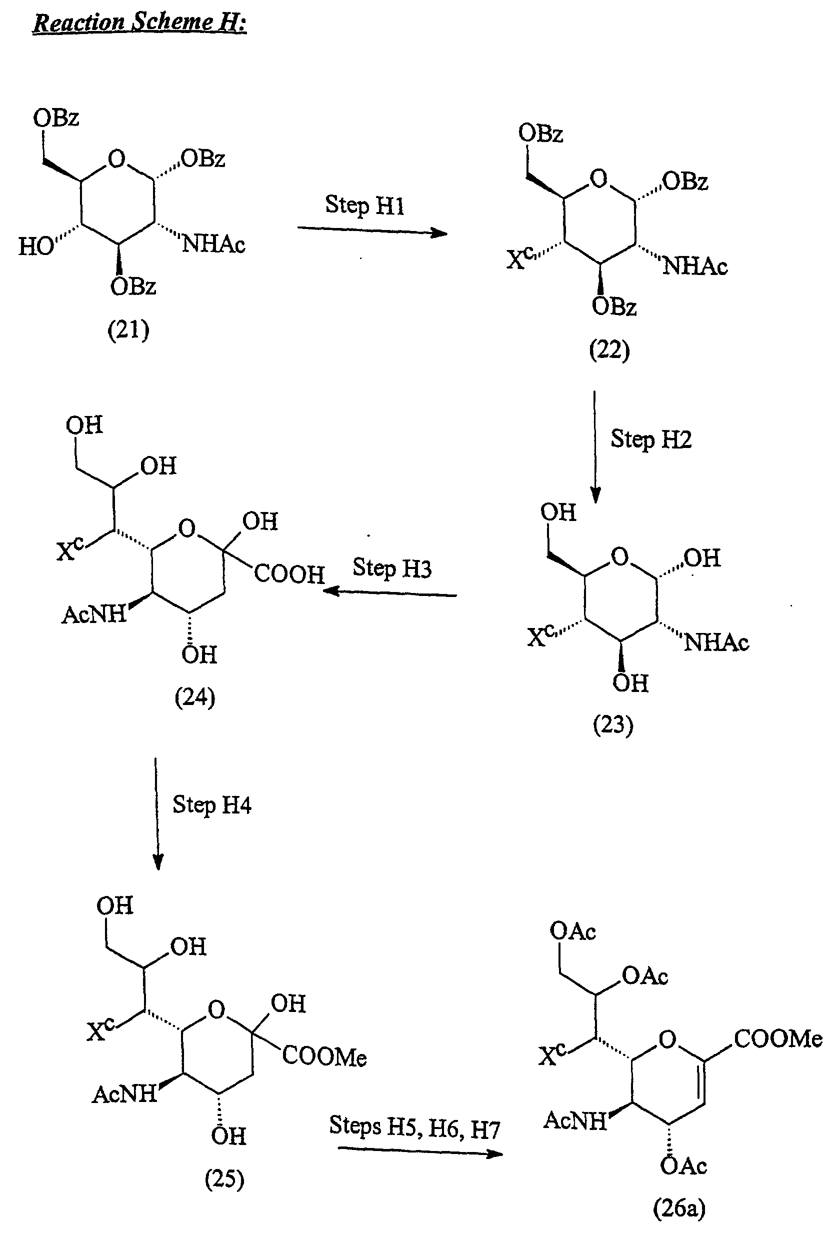

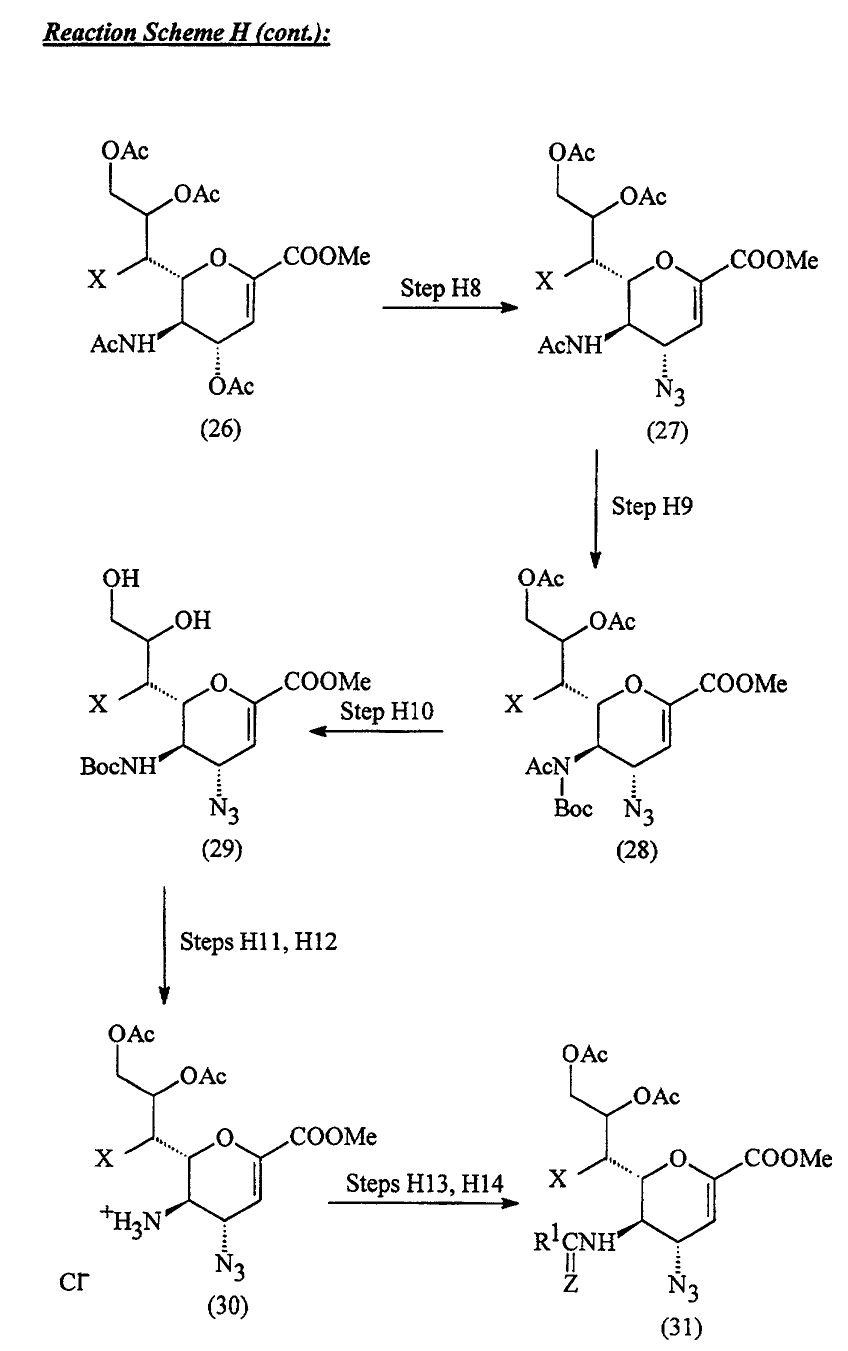

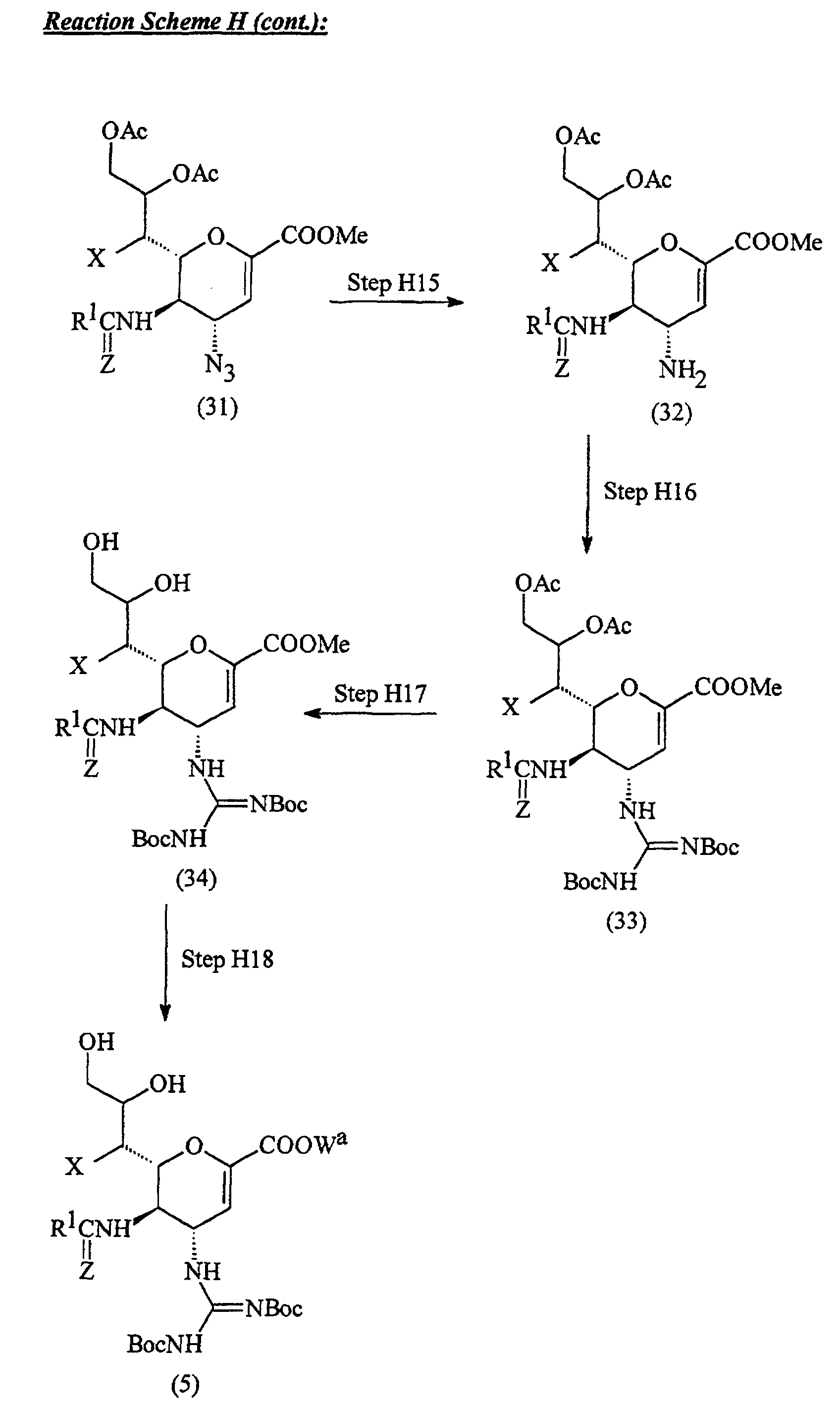

In the above formulae:
R¹, X, Z, W^{a}, Ac, Boc and Me are as defined above;
X^{c} represents an alkoxy group having from 1 to 4 carbon atoms; and
Bz represents a benzyl group.

### Step H1

In this Step, a compound of formula (22) is prepared by reacting an alkyl halide with a compound of formula (21) [described in Carbohydrate Research 83, 163 - 169 (1980)] in the presence of a base and in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; ethers, such as diethyl ether, diisopropyl ether, tetrahydrofuran, dioxane, dimethoxyethane and diethylene glycol dimethyl ether; amides, such as formamide, dimethylformamide, N,N-dimethylacetamide, N-methyl-2-pyrrolidone, N-methylpyrrolidinone and hexamethylphosphoric triamide; and sulphoxides, such as dimethyl sulphoxide and sulpholane. Of these, we prefer the amides (particularly dimethylformamide).

There is likewise no particular restriction on the nature of the bases used, and any base commonly used in reactions of this type may equally be used here. Examples of such bases include: alkali metal hydrides, such as lithium hydride, sodium hydride and potassium hydride; alkali metal alkoxides, such as sodium methoxide, sodium ethoxide, potassium methoxide, potassium ethoxide, potassium t-butoxide and lithium methoxide; alkali metal mercaptans, such as methyldicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU); and organic metal bases, such as butyllithium, lithium diisopropylamide and lithium bis(trimethylsilyl)amide. Of these, we prefer the alkali metal hydrides (particularly sodium hydride).

There is likewise no particular restriction on the nature of the alkyl halides used, and any alkyl halide commonly used in reactions of this type may equally be used here. Preferred such alkyl halides include the alkyl bromides and alkyl iodides.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for the reaction may also vary widely, depending on many factors, notably the reaction temperature and the nature of the reagents, base and solvent employed. However, provided that the reaction is effected under the preferred conditions outlined above, a period of from 15 minutes to 24 hours, more preferably from 2 to 10 hours, will usually suffice.

After completion of the reaction, the desired compound can be recovered from the reaction mixture by conventional means. For example, one suitable recovery procedure comprises: adding a water-immiscible solvent, such as ethyl acetate, and an aqueous solution of sodium hydrogencarbonate to the reaction solution; extracting the desired compound with a suitable solvent, such as ethyl acetate; and distilling off the solvent. The desired compound can, if desired, be further purified by recrystallization or the various types of chromatography, such as column chromatography or preparative thin layer chromatography.

### Step H2

In this Step, a compound of formula (23) is prepared by reacting a reducing agent with the compound of formula (22) in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; fatty acids, such as acetic acid; or a mixture of any one or more of these organic solvents and water. Of these, we prefer acetic acid.

The reduction is preferably effected by means of hydrogen in the presence of a hydrogenation catalyst. There is likewise no particular restriction on the nature of the hydrogenation catalysts used, and any hydrogenation catalyst commonly used in reactions of this type may equally be used here. Examples of such hydrogenation catalysts include: palladium-on-carbon, platinum and Raney nickel, preferably palladium-on-carbon.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for the reaction may also vary widely, depending on many factors, notably the reaction temperature and the nature of the reagents and solvent employed. However, provided that the reaction is effected under the preferred conditions outlined above, a period of from 15 minutes to 24 hours, more preferably from 2 to 10 hours, will usually suffice.

After completion of the reaction, the desired compound can be recovered from the reaction mixture by conventional means. For example, one suitable recovery procedure comprises: removing the hydrogenation catalyst by filtration; distilling off the solvent; and purifying the residue thus obtained by recrystallization or by the various forms of chromatography.

### Step H3

In this Step, a compound of formula (24) is prepared by reacting sodium pyruvate with the compound of formula (23) in the presence of N-acetylneuraminic acid aldolase and sodium azide in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. A preferred solvent is water.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for the reaction may also vary widely, depending on many factors, notably the reaction temperature and the nature of the reagents and solvent employed. However, provided that the reaction is effected under the preferred conditions outlined above, a period of from 5 hours to 5 days, more preferably from 1 to 3 days, will usually suffice.

After completion of the reaction, the desired compound can be recovered from the reaction mixture by conventional means. For example, one suitable recovery procedure comprises: desalting the compound by means of a cation exchange resin; and purifying the compound by chromatography using an anion exchange resin.

### Step H4

In this Step, a compound of formula (25) is prepared by esterifying the compound of formula (24) in the presence of an acid in a methanol solvent.

There is no particular restriction on the nature of the acids used, and any acid commonly used as an acid catalyst in reactions of this type may equally be used here. Examples of such acids include: Brønsted acids, such as inorganic acids (e.g. hydrochloric acid, hydrobromic acid, sulphuric acid, perchloric acid or phosphoric acid) or organic acids (e.g. acetic acid, formic acid, oxalic acid, methanesulphonic acid, p-toluenesulphonic acid, trifluoroacetic acid or trifluoromethanesulphonic acid); Lewis acids, such as zinc chloride, stannous tetrachloride, boron trichloride, boron trifluoride and boron tribromide; and cation exchange resins. Of these, we prefer the cation exchange resins.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for the reaction may also vary widely, depending on many factors, notably the reaction temperature and the nature of the reagents and solvent employed. However, provided that the reaction is effected under the preferred conditions outlined above, a period of from 1 to 48 hours, more preferably from 5 to 24 hours, will usually suffice.

After completion of the reaction, the desired compound can be recovered from the reaction mixture by conventional means. For example, one suitable recovery procedure comprises: removing the cation exchange resin by filtration; distilling off the solvent; and purifying the residue thus obtained by recrystallization or by the various forms of chromatography.

### Step H5

In this Step, a compound of formula (25) is acylated in an inert solvent.

This reaction is essentially the same as and may be carried out in the same manner as and using the same reagents and reaction conditions as Step C1 of Method C.

### Step H6

In this Step, the compound obtained in Step H5 is chlorinated by reacting the compound obtained in Step H5 with hydrogen chloride in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; and ethers, such as diethyl ether, diisopropyl ether, tetrahydrofuran, dioxane, dimethoxyethane and diethylene glycol dimethyl ether. Of these, we prefer the ethers (particularly dioxane).

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for the reaction may also vary widely, depending on many factors, notably the reaction temperature and the nature of the reagents and solvent employed. However, provided that the reaction is effected under the preferred conditions outlined above, a period of from 5 hours to 2 days, more preferably from 10 to 24 hours, will usually suffice.

After completion of the reaction, the solvent and hydrogen chloride are distilled off under reduced pressure and the product is used as such for the next reaction.

### Step H7

In this Step, a compound of formula (26a) is prepared by reacting a base with the compound obtained in Step H6 to carry out dehydrochlorination.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; and ethers, such as diethyl ether, diisopropyl ether, tetrahydrofuran, dioxane, dimethoxyethane and diethylene glycol dimethyl ether. Of these, we prefer the aromatic hydrocarbons (particularly benzene).

There is likewise no particular restriction on the nature of the bases used, and any base commonly used in reactions of this type may equally be used here. Examples of such bases include: alkali metal alkoxides, such as sodium methoxide, sodium ethoxide, potassium methoxide, potassium ethoxide, potassium t-butoxide and lithium methoxide; mercaptan alkali metals, such as methylmercaptan sodium and ethylmercaptan sodium; organic bases, such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), more preferably DBU.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 0°C to 50°C, more preferably at about room temperature. The time required for. the reaction may also vary widely, depending on many factors, notably the reaction temperature and the nature of the reagents and solvent employed. However, provided that the reaction is effected under the preferred conditions outlined above, a period of from 5 hours to 2 days, more preferably from 10 to 24 hours, will usually suffice.

After completion of the reaction, the desired compound can be recovered from the reaction mixture by conventional means. For example, one suitable recovery procedure comprises: adding a water-immiscible solvent, such as methylene chloride, and an aqueous solution of ammonium chloride to the reaction solution; extracting the desired compound; and distilling off the solvent. The desired compound can be purified by further recrystallization and the various types of chromatography, such as column chromatography or preparative thin layer chromatography.

### Step H8

In this Step, a compound of formula (27) is prepared by reacting the compound of formula (26a), prepared as described in Step H7, or another compound of formula (26), which may have been prepared as described in WO95/32955, with an azidating agent in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene and xylene; halogenated hydrocarbons, such as methylene chloride and chloroform; ethers, such as ether, tetrahydrofuran, dioxane and dimethoxyethane, and nitriles, such as acetonitrile.

There is likewise no particular restriction on the nature of the azidating agents used, and any azidating agent commonly used in reactions of this type may equally be used here. Examples of such azidating agents include: diarylphosphoric azide derivatives, such as diphenylphosphoric azide; trialkylsilyl azides, such as trimethylsilyl azide and triethylsilyl azide; and alkali metal azides, such as sodium azide and potassium azide. Of these, we prefer sodium azide.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: neutralising the reaction solution with a solution of hydrogen chloride in dioxane; distilling off the solvent under reduced pressure; and purifying the residue thus obtained by silica gel chromatography.

### Step H9

In this Step, a compound of formula (28) is prepared by reacting the compound of formula (27) with a t-butoxycarbonylating agent in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene and xylene; halogenated hydrocarbons, such as methylene chloride and chloroform; ethers, such as ether, tetrahydrofuran, dioxane and dimethoxyethane, and amides, such as dimethylformamide.

The t-butoxycarbonylation can be carried out by reacting di-t-butyl dicarbonate or 2-(t-butoxycarbonyloxyimino)-2-phenylacetonitrile in the presence of a base, for example 4-(N,N-dimethylamino)pyridine.

After completion of the reaction, the desired compound can be recovered from the reaction mixture by conventional means. For example, one suitable recovery procedure comprises: neutralising the reaction solution; distilling off the solvent under reduced pressure; adding a water-immiscible solvent, such as ethyl acetate, and water to the residue; extracting the desired compound with a suitable solvent, such as ethyl acetate; and distilling off the solvent. The desired compound can, if desired, be further purified by recrystallization or the various types of chromatography, such as column chromatography or preparative thin layer chromatography.

### Step H10

In this Step, a compound of formula (29) is prepared by reacting the compound of formula (28) with a base in an inert solvent.

This reaction is essentially the same as and may be carried out in the same manner as and using the same reagents and reaction conditions as Step D1 of Method D.

### Step H11

In this Step, the compound of formula (29) is acetylated in an inert solvent.

The acetylation may be carried out by conventional means, commonly used for the protection of a hydroxy group. For example, the acetylation may be carried out 1) by reacting the compound of formula (29) with acetic anhydride in pyridine or 2) by reacting the compound of formula (29) with an acetyl halide (particularly the chloride) in the presence of a base catalyst (for example, triethylamine, 4-N,N-dimethylaminopyridine) in methylene chloride.

After completion of the reaction, the desired compound can be recovered from the reaction mixture by conventional means. For example, one suitable recovery procedure comprises: distilling off the solvent under reduced pressure; adding a water-immiscible solvent, such as ethyl acetate, and an aqueous solution of sodium hydrogencarbonate to the residue; extracting the desired compound with a suitable solvent, such as ethyl acetate; and distilling off the solvent. The desired compound can, if desired, be further purified by recrystallization or the various types of chromatography, such as column chromatography or preparative thin layer chromatography.

### Step H12

In this Step, a compound of formula (30) is prepared by treating the compound obtained in Step H11 with a reagent which eliminates a t-butoxycarbonyl group in an inert solvent.

Elimination of the t-butoxycarbonyl group may be carried out by conventional processes.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; halogenated hydrocarbons, such as methylene chloride, chloroform, carbon tetrachloride, dichloroethane, chlorobenzene and dichlorobenzene; esters, such as ethyl formate, ethyl acetate, propyl acetate, butyl acetate and diethyl carbonate; ethers, such as diethyl ether, diisopropyl ether, tetrahydrofuran, dioxane, dimethoxyethane and diethylene glycol dimethyl ether; amides, such as formamide, dimethylformamide, N,N-dimethylacetamide, N-methyl-2-pyrrolidone, N-methylpyrrolidinone and hexamethylphosphoric triamide; and sulphoxides, such as dimethyl sulphoxide and sulpholane. Of these, we prefer the halogenated hydrocarbons (particularly methylene chloride).

There is likewise no particular restriction on the nature of the reagent used to eliminate the t-butoxycarbonyl group, and any reagent commonly used in reactions of this type may equally be used here. An example of such a reagent is hydrochloric acid.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -10 to 50°C, more preferably from 10 to 30°C. The time required for the reaction may also vary widely, depending on many factors, notably the reaction temperature and the nature of the reagents and solvent employed. However, provided that the reaction is effected under the preferred conditions outlined above, a period of from 15 minutes to 24 hours, more preferably from 1 to 10 hours, will usually suffice.

After completion of the reaction, the desired compound can be recovered from the reaction mixture by conventional means. For example, one suitable recovery procedure comprises: distilling off the solvent under reduced pressure; adding a water-immiscible solvent, such as ethyl acetate, and an aqueous solution of sodium hydrogencarbonate to the reaction solution; extracting the desired compound with a suitable solvent, such as ethyl acetate; and distilling off the solvent. The desired compound can, if desired, be further purified by recrystallization or the various types of chromatography, such as column chromatography or preparative thin layer chromatography.

### Step H13

In this Step, an acyl group is introduced into the compound of formula (30) by reaction with an acylating agent in an inert solvent.

This reaction is essentially the same as and may be carried out in the same manner as and using the same reagents and reaction conditions as Step C1 of Method C.

### Step H14

In this Step, a compound of formula (31) is prepared by reacting the compound obtained in Step H13 with a reagent which converts a carbonyl group into a thiocarbonyl group in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane, heptane, ligroin and petroleum ether; aromatic hydrocarbons, such as benzene, toluene and xylene; halogenated hydrocarbons, such as methylene chloride, chloroform, carbon tetrachloride, dichloroethane, chlorobenzene and dichlorobenzene; and ethers, such as diethyl ether, diisopropyl ether, tetrahydrofuran, dioxane, dimethoxyethane and diethylene glycol dimethyl ether. Of these, we prefer the ethers (particularly tetrahydrofuran).

There is no particular restriction on the nature of the reagent employed to convert the carbonyl group into a thiocarbonyl group is preferably Lawesson's reagent.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from 10 to 100°C, more preferably from 40 to 70°C. The time required for the reaction may also vary widely, depending on many factors, notably the reaction temperature and the nature of the reagents and solvent employed. However, provided that the reaction is effected under the preferred conditions outlined above, a period of from 15 minutes to 10 hours, more preferably from 1 to 5 hours, will usually suffice.

After completion of the reaction, the desired compound can be recovered from the reaction mixture by conventional means. For example, one suitable recovery procedure comprises: adding a water-immiscible solvent, such as ethyl acetate, and an aqueous solution of sodium hydrogencarbonate to the reaction solution; extracting the desired compound with a suitable solvent, such as ethyl acetate; and distilling off the solvent. The desired compound can, if desired, be further purified by recrystallization or the various types of chromatography, such as column chromatography or preparative thin layer chromatography.

### Step H15

In this Step, a compound of formula (32) is prepared from the compound of formula (31) by using a reducing agent in an inert solvent.

This reaction is essentially the same as and may be carried out in the same manner as and using the same reagents and reaction conditions as Step D4 of Method D.

### Step H16

In this Step, a compound of formula (33) is prepared by reacting the compound of formula (32) with N,N'-di-t-butoxycarbonylthiourea in the presence of a base and mercuric chloride in an inert solvent.

This reaction is essentially the same as and may be carried out in the same manner as and using the same reagents and reaction conditions as Step A1 of Method A.

### Step H17

In this Step, a compound of formula (34) is prepared by reacting the compound of formula (33) described later with a base in an inert solvent.

This reaction is essentially the same as and may be carried out in the same manner as and using the same reagents and reaction conditions as Step D1 of Method D.

### Step H18

The present step may be carried out, if desired, by 1) converting a methyl group of a carboxylic acid ester into another substituent, 2) hydrolysing a carboxylic acid ester or 3) protecting the carboxylic acid after step 2) above.

This reaction is essentially the same as and may be carried out in the same manner as and using the same reagents and reaction conditions as Step D3 of Method D.

In some cases, the compounds of the present invention can be prepared efficiently by carrying out the above steps in a different order, as will be appreciated by the skilled reader.

The compounds of the present invention may be administered alone or in admixture with conventional pharmaceutically acceptable carriers, diluents or adjuvants and in various formulations, as is well known in the art. For example, the compounds of the present invention may be given by oral or intranasal administration as a liquid preparation, such as a solution or suspension (optionally in an aqueous medium or in a mixture or such a medium and an aqueous cosolvent), or as an aerosol or a powder preparation. The liquid preparations, such as solutions, optionally including an aqueous cosolvent, may be prepared by conventional means, for example using purified water, a pharmaceutically acceptable organic solvent (for example, ethanol, propylene glycol or PEG400), a stabilizing agent (a paraoxybenzoate, such as methylparaben or propylparaben; an alcohol, such as chlorobutanol, benzyl alcohol or phenylethyl alcohol; a benzalkonium chloride; a phenol, such as phenol or cresol; thimerosal; or dehydroacetic acid). Aerosols may be prepared by conventional means, using a propellant, such as the various Freon (trade mark) gases or nitrogen gas and a surface active agent, such as lecithin. Powder preparations may be prepared by conventional means, using any one or more of an excipient, a lubricant, a stabilizing agent, a corrigent and a diluent. Examples of such excipients include organic excipients including: sugar derivatives, such as lactose, sucrose, glucose, mannitol and sorbitol; starch derivatives, such as corn starch, potato starch, α-starch, dextrin and carboxymethyl starch; cellulose derivatives, such as crystalline cellulose, low substitution hydroxypropyl cellulose, hydroxypropylmethyl cellulose, carboxymethyl cellulose, calcium carboxymethyl cellulose and internally crosslinked sodium carboxymethyl cellulose; gum arabic; dextran; and pullulan. Othere excipients include inorganic excipients including: silicate derivatives, such as soft silicic anhydride, synthetic aluminium silicate and magnesium aluminate metasilicate; phosphates, such as calcium phosphate; carbonates, such as calcium carbonate; and sulphates, such as calcium sulphate. Examples of lubricants include: stearic acid; metal salts of stearic acid such as calcium stearate and magnesium stearate; talc; colloid silica; waxes, such as beeswax and sperm whale; boric acid; adipic acid; sulphates, such as sodium sulphate; glycol; fumaric acid; sodium benzoate; DL-leucine; fatty acid sodium salts; laurylsulphates, such as sodium laurylsulphate and magnesium laurylsulphate; silicic acids, such as silicic anhydride and silicic hydrate; and the above starch derivatives. Examples of stabilizing agent include: paraoxybenzoates, such as methylparaben and propylparaben; alcohols, such as chlorobutanol, benzyl alcohol and phenylethyl alcohol; benzalkonium chlorides; phenols, such as phenol and cresol; thimerosal; dehydroacetic acid; and sorbic acid. Examples of corrigents include sweeteners, souring agents and flavors.

The amount of the active component to be used will vary depending on the symptoms, age, and body weight of the patient, as well as upon the mode of administration and the severity of the disease. However, in general, it is desirable to administer the active component in an amount of from 0.1 mg per day (preferably 1 mg) to 1000 mg (preferably 500 mg) in the case of liquid preparations; in the case of dry powder preparations, the active component is preferably administered in an amount of from 0.1 mg per day (preferably 1 mg) to 1000 mg (preferably 500 mg); in the case of aerosols, the active component is preferably administered in an amount of from 0.1 mg per day (preferably 1 mg) to 1000 mg (preferably 500 mg). This may be administered in a single dose or in divided doses, depending on the condition.

### BIOLOGICAL ACTIVITY

The 2-deoxy-2,3-didehydro-N-acylneuraminic acid derivatives of the present invention have excellent sialidase inhibitory activity, and are, accordingly, useful in the prophylaxis and therapy of influenza. The activity of compounds of the present invention is further illustrated by the following Experiments.

### EXPERIMENT 1

### Influenza Virus Replication Inhibitory Activity

Influenza virus strain A/Yamagata/32/89(H1N1) was proliferated in a live hen's egg, and the virus produced by this technique was then applied to plates of MDCK cells (derived from canine kidney) to obtain plaques. MDCK cells were infected with virus in the presence or absence of various concentrations of test compound. Influenza virus replication inhibitory activity of test compounds could then be calculated by comparing the amounts of plaques in the control and test samples.

The methodology of the present Experiment was generally in accordance with Antimicrobial Agents And Chemotherapy, 17, pp.865 - 870 (1980).

More specifically, MDCK cells were cultured to a single, confluent layer on the surface of petri-dishes having a diameter of 35 mm. The culture conditions of the MDCK cells on these plates were 37°C under a sterile atmosphere containing 5% v/v carbon dioxide gas. After the cells had reached confluence, the culture liquid was sucked off. An amount of phosphate buffered physiological saline solution containing 50 - 100 pfu (plaque forming units) of virus was then added to the plates.

The resulting plates were then left to stand for 1 hour at 37°C to permit adsorption of the virus, after which time the remaining phosphate buffered physiological saline solution was sucked off and replaced with MEM medium (Gibco BRL) containing 1 µg/ml of trypsin (Cooper Biomedical), 0.01% w/w DEAE dextran (Pharmacia LKB), 0.6% w/w agar (Sigma) together with an amount of between 0.00056 and 5.6 µg/ml of test compound.

Cultivation of the plates was continued at 37°C for 40 hours under a sterile atmosphere containing 5% carbon dioxide gas, after which time solidified agar medium was recovered. Crystal violet (Merck) was dissolved in 19% methanol to a final concentration of 0.01% and was added to the recovered agar in order to fix and stain the cells, thereby permitting determination of the number of plaques.

The number of plaques formed in the absence of test compound is taken as 100% (the control). It was then possible to calculate IC₅₀ (the concentration in nM/ml at which the test compound reduces the number of observed plaques to 50%,) as a measure of influenza virus replication inhibitory activity.

The compounds of the present invention were found to exhibit high influenza virus replication inhibitory activity, as shown in Tables 3 and 4, below.

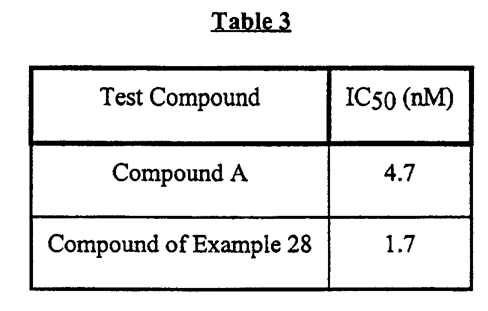

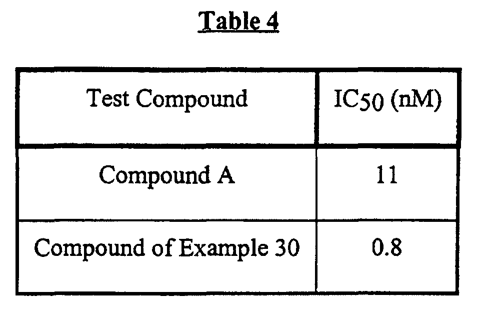

Compound A in the above Tables 3 and 4, as well as in Tables 5 and 6 below, is the compound prepared in Example 3 of Toku-Hyo-Hei 5-507068. Its structure is shown below:

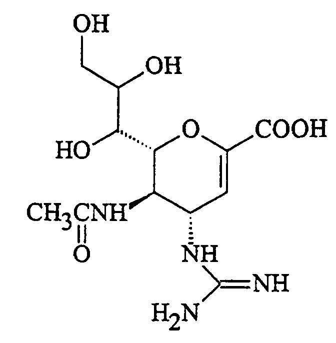

### EXPERIMENT 2

### Influenza Virus Sialidase Inhibitory Activity

In this experiment, a sample of influenza virus strain A/PR/8/34(H1N1) was dissolved by means of a surfactant, after which the virus membrane fraction was purified by centrifugation. The thus purified fraction was used as a crude influenza virus sialidase, and p-nitrophenyl-N-acetylneuraminic acid (Wako Pure Chemical) was used as a substrate to perform similar tests to those described in Analytical Biochemistry, 94, 287-296 (1979).

An enzyme solution was first prepared such that the final enzyme activity was 0.0011 units/ml (1 unit corresponds to that amount of enzyme which hydrolyzes 1 µmole of substrate in 1 minute). Also prepared beforehand were aqueous solutions of test compound in various concentrations; an aqueous solution of the substrate to a concentration of 33 µg/ml, and a 20 mM 2-(N-morpholino)-ethanesulphonic acid buffer (pH 6.5) containing 50 mM calcium chloride. These solutions were combined to prepare a mixture having a final volume of 150 µl, and which was then left to stand at 37°C for 20 minutes. The absorbance of any p-nitrophenol formed in the mixture was measured at 415 nm. The absorbance measured at 415 nm where the mixture was prepared by adding water in place of the aqueous solution of test compound was taken as 100%. Thus, IC₅₀ (nM) was determined as the concentration of the sample at which the absorbance was reduced by 50%, to provide a measure of influenza virus sialidase inhibitory activity.

The results are shown below in Table 5.

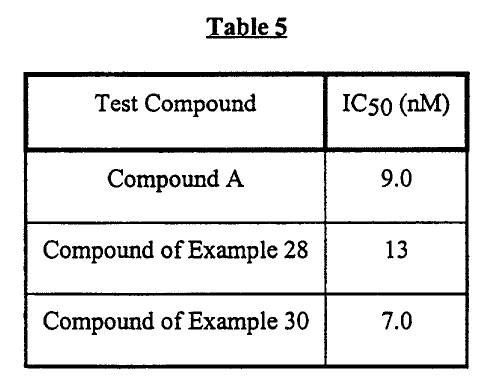

### EXPERIMENT 3

### Mouse Infection Test

A solution of 1500 pfu of mouse-adapted influenza virus strain A/PR/8/34 in 50 µl of a 0.42 % v/v bovine serum albumin-containing phosphate buffer was prepared, and mice (BALB/C, female, age: 5 to 6 weeks, 20 g) were infected dropwise, intranasally, with the resulting solution. Test compound was suspended in a physiological saline solution to provide a dosage of 0.9 µmol/kg/50 µl. The resulting suspension was administered dropwise intranasally 3 times in total, once 4 hours before the virus infection, once 4 hours after the infection and once at 17 hours after the infection. The result is shown in terms of the number of mice surviving after 6 days, 8 days, 10 days and 15 days from infection. The test was conducted on groups of mice, each group consisting of 12 animals. The results are shown in Table 6 below.

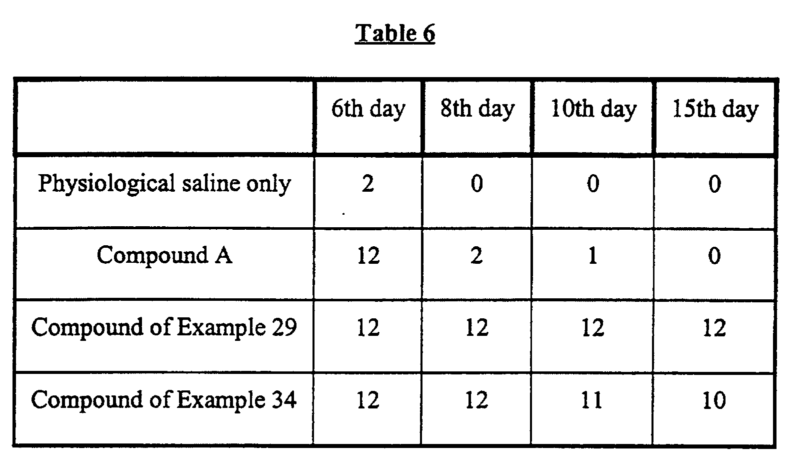

Thus, it can be seen that the compounds of the present invention greatly enhance the survival rate of animals exposed to the influenza virus.

### EXAMPLE 6

### 5-Acetamido-4-guanidino-2,3,4,5,7-pentadeoxy-7-fluoro-D-glycero-D-galacto-non-2-enopyranosoic acid trifluoroacetic acid salt (Compound No. 1-1)

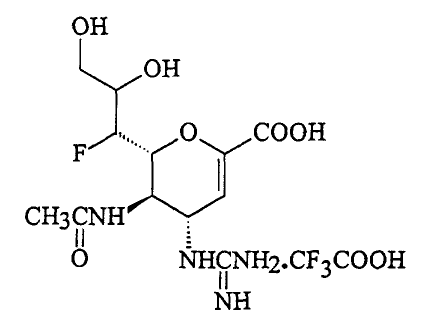

### 6(i) Methyl 5-acetamido-4-azido-8,9-di-O-acetyl-2,3,4,5,7-pentadeoxy-7-fluoro-D-glycero-D-galacto-non-2-enopyranosoate

300 mg (0.70 mmol) of methyl 5-acetamido-4,8,9-tri-O-acetyl-2,6-anhydro-3,5,7-trideoxy-7-fluoro-D-glycero-D-galacto-non-2-enopyranosoate [prepared according to the procedure described in WO 95/32955] were dissolved in 10 ml of anhydrous methylene chloride, and 25 mg (0.78 mmol) of methanol were added to the resulting solution. The atmosphere in the reaction system was replaced by nitrogen, and then 1.0 g (7.0 mmol) of a boron trifluoride diethyl ether complex salt was added to the reaction system, and the mixture was stirred at room temperature for 24 hours. At the end of this time, the reaction mixture was poured into a mixture of 50 ml of water, 10 g of ice, 10 g of solid sodium hydrogencarbonate and 50 ml of ethyl acetate, and the mixture was vigorously stirred for 10 minutes. The organic layer was separated, washed with 10 ml of a saturated aqueous solution of sodium chloride and dried over anhydrous sodium sulphate, after which the solvent was removed by distillation under reduced pressure. The resulting residue (210 mg) was dissolved in 4 ml of dimethylformamide, and 400 mg of a cation exchange resin, Dowex-50x8 (H⁺) (Dowex is a trade mark) and 100 mg (1.53 mmol) of sodium azide were added to the resulting solution. The mixture was then stirred at 80°C for 4 hours. At the end of this time, the Dowex-50x8 (H⁺) was separated by filtration and the solvent was removed by distillation under reduced pressure. 30 ml of ethyl acetate and 20 ml of a saturated aqueous solution of sodium hydrogencarbonate were then added to dissolve the residue. The organic layer was separated, washed with a saturated aqueous solution of sodium chloride and dried over anhydrous sodium sulphate, after which the solvent was removed by distillation under reduced pressure. The resulting residue was purified by silica gel column chromatography, using a 50 : 1 by volume mixture of methylene chloride and methanol as the eluent, to obtain 170 mg (yield 59%) of the title compound as a colourless viscous substance.
Rf = 0.31 (20 : 1 = methylene chloride : methanol).
[α]_{D}²⁵ +69.4° (c = 0.18, CHCl₃).
Mass Spectrum (FAB) m/e 417 (M⁺+H).
¹H-Nuclear Magnetic Resonance Spectrum (CDCl₃, 270 MHz) δ (ppm):
2.07 (9H, singlet);
3.58 (1H, doubled doublet of doublets, J = 9.0, 9.0 & 9.0 Hz);
3.80 (3H, singlet);
4.22 (1H, doubled doublet of doublets, J = 1.8, 5.2 & 13.0 Hz);
4.65 - 5.00 (4H, multiplet);
5.45 (1H, multiplet);
5.98 (1H, doublet, J = 2.8 Hz);
6.00 (1H, doublet, J = 8.5 Hz).

### 6(ii) Methyl 5-acetamido-4-(N,N'-bis-t-butoxycarbonylguanidino)-8,9-di-O-acetyl-2,3,4,5,7-pentadeoxy-7-fluoro-D-glycero-D-galacto-non-2-enopyranosoate

36 mg (0.08 mmol) of methyl 5-acetamido-4-azido-8,9-di-O-acetyl-2,3,4,5,7-pentadeoxy-7-fluoro-D-glycero-D-galacto-non-2-enopyranosoate [prepared as described in step (i) above] were dissolved in 3 ml of methanol, and 10 mg of a Lindlar catalyst were added to the resulting solution. The atmosphere in the reaction system was replaced by hydrogen, and then the mixture was stirred for 2 hours. The catalyst was then separated by filtration, and the solvent was removed by distillation under reduced pressure. The resulting residue was dissolved in 2 ml of dimethylformamide, and 26 mg (0.093 mmol) of N,N'-di-t-butoxycarbonylthiourea, 19 mg (0.186 mmol) of triethylamine and 25 mg (0.093 mmol) of mercuric chloride were added to the resulting solution. The mixture was then stirred at room temperature for 1 hour. The solid was separated by filtration and the filtrate was poured into a 2-layer mixture of 5 ml of ethyl acetate and 3 ml of a saturated aqueous solution of sodium hydrogencarbonate. The organic layer was separated, washed with a saturated aqueous solution of sodium chloride and dried over anhydrous sodium sulphate, after which the solvent was removed by distillation under reduced pressure. The residue was purified by silica gel column chromatography, using a 2 : 1 by volume mixture of ethyl acetate and hexane as the eluent, to obtain 32 mg (yield 59%) of the title compound as a colourless amorphous substance.
Rf= 0.35 (2 : 1 = ethyl acetate : hexane).
[α]_{D}²⁵ +5.6° (c = 0.16, CHCl₃).
Mass Spectrum (FAB) m/e 633 (M⁺+H).
¹H-Nuclear Magnetic Resonance Spectrum (CDCl₃, 270 MHz) δ (ppm):
1.49 (9H, singlet);
1.52 (9H, singlet);
1.98 (3H, singlet);
2.05 (3H, singlet);
2.07 (3H, singlet);
3.80 (3H, singlet);
4.00 - 4.30 (3H, multiplet);
4.75 (1H, doublet of doublets, J = 9.2, 50 Hz);
4.78 (1H, doublet, J = 18 Hz);
5.23 (1H, doublet of doublets, J = 9.0, 9.0 Hz);
5.43 (1H, multiplet);
5.88 (1H, doublet, J = 2.8 Hz);
6.80 (1H, doublet, J = 8.2 Hz);
8.60 (1H, doublet, J = 8.5 Hz).

### 6(iii) 5-Acetamido-4-guanidino-2,3,4,5,7-pentadeoxy-7-fluoro-D-glycero-D-galacto-non-2-enopyranosoic acid trifluoroacetic acid salt

32 mg (0.05 mmol) of methyl 5-acetamido-4-(N,N'-bis-t-butoxycarbonylguanidino)-8,9-di-O-acetyl-2,3,4,5,7-pentadeoxy-7-fluoro-D-glycero-D-galacto-non-2-enopyranosoate [prepared as described in step (ii) above] were dissolved in 2 ml of methanol, and 0.5 ml of a 0.1 N methanolic solution of sodium methoxide was added to the resulting solution. The mixture was then stirred at room temperature for 1 hour. At the end of this time, the reaction mixture was neutralized with a 4 M solution of hydrogen chloride in dioxane, and the solvent was removed by distillation under reduced pressure. The residue was then dissolved in a 3 : 1 by volume mixture of methylene chloride and trifluoroacetic acid, and the mixture was stirred at room temperature for 5 hours. At the end of this time, the solvent was removed by distillation under reduced pressure, and the resulting residue was dissolved in 1 ml of distilled water, and then 70 ml of a 1 N aqueous solution of sodium hydroxide were added to the resulting solution. The mixture was then stirred at room temperature for 1 hour. Dowex-50x8 (H⁺) (Dowex is a trade mark) was added to the resulting mixture to neutralize it, and then the water was removed by distillation. The resulting residue was purified by silica gel column chromatography, using a 5 : 1 : 1 by volume mixture of isopropanol, ethyl acetate and water as the eluent, to obtain 16 mg (yield 71%) of the title compound as a colourless solid.
Rf= 0.30 (4 : 1 : 1 = isopropanol: acetic acid : water).
[α]_{D}²⁵ +28.0° (c = 0.10, H₂O).
Mass Spectrum (FAB) m/e 335 (M⁺+H).
¹H-Nuclear Magnetic Resonance Spectrum (D₂O, 270 MHz) δ (ppm):
2.00 (3H, singlet);
3.65 (1H, doubled doublet of doublets, J = 2.4, 5.5 & 12.0 Hz);
3.85 (1H, doubled doublet of doublets, J = 2.5, 2.5 & 12 Hz);
4.15 (1H, multiplet);
4.23 (1H, doublet, J = 9.0 Hz);
4.30 - 4.60 (3H, multiplet);
5.63 (1H, singlet).

### EXAMPLE 7

### Tetradecyl 5-acetamido-4-guanidino-2,3,4,5,7-pentadeoxy-7-fluoro-D-glycero-D-galacto-non-2-enopyranosoate trifluoroacetic acid salt (Compound No. 1-5)

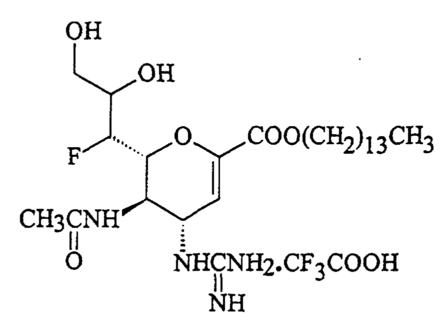

### 7(i) Methyl 5-acetamido-4-azido-8,9-O-isopropylidene-2,3,4,5,7-pentadeoxy-7-fluoro-D-glycero-D-galacto-non-2-enopyranosoate

300 mg (0.72 mmol) of methyl 5-acetamido-4-azido-8,9-di-O-acetyl-2,3,4,5,7-pentadeoxy-7-fluoro-D-glycero-D-galacto-non-2-enopyranosoate [prepared as described in Example 6(i)] were dissolved in 5 ml of methanol, and 0.2 ml of a 1 M methanolic solution of sodium methoxide was added to the resulting solution. The mixture was then stirred at room temperature for 1 hour. At the end of this time, the reaction mixture was neutralized with a 4 M solution of hydrogen chloride in dioxane, and then the solvent was removed by distillation under reduced pressure. The resulting residue was dissolved in 5 ml of acetone, and 400 mg (3.85 mmol) of 2,2-dimethoxypropane and 20 mg (0.11 mmol) of p-toluenesulphonic acid were added to the resulting solution. The mixture was then stirred at room temperature for 3 hours. The solids were separated by filtration and the solvent was removed from the filtrate by distillation under reduced pressure. The residue was purified by silica gel column chromatography, using a 50 : 1 by volume mixture of methylene chloride and methanol as the eluent, to obtain 140 mg (yield 52%) of the title compound as a colourless amorphous substance.
Rf = 0.33 (20 : 1 = methylene chloride : methanol).
[α]_{D}²⁵ +111° (c = 0.13, CHCl₃).
Mass Spectrum (FAB) m/e 373 (M⁺+H).
¹H-Nuclear Magnetic Resonance Spectrum (CDCl₃, 270 MHz) δ (ppm):
1.37 (3H, singlet);
1.42 (3H, singlet);
2.05 (3H, singlet);
3.50 (1H, doubled doublet of doublets, J = 7.5, 7.5 & 9.5 Hz);
3.80 (3H, singlet);
4.13 (1H, doubled doublet of doublets, J = 1.2, 6.0 & 9.0 Hz);
4.20 (1H, doubled doublet of doublets, J = 1.2, 6.0 & 9.0 Hz);
4.45 (1H, multiplet);
4.70 (1H, doubled doublet of doublets, J = 1.5, 5.7 & 47.0 Hz);
4.90 (1H, doublet of doublets, J = 2.8 & 9.5 Hz);
4.92 (1H, doubled doublet of doublets, J = 1.0, 11.0 & 28.0 Hz);
5.90 (1H, doublet, J = 7.3 Hz);
5.96 (1H, doublet, J = 2.8 Hz).

### 7(ii) Tetradecyl 5-acetamido-4-azido-8,9-O-isopropylidene-2,3,4,5,7-pentadeoxy-7-fluoro-D-glycero-D-galacto-non-2-enopyranosoate

113 mg (0.31 mmol) of methyl 5-acetamido-4-azido-8,9-O-isopropylidene-2,3,4,5,7-pentadeoxy-7-fluoro-D-glycero-D-galacto-non-2-enopyranosoate [prepared as described in step (i) above] were dissolved in 4 ml of a 6 : 1 by volume mixture of methanol and water, and 0.33 ml of a 1 M aqueous solution of potassium hydroxide was added to the resulting solution. The mixture was then stirred at room temperature for 2 hours. At the end of this time, the solvent was removed by distillation under reduced pressure, and the residue was dried over anhydrous sodium sulphate at room temperature and under reduced pressure for 2 hours to obtain a pale yellow solid. The solid was dissolved in 8 ml of acetonitrile, and 80 mg (0.3 mmol) of 18-crown-6 and 415 mg (1.5 mmol) of tetradecyl bromide were added to the resulting solution. The mixture was then stirred at 80°C for 2 hours. At the end of this time, the reaction mixture was poured into a 2-layer solution of 15 ml of ethyl acetate and 10 ml of a saturated aqueous solution of sodium hydrogencarbonate, and the organic layer was separated, washed with a saturated aqueous solution of sodium chloride and dried over anhydrous sodium sulphate, after which the solvent was removed by distillation under reduced pressure. The residue was purified by silica gel column chromatography, using a 50 : 1 by volume mixture of methylene chloride and methanol as the eluent, to obtain 110 mg (yield 64%) of the title compound as a colourless viscous substance.
Rf= 0.47 (20 : 1 = methylene chloride : methanol).
[α]_{D}²⁵ +52.9° (c = 0.09, CHCl₃).
Mass Spectrum (FAB) m/e 555 (M⁺+H).
¹H-Nuclear Magnetic Resonance Spectrum (CDCl₃, 270 MHz) δ (ppm):
0.80 - 0.95 (3H, singlet);
1.20 - 1.50 (24H, multiplet);
1.37 (3H, singlet);
1.42 (3H, singlet);
1.60 - 1.80 (2H, multiplet);
2.05 (3H, singlet);
4.10 - 4.30 (2H, multiplet);
4.45 (1H, multiplet);
4.72 (1H, doubled doublet of doublets, J = 1.5, 5.3 & 38.0 Hz);
4.90 (1H, doublet of doublets, J = 2.4 & 9.3 Hz);
4.92 (1H, doubled doublet of doublets, J = 1.0, 10.0 & 28.0 Hz);
5.97 (1H, doublet, J = 6.5 Hz);
5.95 (1H, doublet, J = 2.4 Hz).

### 7(iii) Tetradecyl 5-acetamido-4-(N,N'-bis-t-butoxycarbonylguanidino)-8,9-O-isopropylidene-2,3,4,5,7-pentadeoxy-7-fluoro-D-glycero-D-galacto-non-2-enopyranosoate

110 mg (0.20 mmol) of tetradecyl 5-acetamido-4-azido-8,9-O-isopropylidene-2,3,4,5,7-pentadeoxy-7-fluoro-D-glycero-D-galacto-non-2-enopyranosoate [prepared as described in step (ii) above] were dissolved in 5 ml of methanol, and 30 mg of a Lindlar catalyst were added to the resulting solution. The atmosphere in the reaction system was then replaced by hydrogen. The mixture was then stirred for 2 hours. At the end of this time, the catalyst was separated by filtration and the solvent was removed by distillation under reduced pressure. The residue was dissolved in 4 ml of dimethylformamide, and 47 mg (0.17 mmol) of N,N-di-t-butoxycarbonylthiourea, 35 mg (0.342 mmol) of triethylamine and 47 mg (0.17 mmol) of mercuric chloride were added to the resulting solution. The mixture was then stirred at room temperature for 1 hour. The solid was separated by filtration and the filtrate was poured into a 2-layer solution of 15 ml of ethyl acetate and 10 ml of a saturated aqueous solution of sodium hydrogencarbonate. The organic layer was separated, washed with a saturated aqueous solution of sodium chloride and dried over anhydrous sodium sulphate, after which the solvent was removed by distillation under reduced pressure. The residue was purified by silica gel column chromatography, using a 2 : 1 by volume mixture of ethyl acetate and hexane as the eluent, to obtain 98 mg (yield 63%) of the title compound as a colourless viscous substance.
Rf= 0.30 (20 : 1 = methylene chloride : methanol).
[α]_{D}²⁵ +2.2° (c = 0.14, CHCl₃).
Mass Spectrum (FAB) m/e 771 .(M⁺+H)
¹H-Nuclear Magnetic Resonance Spectrum (CACl₃, 270 MHz) δ (ppm):
0.80 - 0.95 (3H, singlet);
1.20 - 1.40 (24H, multiplet);
1.36 (3H, singlet);
1.41 (3H, singlet);
1.49 (9H, singlet);
1.50 (9H, singlet);
1.60 - 1.80 (2H, multiplet);
2.05 (3H, singlet);
4.10 - 4,70 (6H, multiplet);
5.20 (1H, doubled doublet of doublets, J = 2.4, 7.5 & 7.5 Hz);
5.83 (1H, doublet, J = 2.4 Hz);
5.65 (1H, doublet, J = 7.0 Hz);
8.60 (1H, doublet, J = 8.5 Hz).

### 7(iv) Tetradecyl 5-acetamido-4-guanidino-2,3,4,5,7-pentadeoxy-7-fluoro-D-glycero-D-galacto-non-2-enopyranosoate trifluoroacetic acid salt

80 mg (0.11 mmol) of tetradecyl 5-acetamido-4-(N,N'-bis-t-butoxycarbonylguanidino)-8,9-O-isopropylidene-2,3,4,5,7-pentadeoxy-7-fluoro-D-glycero-D-galacto-non-2-enopyranosoate [prepared as described in step (iii) above] were dissolved in of a 3 : 1 by volume mixture of methylene chloride and trifluoroacetic acid, and the mixture was stirred at room temperature for 4 hours. At the end of this time, the solvent was removed by distillation under reduced pressure, and the residue was purified by silica gel column chromatography, using a 2 : 8 : 1 by volume mixture of isopropanol, ethyl acetate and water as the eluent, to obtain 40 mg (yield 56%) of the title compound as a colourless solid.
Rf= 0.35 (5 : 1 : 1 = t-butanol : acetic acid : water).
[α]_{D}²⁵ +22.4° (c = 0.13, CH₃OH).
Mass Spectrum (FAB) m/e 531 (M⁺+H).
¹H-Nuclear Magnetic Resonance Spectrum (D₂O, 270 MHz) δ (ppm):
0.70 - 0.85 (3H, singlet);
1.10 - 1.30 (24H, multiplet);
1.50 - 1.80 (2H, multiplet);
2.00 (3H, singlet);
3.60 - 3.80 (2H, multiplet);
4.10 - 4.30 (3H, multiplet);
4.40 - 4.60 (2H, multiplet);
5.88 (1H, singlet).

### EXAMPLE 13

### 5-Acetamido-4-guanidino-9-O-hexanoyl-2,3,4,5,7-pentadeoxy-7-fluoro-D-glycero-D-galacto-non-2-enopyranosoic acid trifluoroacetic acid salt (Compound No. 1-36)

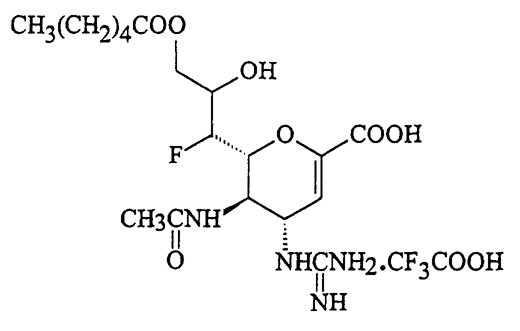

### 13(i) Diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate

100 mg (0.24 mmol) of methyl 5-acetamido-4-(N,N'-bis-t-butoxycarbonylguanidino)-8,9-di-O-acetyl-2,3,4,5,7-pentadeoxy-7-fluoro-D-glycero-D-galacto-non-2-enopyranosoate [prepared as described in Example 6(ii)] were dissolved in 3 ml of methanol, and 0.5 ml of a 0.1 N methanolic solution of sodium methoxide was added to the resulting solution. The mixture was then stirred at room temperature for 1 hour, after which the reaction mixture was neutralized with a 4 M solution of hydrogen chloride in dioxane, and the solvent was removed by distillation under reduced pressure. The resulting residue was dissolved in 1 ml of distilled water and 280 ml of a 1 N aqueous solution of sodium hydroxide were added to the resulting solution. The mixture was then stirred at room temperature for 1 hour, after which the reaction mixture was neutralized with a 4 M solution of hydrogen chloride in dioxane, and then the water was removed by distillation. The resulting residue was dissolved in 3 ml of a 6 : 1 by volume mixture of methanol and water, and 100 mg (0.52 mmol) of diphenyldiazomethane and 8 mg (0.06 mmol) of a boron trifluoride-diethyl ether complex were added to the resulting solution. The mixture was then stirred at room temperature for 2 hours. At the end of this time, acetic acid was added to the reaction mixture, and the solvent was removed by distillation under reduced pressure. The residue was purified by silica gel column chromatography, using a 20 : 1 by volume mixture of methylene chloride and methanol as the eluent, to obtain 82 mg (yield 50%) of the title compound as a colourless amorphous substance.
Rf= 0.45 (20 : 1 = methylene chloride : methanol).
[α]_{D}²⁵ = -12.2° (c = 0.1, CHCl₃).
Mass Spectrum (FAB) m/e 701 (M⁺+H).
¹H-Nuclear Magnetic Resonance Spectrum (CDCl₃, 270 MHz) δ (ppm):
1.50 (9H, singlet);
1.51 (9H, singlet);
1.95 (3H, singlet);
3.25 (2H, multiplet);
3.90 (2H, multiplet);
4.20 - 4.70 (3H, multiplet);
5.25 (1H, multiplet);
5.95 (1H, doublet, J = 2.5 Hz);
6.93 (1H, singlet);
7.10 (1H, doublet, J = 8.5 Hz);
7.20-7.40(10H, multiplet);
8.60 (1H, doublet, J = 8.5 Hz).

### 13(ii) Diphenylmethyl 5-acetamido-4-(N,N'-bis-t-butoxycarbonylguanidino)-9-O-hexanoyl-2,3,4,5,7-pentadeoxy-7-fluoro-D-glycero-D-galacto-non-2-enopyranosoate

50 mg (0.072 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in step (i) above] were dissolved in 2 ml of methylene chloride, and 11 mg (0.11 mmol) of triethylamine and 11 mg (0.086 mmol) of hexanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 47 mg (yield 83%) of the title compound as a colourless amorphous substance.
Rf= 0.6 (20 : 1 = methylene chloride : methanol).
[α]D²⁵ = -4.0° (c = 0.1, CHCl₃).
Mass Spectrum (FAB) m/e 786 (M⁺+H).
¹H-Nuclear Magnetic Resonance Spectrum (CDCl₃, 270 MHz) δ (ppm):
0.90 (3H, multiplet);
1.20 - 1.40 (6H, multiplet);
1.48 (9H, singlet);
1.50 (9H, singlet);
1.97 (3H, singlet);
2.35 (2H, triplet, J = 7.5 Hz);
2.95 (1H, doublet, J = 5.0 Hz);
4.20 - 4.60 (6H, multiplet);
5.25 (1H, multiplet);
5.97 (1H, doublet, J = 2.6 Hz);
6.65 (1H, doublet, J = 8.5 Hz);
6.93 (1H, singlet);
7.20 - 7.40 (10H, multiplet);
8.60 (1H, doublet, J = 8.5 Hz).

### 13(iii) 5-Acetamido-4-guanidino-9-O-hexanoyl-2,3,4,5,7-pentadeoxy-7-fluoro-D-glycero-D-galacto-non-2-enopyranosoic acid trifluoroacetic acid salt

40 mg (0.05 mmol) of diphenylmethyl 5-acetamido-4-(N,N'-bis-t-butoxycarbonylguanidino)-9-O-hexanoyl-2,3,4,5,7-pentadeoxy-7-fluoro-D-glycero-D-galacto-non-2-enopyranosoate [prepared as described in step (ii) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 18 mg (yield 65%) of the title compound as a colourless solid.
Rf= 0.3 (2 : 5 : 1 = isopropanol : ethyl acetate : water).
[α]_{D}²⁵ = +60.0° (c = 0.1, CH₃OH).
Mass Spectrum (FAB) m/e 433 (M⁺+H).
¹H-Nuclear Magnetic Resonance Spectrum (CD₃OD, 270 MHz) δ (ppm):
0.85 - 0.95 (3H, multiplet);
1.25 - 1.40 (4H, multiplet);
1.55 - 1.70 (2H, multiplet);
2.00 (3H, singlet);
2.35 (2H, triplet, J = 4.5 Hz);
4.10 - 4.40 (5H, multiplet);
4.45 - 4.70 (2H, multiplet);
5.60 (1H, doublet, J = 2.4 Hz).

### EXAMPLE 14

### 5-Acetamido-4-guanidino-9-O-octanoyl-2,3,4,5,7-pentadeoxy-7-fluoro-D-glycero-D-galacto-non-2-enopyranosoic acid trifluoroacetic acid salt (Compound No. 1-38)

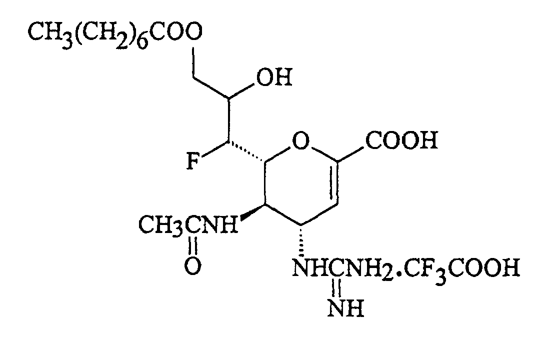

### 14(i) Diphenylmethyl 5-acetamido-4-(N,N'-bis-t-butoxycarbonylguanidino)-9-O-octanoyl-2,3,4,5,7-pentadeoxy-7-fluoro-D-glycero-D-galacto-non-2-enopyranosoate

50 mg (0.072 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml methylene chloride, and 11 mg (0.11 mmol) of triethylamine and 16 mg (0.086 mmol) of octanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 45 mg (yield 77%) of the title compound as a colourless amorphous substance.
Rf= 0.5 (20 : 1 = methylene chloride : methanol).
[α]_{D}²⁵ =-5.2° (c = 0.2, CHCl₃).
Mass Spectrum (FAB) m/e 814 (M⁺+H).
¹H-Nuclear Magnetic Resonance Spectrum (CDCl₃, 270 MHz) δ (ppm):
1.48 (9H, singlet);
1.50 (9H, singlet);
1.95 (3H, singlet);
3.65 (1H, doublet of doublets, J = 5.0 & 18 Hz);
3.80 (1H, doublet of doublets, J = 1.8 & 18 Hz);
4.15 (1H, multiplet);
4.23 (1H, doublet, J = 8.5 Hz);
4.30 - 4.60 (3H, multiplet);
5.95 (1H, doublet, J = 2.3 Hz).

### 14(ii) 5-Acetamido-4-guanidino-9-O-octanoyl-2,3,4,5,7-pentadeoxy-7-fluoro-D-glycero-D-galacto-non-2-enopyranosoic acid trifluoroacetic acid salt

40 mg (0.05 mmol) of diphenylmethyl 5-acetamido-4-(N,N'-bis-t-butoxycarbonylguanidino)-9-O-octanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 18 mg (yield 64%) of the title compound as a colourless solid.
Rf= 0.3 (2 : 5 : 1 = isopropanol : ethyl acetate : water).
[α]_{D}²⁵ = +56° (c = 0.2 CH₃OH).
Mass Spectrum (FAB) m/e 461 (M⁺+H).
¹H-Nuclear Magnetic Resonance Spectrum (CD₃OD, 270 MHz) δ (ppm):
0.85 - 0.95 (3H, multiplet);
1.25 - 1.40 (8H, multiplet);
1.55 - 1.70 (2H, multiplet);
2.00 (3H, singlet);
2.35 (2H, triplet, J = 4.5 Hz);
4.10 - 4.40 (5H, multiplet);
4.45 - 4.70 (2H, multiplet);
5.60 (1H, doublet, J = 2.4 Hz).

### EXAMPLE 15

### 5-Acetamido-4-guanidino-9-O-dodecanoyl-2,3,4,5,7-pentadeoxy-7-fluoro-D-glycero-D-galacto-non-2-enopyranosoic acid trifluoroacetic acid salt (Compound No. 1-40)

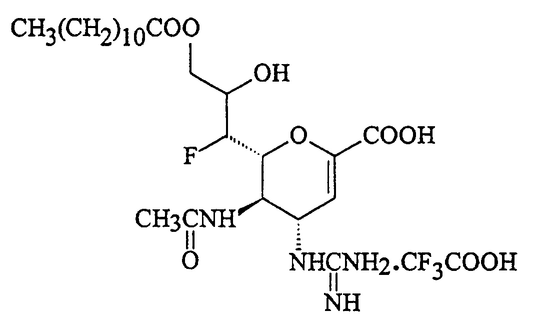

### 15(i) Diphenylmethyl 5-acetamido-4-(N,N'-bis-t-butoxycarbonylguanidino)-9-O-dodecanoyl-2,3,4,5,7-pentadeoxy-7-fluoro-D-glycero-D-galacto-non-2-enopyranosoate

55 mg (0.078 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, 10 mg (0.10 mmol) of triethylamine and 21 mg (0.094 mmol) of dodecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 1 : 1 by volume mixture of ethyl acetate and hexane as the eluent, to obtain 48 mg (yield 69%) of the title compound as a colourless amorphous substance.
Rf= 0.4 (1 : 1 = ethyl acetate : hexane).
[α]_{D}²⁵ = -5.7° (c = 0.12, CHCl₃).
Mass Spectrum (FAB) m/e 883 (M⁺+H).
¹H-Nuclear Magnetic Resonance Spectrum (CDCl₃, 270 MHz) δ (ppm):
0.90 (3H, multiplet);
1.20 - 1.40 (18H, multiplet);
1.48 (9H, singlet);
1.50 (9H, singlet);
1.97 (3H, singlet);
2.35 (2H, triplet, J = 7.5 Hz);
2.95 (1H, doublet, J = 5.0 Hz);
4.20 - 4.60 (6H, multiplet);
5.25 (1H, multiplet);
5.97 (1H, doublet, J = 2.6 Hz);
6.65 (1H, doublet, J = 8.5 Hz);
6.93 (1H, singlet);
7.20 - 7.40 (10H, multiplet);
8.60 (1H, doublet, J = 8.5 Hz).

### 15(ii) 5-Acetamido-4-guanidino-9-O-dodecanoyl-2,3,4,5,7-pentadeoxy-7-fluoro-D-glycero-D-galacto-non-2-enopyranosoic acid trifluoroacetic acid salt

43 mg (0.05 mmol) of diphenylmethyl 5-acetamido-4-(N,N'-bis-t-butoxycarbonylguanidino)-9-O-dodecanoyl-2,3,4,5,7-pentadeoxy-7-fluoro-D-glycero-D-galacto-non-2-enopyranosoate [prepared as described in step (i) above] were dissolved in 3 ml of a 3 : by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 33 mg (yield 91%) of the title compound as a colourless solid.
Rf= 0.4 (2 : 5 : 1 = isopropanol: ethyl acetate : water).
[α]_{D}²⁵ = +22.7° (c = 0.1, CH₃OH).
Mass Spectrum (FAB) m/e 517 (M⁺+H).
¹H-Nuclear Magnetic Resonance Spectrum (CD₃OD, 270 MHz) δ (ppm):
0.85 - 0.95 (3H, multiplet);
1.25 - 1.40 (16H, multiplet);
1.55 - 1.70 (2H, multiplet);
2.00 (3H, singlet);
2.35 (2H, triplet, J = 7.0 Hz);
4.10 - 4.40 (5H, multiplet);
4.45 - 4.70 (2H, multiplet);
5.60 (1H, doublet, J = 2.4 Hz).

### EXAMPLE 16

### 5-Acetamido-4-guanidino-9-O-tetradecanoyl-2,3,4,5,7-pentadeoxy-7-fluoro-D-glycero-D-galacto-non-2-enopyranosoic acid trifluoroacetic acid salt (Compound No. 1-41)

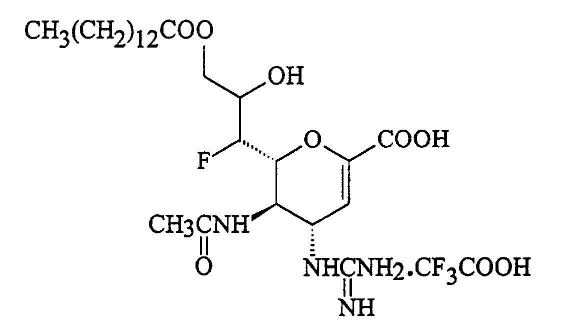

### 16(i) Diphenylmethyl 5-acetamido-4-(N,N'-bis-t-butoxycarbonylguanidino)-9-O-tetradecanoyl-2,3,4,5,7-pentadeoxy-7-fluoro-D-glycero-D-galacto-non-2-enopyranosoate

50 mg (0.072 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 11 mg (0.11 mmol) of triethylamine and 21 mg (0.086 mmol) of tetradecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 50 mg (yield 77%) of the title compound as a colourless amorphous substance.
Rf= 0.4 (1 : 1 = ethyl acetate : hexane).
[α]_{D}²⁵ = -7.0° (c = 0.10, CHCl₃).
Mass Spectrum (FAB) m/e 898 (M⁺+H).
¹H-Nuclear Magnetic Resonance Spectrum (CDCl₃, 270 MHz) δ (ppm):
0.90 (3H, multiplet);
1.20 - 1.40 (22H, multiplet);
1.48 (9H, singlet);
1.50 (9H, singlet);
1.97 (3H, singlet);
2.35 (2H, triplet, J = 7.5 Hz);
2.95 (1H, doublet, J = 5.0 Hz);
4.20 - 4.60 (6H, multiplet);
5.25 (1H, multiplet);
5.97 (1H, doublet, J = 2.6 Hz);
6.65 (1H, doublet, J = 8.5 Hz);
6.93 (1H, singlet);
7.20 - 7.40 (10H, multiplet);
8.60 (1H, doublet, J = 8.5 Hz).

### 16(ii) 5-Acetamido-4-guanidino-9-O-tetradecanoyl-2,3,4,5,7-pentadeoxy-7-fluoro-D-glycero-D-galacto-non-2-enopyranosoic acid trifluoroacetic acid salt

40 mg (0.05 mmol) of diphenylmethyl 5-acetamido-4-(N,N'-bis-t-butoxycarbonylguanidino)-9-O-tetradecanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 21 mg (yield 64%) of the title compound as a colourless solid.
Rf= 0.4 (2 : 5 : 1 = isopropanol : ethyl acetate : water).
[α]_{D}²⁵ = +22.0° (c = 0.06, CH₃OH).
Mass Spectrum (FAB) m/e 545 (M⁺+H).
¹H-Nuclear Magnetic Resonance Spectrum (CD₃OD, 270 MHz) δ (ppm):
0.85 - 0.95 (3H, multiplet);
1.25 - 1.70 (20H, multiplet);
1.55 - 1.70 (2H, multiplet);
2.00 (3H, singlet);
2.35 (2H, triplet, J = 4.5 Hz);
4.10 - 4.40 (5H, multiplet);
4.45 - 4.70 (1H, multiplet);
5.60 (1H, doublet, J = 2.4 Hz).

### EXAMPLE 17

### 5-Acetamido-4-guanidino-9-O-hexadecanoyl-2,3,4,5,7-pentadeoxy-7-fluoro-D-glycero-D-galacto-non-2-enopyranosoic acid trifluoroacetic acid salt (Compound No. 1-42)

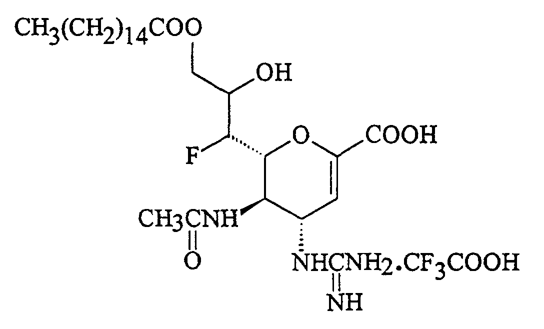

### 17(i) Diphenylmethyl 5-acetamido-4-(N,N'-bis-t-butoxycarbonylguanidino)-9-O-hexadecanoyl-2,3,4,5,7-pentadeoxy-7-fluoro-D-glycero-D-galacto-non-2-enopyranosoate

58 mg (0.08 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 10 mg (0.10 mmol) of triethylamine and 26 mg (0.0946 mmol) of hexadecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 1 : 1 by volume mixture of ethyl acetate and hexane as the eluent, to obtain 50 mg (yield 67%) of the title compound as a colourless amorphous substance.
Rf= 0.4 (1 : 1 = ethyl acetate : hexane).
[α]_{D}²⁵ = -11.0° (c = 0.11, CHCl₃).
Mass Spectrum (FAB) m/e 939 (M⁺+H).
¹H-Nuclear Magnetic Resonance Spectrum (CDCl₃, 270 MHz) δ (ppm):
0.90 (3H, multiplet);
1.20 - 1.40 (22H, multiplet);
1.48 (9H, singlet);
1.50 (9H, singlet);
1.97 (3H, singlet);
2.35 (2H, triplet, J = 7.5 Hz);
2.95 (1H, doublet, J = 5.0 Hz);
4.20 - 4.60 (6H, multiplet);
5.25 (1H, multiplet);
5.97 (1H, doublet, J = 2.6 Hz);
6.65 (1H, doublet, J = 8.5 Hz);
6.93 (1H, singlet);
7.20 - 7.40 (10H, multiplet);
8.60 (1H, doublet, J = 8.5 Hz).

### 17(ii) 5-Acetamido-4-guanidino-9-O-hexadecanoyl-2,3,4,5,7-pentadeoxy-7-fluoro-D-glycero-D-galacto-non-2-enopyranosoic acid trifluoroacetic acid salt

45 mg (0.05 mmol) of diphenylmethyl 5-acetamido-4-(N,N'-bis-t-butoxycarbonylguanidino)-9-O-hexadecanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 28 mg (yield 73%) of the title compound as a colourless solid.
Rf= 0.4 (2 : 5 : 1 = isopropanol : ethyl acetate : water).
[α]_{D}²⁵ = +19.7° (c = 0.1, CH₃OH).
Mass Spectrum (FAB) m/e 573 (M⁺+H).
¹H-Nuclear Magnetic Resonance Spectrum (CD₃OD, 270 MHz) δ (ppm):
0.85 - 0.95 (3H, multiplet);
1.25 - 1.40 (16H, multiplet);
1.55 - 1.70 (2H, multiplet);
2.00 (3H, singlet);
2.35 (2H, triplet, J = 7.0 Hz);
4.10 - 4.40 (5H, multiplet);
4.45 - 4.70 (2H, multiplet);
5.60 (1H, doublet, J = 2.4 Hz).

### EXAMPLE 18

### 5-Acetamido-4-guanidino-9-O-octadecanoyl-2,3,4,5,7-pentadeoxy-7-fluoro-D-glycero-D-galacto-non-2-enopyranosoic acid trifluoroacetic acid salt (Compound No. 1-43)

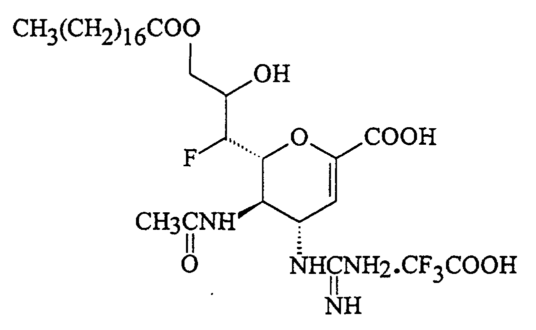

### 18(i) Diphenylmethyl 5-acetamido-4-(N,N'-bis-t-butoxycarbonylguanidino)-9-O-octadecanoyl-2,3,4,5,7-pentadeoxy-7-fluoro-D-glycero-D-galacto-non-2-enopyranosoate

55 mg (0.08 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 10 mg (0.10 mmol) of triethylamine and 29 mg (0.094 mmol) of octadecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 1 : 1 by volume mixture of ethyl acetate and hexane as the eluent, to obtain 51 mg (yield 67%) of the title compound as a colourless amorphous substance.
Rf= 0.5 (1 : 1 = ethyl acetate : hexane).
[α]D²⁵ = -8.1° (c = 0.16, CHCl₃).
Mass Spectrum (FAB) m/e 967 (M⁺+H).
¹H-Nuclear Magnetic Resonance Spectrum (CDCl₃, 270 MHz) δ (ppm):
0.90 (3H, multiplet);
1.20 - 1.40 (30H, multiplet);
1.48 (9H, singlet);
1.50 (9H, singlet);
1.97 (3H, singlet);
2.35 (2H, triplet, J = 7.5 Hz);
2.95 (1H, doublet, J = 5.0 Hz);
4.20 - 4.60 (6H, multiplet);
5.25 (1H, multiplet);
5.97 (1H, doublet, J = 2.6 Hz);
6.65 (1H, doublet, J = 8.5 Hz);
6.93 (1H, singlet);
7.20 - 7.40 (10H, multiplet);
8.60 (1H, doublet, J = 8.5 Hz).

### 18(ii) 5-Acetamido-4-guanidino-9-O-octadecanoyl-2,3,4,5,7-pentadeoxy-7-fluoro-D-glycero-D-galacto-non-2-enopyranosoic acid trifluoroacetic acid salt

45 mg (0.047 mmol) of diphenylmethyl 5-acetamido-4-(N,N'-bis-t-butoxycarbonylguanidino)-9-O-octadecanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 24 mg (yield 62%) of the title compound as a colourless solid.
Rf= 0.4 (2 : 5 : 1 = isopropanol: ethyl acetate : water).
[α]D²⁵ = +14.3° (c = 0.15, CH₃OH).
Mass Spectrum (FAB) m/e 601 (M⁺+H).
¹H-Nuclear Magnetic Resonance Spectrum (CD₃OD, 270 MHz) δ (ppm):
0.85 - 0.95 (3H, multiplet);
1.25 - 1.40 (28H, multiplet);
1.55 - 1.70 (2H, multiplet);
2.00 (3H, singlet);
2.35 (2H, triplet, J = 7.0 Hz);
4.10 - 4.40 (5H, multiplet);
4.45 - 4.70 (2H, multiplet);
5.60 (1H, doublet, J = 2.4 Hz).

### EXAMPLE 19

### 5-Acetamido-4-guanidino-8,9-di-O-acetyl-2,3,4,5,7-pentadeoxy-7-fluoro-D-glycero-D-galacto-non-2-enopyranosoic acid trifluoroacetic acid salt (Compound No. 1-54)

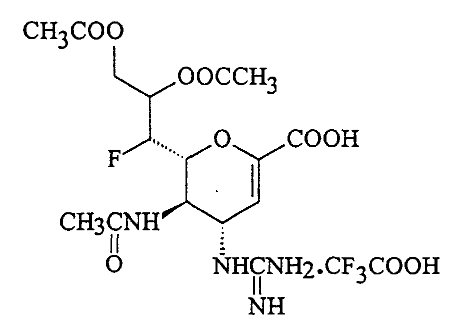

### 19(i) Diphenylmethyl 5-acetamido-4-(N,N'-bis-t-butoxycarbonylguanidino)-8,9-di-O-acetyl-2,3,4,5,7-pentadeoxy-7-fluoro-D-glycero-D-galacto-non-2-enopyranosoate

58 mg (0.083 mmol) of diphenylmethyl 5-acetamido-4-(N,N'-bis-t-butoxy-carbonylguanidino)-2,3,4,5,7-pentadeoxy-7-fluoro-D-glycero-D-galacto-non-2-enopyranosoate [prepared as described in Example 13(i)] were dissolved in 2 ml of pyridine, and 0.5 ml of acetic anhydride was added to the resulting solution, whilst ice-cooling. The mixture was then stirred at room temperature for 3 hours. At the end of this time, the reaction mixture was poured into a 2-layer solution of 5 ml of ethyl acetate and 3 ml of a 1 N aqueous hydrochloric acid solution, and the organic layer was separated and washed with a saturated aqueous solution of sodium hydrogencarbonate and then with a saturated aqueous solution of sodium chloride. It was then dried over anhydrous sodium sulphate, and the solvent was removed by distillation under reduced pressure. The resulting residue was purified by silica gel column chromatography, using a 1 : 1 by volume mixture of hexane and ethyl acetate as the eluent, to obtain 59 mg (yield 91%) of the title compound as a colourless amorphous substance.
Rf= 0.47 (1 : 1 = hexane : ethyl acetate).
[α]_{D}²⁵ = +2.5° (c = 0.12, CHCl₃).
Mass Spectrum (FAB) m/e 785 (M⁺+H).
¹H-Nuclear Magnetic Resonance Spectrum (CDCl₃, 270 MHz) δ (ppm):
1.48 (9H, singlet);
1.49 (9H, singlet);
1.99 (3H, singlet);
2.04 (3H, singlet);
2.07 (3H, singlet);
4.00 - 4.30 (3H, multiplet);
4.75 (1H, doublet, J = 13 Hz);
4.80 (1H, doublet of doublets, J = 7.5 & 45 Hz);
5.20 (1H, doubled doublet of doublets, J = 1.0, 9.0 & 9.0 Hz);
5.50 (1H, multiplet);
5.92 (1H, doublet, J = 2.4 Hz);
6.72 (1H, doublet, J = 7.5 Hz);
6.94 (1H, singlet);
7.20 - 7.50 (10H, multiplet);
8.60 (1H, doublet, J = 8.5 Hz).

### 19(ii) 5-Acetamido-4-guanidino-8,9-di-O-acetyl-2,3,4,5,7-pentadeoxy-7-fluoro-D-glycero-D-galacto-non-2-enopyranosoic acid trifluoroacetic acid salt

50 mg (0.064 mmol) of diphenylmethyl 5-acetamido-4-(N,N'-bis-t-butoxycarbonylguanidino)-8,9-di-O-acetyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 23 mg (yield 56%) of the title compound as a colourless solid.
Rf= 0.16 (2 : 5 : 1 = isopropanol : ethyl acetate : water).
[α]_{D}²⁵ = +36.9° (c = 0.15, CH₃OH).
Mass Spectrum (FAB) m/e 419 (M⁺+H).
¹H-Nuclear Magnetic Resonance Spectrum (CD₃OD, 270 MHz) δ (ppm):
2.00 (3H, singlet);
2.03 (3H, singlet);
2.05 (3H, singlet);
4.10 - 4.40 (4H, multiplet);
4.70 - 5.00 (2H, multiplet);
5.50 (1H, multiplet);
5.70 (1H, doublet, J = 3.0 Hz).

### EXAMPLE 20

### 5-Acetamido-4-guanidino-8,9-di-O-propionyl-2,3,4,5,7-pentadeoxy-7-fluoro-D-glycero-D-galacto-non-2-enopyranosoic acid trifluoroacetic acid salt (Compound No. 1-55)

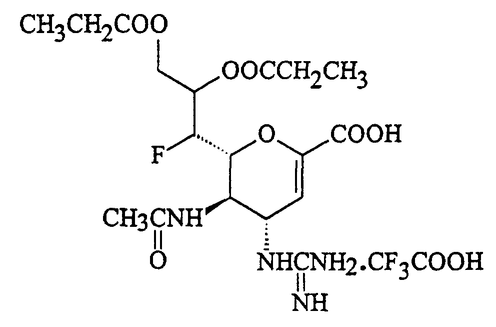

### 20(i) Diphenylmethyl 5-acetamido-4-(N,N'-bis-t-butoxycarbonylguanidino)-8,9-di-O-propionyl-2,3,4,5,7-pentadeoxy-7-fluoro-D-glycero-D-galacto-non-2-enopyranosoate

52 mg (0.074 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of pyridine, and 0.5 ml of propionic anhydride was added to the resulting solution, whilst ice-cooling. The mixture was then stirred at room temperature for 3 hours. At the end of this time, the reaction mixture was poured into a 2-layer solution of 5 ml of ethyl acetate and 3 ml of a 1 N aqueous hydrochloric acid solution, and the organic layer was separated and washed with a saturated aqueous solution of sodium hydrogencarbonate and then with a saturated aqueous solution of sodium chloride. It was then dried over anhydrous sodium sulphate, and the solvent was removed by distillation under reduced pressure. The resulting residue was purified by silica gel column chromatography, using a 1 : 1 by volume mixture of hexane and ethyl acetate as the eluent, to obtain 54 mg (yield 90%) of the title compound as a colourless amorphous substance.
Rf= 0.54 (1 : 1 = hexane : ethyl acetate).
[α]_{D}²⁵ = +9.6° (c = 0.14, CHCl₃).
Mass Spectrum (FAB) m/e 813 (M⁺+H).
¹H-Nuclear Magnetic Resonance Spectrum (CDCl₃, 270 MHz) δ (ppm):
1.11 (9H, triplet, 7.5 Hz);
1.48 (9H, singlet);
1.49 (9H, singlet);
2.04 (3H, singlet);
2.20 - 2.35 (4H, multiplet);
4.00-4.30 (3H, multiplet);
4.75 (1H, doublet, J = 13 Hz);
4.80 (1H, doublet of doublets; J = 7.5 & 45 Hz);
5.20 (1H, doubled doublet of doublets, J = 1.0, 9.0 & 9.0 Hz);
5.50 (1H, multiplet);
5.92 (1H, doublet, J = 2.4 Hz);
6.72 (1H, doublet, J = 7.5 Hz);
6.94 (1H, singlet);
7.20 - 7.50 (10H, multiplet);
8.60 (1H, doublet, J = 8.5 Hz).

### 20(ii) 5-Acetamido-4-guanidino-8,9-di-O-propionyl-2,3,4,5,7-pentadeoxy-7-fluoro-D-glycero-D-galacto-non-2-enopyranosoic acid trifluoroacetic acid salt

46 mg (0.057 mmol) of diphenylmethyl 5-acetamido-4-(N,N'-bis-t-butoxycarbonylguanidino)-8,9-di-Q-propionyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 24 mg (yield 63%) of the title compound as a colourless solid.
Rf= 0.25 (2 : 5 : 1 = isopropanol : ethyl acetate : water).
[α]D²⁵ = +32.3° (c = 0.14, CH₃OH).
Mass Spectrum (FAB) m/e 447 (M⁺+H).
¹H-Nuclear Magnetic Resonance Spectrum (CD₃OD, 270 MHz) δ (ppm):
1.10 (6H, triplet, J = 7.5 Hz);
2.00 (3H, singlet);
2.35 (4H, quartet, J = 7.5 Hz);
4.10 - 4.40 (4H, multiplet);
4.70 - 5.00 (2H, multiplet);
5.50 (1H, multiplet);
5.60 (1H, doublet, J = 3.0 Hz).

### EXAMPLE 21

### 5-Acetamido-4-guanidino-8,9-di-O-hexanoyl-2,3,4,5,7-pentadeoxy-7-fluoro-D-glycero-D-galacto-non-2-enopyranosoic acid trifluoroacetic acid salt (Compound No. 1-58)

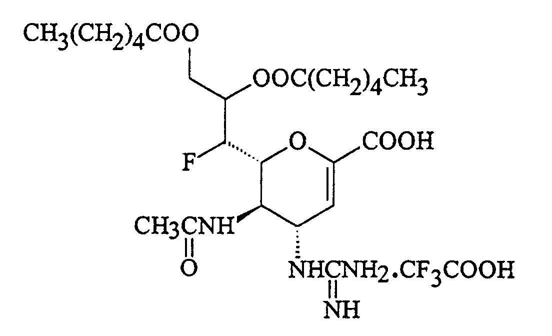

### 21(i) Diphenylmethyl 5-acetamido-4-(N,N'-bis-t-butoxycarbonylguanidino)-8,9-di-O-hexanoyl-2,3,4,5,7-pentadeoxy-7-fluoro-D-glycero-D-galacto-non-2-enopyranosoate

44 mg (0.063 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 19 mg (0.17 mmol) of 4-dimethylaminopyridine and 19 mg (0.14 mmol) of hexanoyl chloride were added to the resulting solution, whilst ice-cooling. The mixture was then stirred at room temperature for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 3 : 1 by volume mixture of hexane and ethyl acetate as the eluent, to obtain 49 mg (yield 87%) of the title compound as a colourless amorphous substance.
Rf= 0.28 (3 : 1 = hexane : ethyl acetate).
[α]_{D}²⁵ = +6.5° (c = 0.10, CHCl₃).
Mass Spectrum (FAB) m/e 897 (M⁺+H).
¹H-Nuclear Magnetic Resonance Spectrum (CDCl₃, 270 MHz) δ (ppm):
0.80 - 1.00 (6H, multiplet);
1.20 - 1.40 (8H, multiplet);
1.48 (9H, singlet);
1.49 (9H, singlet);
1.50 - 1.70 (4H, multiplet);
1.98 (3H, singlet);
2.20 - 2.30 (4H, multiplet);
4.00 - 4.30 (3H, multiplet);
4.75 (1H, doublet, J = 13 Hz);
4.80 (1H, doublet of doublets, J = 7.4 & 45 Hz);
5.18 (1H, doubled doublet of doublets, J = 1.0, 9.0 & 9.0 Hz);
5.50 (1H, multiplet);
5.93 (1H, doublet, J = 2.4 Hz);
6.70 (1H, doublet, J = 7.5 Hz);
6.93 (1H, singlet);
7.20 - 7.50 (10H, multiplet);
8.60 (1H, doublet, J = 8.5 Hz).

### 21(ii) 5-Acetamido-4-guanidino-8,9-di-O-hexanoyl-2,3,4,5,7-pentadeoxy-7-fluoro-D-glycero-D-galacto-non-2-enopyranosoic acid trifluoroacetic acid salt

42 mg (0.047 mmol) of diphenylmethyl 5-acetamido-4-(N,N'-bis-t-butoxycarbonylguanidino)-8,9-di-O-hexanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 21 mg (yield 59%) of the title compound as a colourless solid.
Rf= 0.34 (2 : 5 : 1 = isopropanol: ethyl acetate : water).
[α]_{D}²⁵ = +23.7° (c = 0.11, CH₃OH).
Mass Spectrum (FAB) m/e 531 (M⁺+H).
¹H-Nuclear Magnetic Resonance Spectrum (CD₃OD, 270 MHz) δ (ppm):
0.85 - 0.95 (6H, multiplet);
1.20 - 1.40 (8H, multiplet);
1.55 - 1.70 (4H, multiplet);
2.00 (3H, singlet);
2.30 (2H, triplet, J = 7.5 Hz);
4.10 - 4.40 (4H, multiplet);
4.70 - 5.00 (2H, multiplet);
5.55 (1H, multiplet);
5.65 (1H, doublet, J = 3.0 Hz).

### EXAMPLE 22

### 5-Acetamido-4-guanidino-8,9-di-O-decanoyl-2,3,4,5,7-pentadeoxy-7-fluoro-D-glycero-D-galacto-non-2-enopyranosoic acid trifluoroacetic acid salt (Compound No. 1-61)

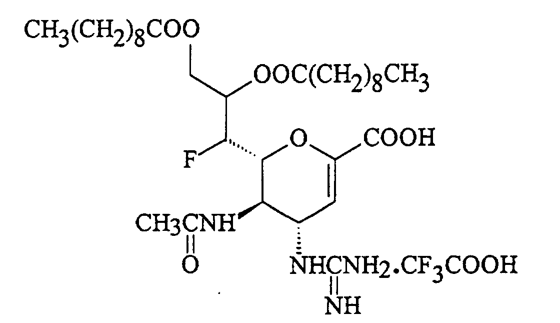

### 22(i) Diphenylmethyl 5-acetamido-4-(N,N'-bis-t-butoxycarbonylguanidino)-8,9-di-O-decanoyl-2,3,4,5,7-pentadeoxy-7-fluoro-D-glycero-D-galacto-non-2-enopyranosoate

42 mg (0.06 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 18 mg (0.15 mmol) of 4-dimethylaminopyridine and 26 mg (0.14 mmol) of decanoyl chloride were added to the resulting solution, whilst ice-cooling. The mixture was then stirred at room temperature for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 3 : 1 by volume mixture of hexane and ethyl acetate as the eluent, to obtain 52 mg (yield 86%) of the title compound as a colourless amorphous substance.
Rf= 0.28 (3 : 1 = hexane : ethyl acetate).
[α]_{D}²⁵ = +13.3° (c = 0.17, CHCl₃).
Mass Spectrum (FAB) m/e 1009 (M⁺+H).
¹H-Nuclear Magnetic Resonance Spectrum (CDCl₃, 270 MHz) δ (ppm):
0.80 - 0.90 (6H, multiplet);
1.20 - 1.40 (24H, multiplet);
1.48 (9H, singlet);
1.49 (9H, singlet);
1.50 - 1.65 (4H, multiplet);
1.98 (3H, singlet);
2.20 - 2.30 (4H, multiplet);
4.00 - 4.30 (3H, multiplet);
4.75 (1H, doublet, J = 13 Hz);
4.80 (1H, doublet of doublets, J = 7.5 & 45 Hz);
5.20 (1H, doubled doublet of doublets, J = 1.0, 9.0 & 9.0 Hz);
5.50 (1H, multiplet);
5.92 (1H, doublet, J = 2.4 Hz);
6.65 (1H, doublet, J = 7.5 Hz);
6.93 (1H, singlet);
7.20 - 7.50 (10H, multiplet);
8.60 (1H, doublet, J = 8.5 Hz).

### 22(ii) 5-Acetamido-4-guanidino-8,9-di-O-decanoyl-2,3,4,5,7-pentadeoxy-7-fluoro-D-glycero-D-galacto-non-2-enopyranosoic acid trifluoroacetic acid salt

45 mg (0.045 mmol) of diphenylmethyl 5-acetamido-4-(N,N'-bis-t-butoxycarbonylguanidino)-8,9-di-O-decanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 29 mg (yield 75%) of the title compound as a colourless solid.
Rf= 0.3 (2 : 5 : 1 = isopropanol : ethyl acetate : water).
[α]D²⁵ = +20.6° (c = 0.14, CH₃OH).
Mass Spectrum (FAB) m/e 643 (M⁺+H).
¹H-Nuclear Magnetic Resonance Spectrum (CD₃OD, 270 MHz) δ (ppm):
0.85 - 0.95 (6H, multiplet);
1.20 - 1.40 (24H, multiplet);
1.55 - 1.70 (4H, multiplet);
2.00 (3H, singlet);
2.30 (2H, triplet, J = 7.0 Hz);
4.10 - 4.40 (4H, multiplet);
4.70 - 5.00 (2H, multiplet);
5.55 (1H, multiplet);
5.70 (1H, doublet, J = 3.0 Hz).

### EXAMPLE 23

### 5-Acetamido-4-guanidino-8,9-di-O-tetradecanoyl-2,3,4,5,7-pentadeoxy-7-fluoro-D-glycero-D-galacto-non-2-enopyranosoic acid trifluoroacetic acid salt (Compound No. 1-63)

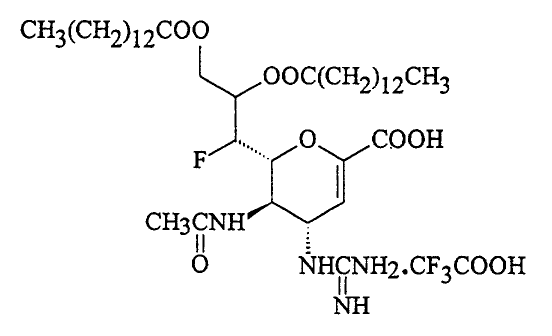

### 23(i) Diphenylmethyl 5-acetamido-4-(N,N'-bis-t-butoxycarbonylguanidino)-8,9-di-O-tetradecanoyl-2,3,4,5,7-pentadeoxy-7-fluoro-D-glycero-D-galacto-non-2-enopyranosoate

43 mg (0.06 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 19 mg (0.15 mmol) of 4-dimethylaminopyridine and 26 mg (0.14 mmol) of tetradecanoyl chloride were added to the resulting solution, whilst ice-cooling. The mixture was then stirred at room temperature for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 3 : 1 by volume mixture of hexane and ethyl acetate as the eluent, to obtain 60 mg (yield 87%) of the title compound as a colourless amorphous substance.
Rf= 0.28 (3 : 1 = hexane : ethyl acetate).
[α]D²⁵ = +15.5° (c = 0.11, CHCl₃).
Mass Spectrum (FAB) m/e 1121 (M⁺+H).
¹H-Nuclear Magnetic Resonance Spectrum (CDCl₃, 270 MHz) δ (ppm):
0.80 - 0.90 (6H, multiplet);
1.20 - 1.40 (40H, multiplet);
1.48 (9H, singlet);
1.49 (9H, singlet);
1.50 - 1.65 (4H, multiplet);
1.98 (3H, singlet);
2.20 - 2.30 (4H, multiplet);
4.00 - 4.30 (3H, multiplet);
4.75 (1H, doublet, J = 13 Hz);
4.80 (1H, doublet of doublets, J = 7.5 & 45 Hz);
5.18 (1H, doubled doublet of doublets, J = 1.0, 9.0 & 9.0 Hz);
5.50 (1H, multiplet);
5.93 (1H, doublet, J = 2.4 Hz);
6.66 (1H, doublet, J = 7.5 Hz);
6.93 (1H, singlet);
7.20 - 7.50 (10H, multiplet);
8.60 (1H, doublet, J = 8.5 Hz).

### 23(ii) 5-Acetamido-4-guanidino-8,9-di-O-tetradecanoyl-2,3,4,5,7-pentadeoxy-7-fluoro-D-glycero-D-galacto-non-2-enopyranosoic acid trifluoroacetic acid salt

52 mg (0.046 mmol) of diphenylmethyl 5-acetamido-4-(N,N'-bis-t-butoxycarbonylguanidino)-8,9-di-O-tetradecanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 38 mg (yield 83%) of the title compound as a colourless solid.
Rf= 0.3 (2 : 5 : 1 = isopropanol : ethyl acetate : water).
[α]_{D}²⁵ = +28.0° (c = 0.16, CH₃OH).
Mass Spectrum 755 (FAB) m/e 545 (M⁺+H).
¹H-Nuclear Magnetic Resonance Spectrum (CD₃OD, 270 MHz) δ (ppm):
0.85 - 0.95 (6H, multiplet);
1.20 - 1.40 (40H, multiplet);
1.55 - 1.70 (4H, multiplet);
2.00 (3H, singlet);
2.30 (2H, triplet, J = 7.0 Hz);
4.10 - 4.40 (4H, multiplet);
4.70 - 5.00 (2H, multiplet);
5.55 (1H, multiplet);
5.70 (1H, doublet, J = 3.0 Hz).

### EXAMPLE 24

### 5-Acetamido-4-guanidino-8,9-di-O-hexadecanoyl-2,3,4,5,7-pentadeoxy-7-fluoro-D-glycero-D-galacto-non-2-enopyranosoic acid trifluoroacetic acid salt (Compound No. 1-64)

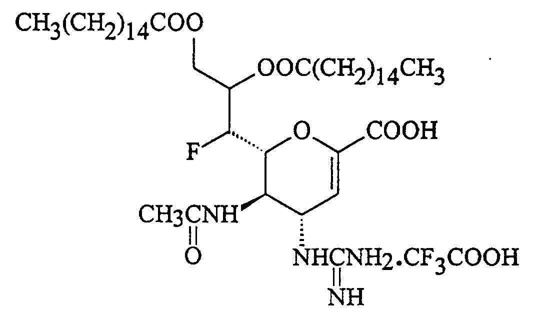

### 24(i) Diphenylmethyl 5-acetamido-4-(N,N'-bis-t-butoxycarbonylguanidino)-8,9-di-O-hexadecanoyl-2,3,4,5,7-pentadeoxy-7-fluoro-D-glycero-D-galacto-non-2-enopyranosoate

37 mg (0.053 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 16 mg (0.13 mmol) of 4-dimethylaminopyridine and 34 mg (0.12 mmol) of hexadecanoyl chloride were added to the resulting solution, whilst ice-cooling. The mixture was then stirred at room temperature for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 3 : 1 by volume mixture of hexane and ethyl acetate as the eluent, to obtain 52 mg (yield 84%) of the title compound as a colourless amorphous substance.
Rf= 0.3 (3 : 1 = hexane : ethyl acetate).
[α]D²⁵ = +10.9° (c = 0.11, CHCl₃).
Mass Spectrum (FAB) m/e 1177 (M⁺+H).
¹H-Nuclear Magnetic Resonance Spectrum (CDCl₃, 270 MHz) δ (ppm):
0.80 - 0.90 (6H, multiplet);
1.20 - 1.40 (48H, multiplet);
1.48 (9H, singlet);
1.49 (9H, singlet);
1.50 - 1.65 (4H, multiplet);
1.98 (3H, singlet);
2.20 - 2.30 (4H, multiplet);
4.00 - 4.30 (3H, multiplet);
4.75 (1H, doublet, J = 13 Hz);
4.80 (1H, doublet of doublets, J = 7.5 & 45 Hz);
5.20 (1H, doubled doublet of doublets, J = 1.0, 9.0 & 9.0 Hz);
5.50 (1H, multiplet);
5.92 (1H, doublet, J = 2.4 Hz);
6.65 (1H, doublet, J = 7.5 Hz);
6.93 (1H, singlet);
7.20 - 7.50 (10H, multiplet);
8.60 (1H, doublet, J = 8.5 Hz).

### 24(ii) 5-Acetamido-4-guanidino-8,9-di-O-hexadecanoyl-2,3,4,5,7-pentadeoxy-7-fluoro-D-glycero-D-galacto-non-2-enopyranosoic acid trifluoroacetic acid salt

42 mg (0.036 mmol) of diphenylmethyl 5-acetamido-4-(N,N'-bis-t-butoxycarbonylguanidino)-8,9-di-O-hexadecanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 22 mg (yield 59%) of the title compound as a colourless solid.
Rf= 0.4 (2 : 5 : 1 = isopropanol : ethyl acetate : water).
[α]_{D}²⁵ = +15.3° (c = 0.11, CH₃OH).
Mass Spectrum (FAB) m/e 811 (M⁺+H).
¹H-Nuclear Magnetic Resonance Spectrum (CD₃OD, 270 MHz) δ (ppm):
0.85 - 0.95 (6H, multiplet);
1.20 - 1.40 (48H, multiplet);
1.55 - 1.70 (4H, multiplet);
2.00 (3H, singlet);
2.30 (2H, triplet, J = 7.0 Hz);
4.10 - 4.40 (4H, multiplet);
4.70 - 5.00 (2H, multiplet);
5.55 (1H, multiplet);
5.70 (1H, doublet, J = 3.0 Hz).

### EXAMPLE 25

### 5-Acetamido-4-guanidino-8.9-di-O-octadecanoyl-2,3,4,5,7-pentadeoxy-7-fluoro-D-glycero-D-galacto-non-2-enopyranosoic acid trifluoroacetic acid salt (Compound No. 65-1)

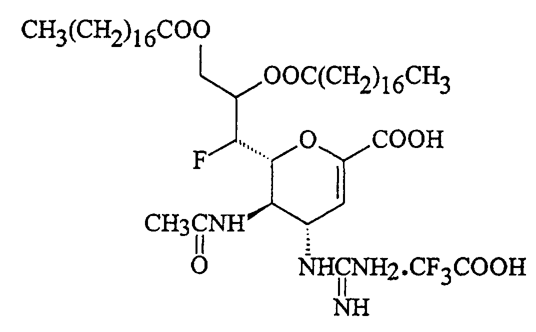

### 25(i) Diphenylmethyl 5-acetamido-4-(N,N'-bis-t-butoxycarbonylguanidino)-8.9-di-O-octadecanoyl-2,3,4,5,7-pentadeoxy-7-fluoro-D-glycero-D-galacto-non-2-enopyranosoate

35 mg (0.05 mmol) of diphenylmethyl 5-acetamido-4-(N,N'-bis-t-butoxycarbonylguanidino)-2,3,4,5,7-pentadeoxy-7-fluoro-D-glycero-D-galacto-non-2-enopyranosoate [prepared as described in Example 13(i)] were dissolved in 2 ml of methylene chloride, and 15 mg (0.13 mmol) of triethylamine and 35 mg (0.115 mmol) of octadecanoyl chloride were added to the resulting solution, whilst ice-cooling. The mixture was then stirred at room temperature for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 3 : 1 by volume mixture of hexane and ethyl acetate as the eluent, to obtain 57 mg (yield 93%) of the title compound as a colourless amorphous substance.
Rf= 0.3 (3 : 1 = hexane : ethyl acetate).
[α]D²⁵ = +11.2° (c = 0.18, CHCl₃).
Mass Spectrum (FAB) m/e 1233 (M⁺+H).
¹H-Nuclear Magnetic Resonance Spectrum (CDCl₃, 270 MHz) δ (ppm):
0.80 - 0.90 (6H, multiplet);
1.20 - 1.40 (56H, multiplet);
1.48 (9H, singlet);
1.49 (9H, singlet);
1.50 - 1.65 (4H, multiplet);
1.98 (3H, singlet);
2.20 - 2.30 (4H, multiplet);
4.00 - 4.30 (3H, multiplet);
4.75 (1H, doublet, J = 13 Hz);
4.80 (1H, doublet of doublets, J = 7.5 & 45 Hz);
5.20 (1H, doubled doublet of doublets, J = 1.0, 9.0 & 9.0 Hz);
5.50 (1H, multiplet);
5.92 (1H, doublet, J = 2.4 Hz);
6.65 (1H, doublet, J = 7.5 Hz);
6.93 (1H, singlet);
7.20 - 7.50 (10H, multiplet);
8.60 (1H, doublet, J = 8.5 Hz).

### 25(ii) 5-Acetamido-4-guanidino-8,9-di-O-octadecanoyl-2,3,4,5,7-pentadeoxy-7-fluoro-D-glycero-D-galacto-non-2-enopyranosoic acid trifluoroacetic acid salt

47 mg (0.04 mmol) of diphenylmethyl 5-acetamido-4-(N,N'-bis-t-butoxycarbonylguanidino)-8,9-di-O-octadecanoyl-2,3,4,5,7-pentadeoxy-7-fluoro-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 28 mg (yield 67%) of the title compound as a colourless solid.
Rf= 0.4 (2 : 5 : 1 = isopropanol : ethyl acetate : water).
[α]D²⁵ = +14.5° (c = 0.14, CH₃OH).
Mass Spectrum (FAB) m/e 867 (M⁺+H).
¹H-Nuclear Magnetic Resonance Spectrum (CD₃OD, 270 MHz) δ (ppm):
0.85 - 0.95 (6H, multiplet);
1.20 - 1.40 (56H, multiplet);
1.55 - 1.70 (4H, multiplet);
2.00 (3H, singlet);
2.30 (2H, triplet, J = 7.0 Hz);
4.10 - 4.40 (4H, multiplet);
4.70 - 5.00 (2H, multiplet);
5.55 (1H, multiplet);
5.70 (1H, doublet, J = 3.0 Hz).

### EXAMPLE 26

### Hexadecyl 5-acetamido-4-guanidino-2,3,4,5,7-pentadeoxy-7-fluoro-D-glycero-D-galacto-non-2-enopyranosoate trifluoroacetic acid salt (Compound No. 1-6)

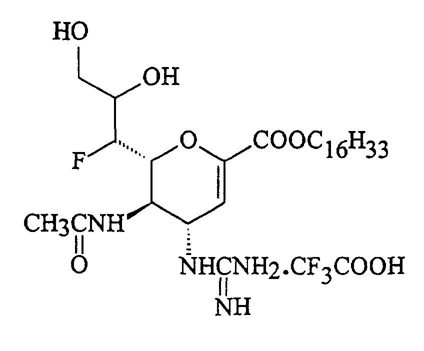

### 26(i) Hexadecyl 5-acetamido-4-azido-8,9-O-isopropylidene-2,3,4,5,7-pentadeoxy-7-fluoro-D-glycero-D-galacto-non-2-enopyranosoate

275 mg (0.74 mmol) of methyl 5-acetamido-4-azido-8,9-O-isopropylidene-2,3,4,5,7-pentadeoxy-7-fluoro-D-glycero-D-galacto-non-2-enopyranosoate [prepared as described in Example 7(i)] were dissolved in 7 ml of a 6 : 1 by volume mixture of methanol and water, and 0.82 ml of a 1 M aqueous solution of potassium hydroxide was added to the resulting solution. The mixture was then stirred at room temperature for 2 hours. At the end of this time, the solvent was removed by distillation under reduced pressure, and dried under reduced pressure at room temperature for 2 hours to obtain a pale yellow solid. This solid was dissolved in 8 ml of acetonitrile, and 195 mg (0.74 mmol) of 18-crown-6 and 677 mg (2.2 mmol) of hexadecyl bromide were added to the resulting solution. The mixture was then stirred at 80°C for 30 minutes. At the end of this time, the solvent was removed by distillation under reduced pressure. A 2-layer solution of 15 ml of ethyl acetate and 10 ml of a saturated aqueous solution of sodium hydrogencarbonate was poured onto the resulting residue, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 240 mg (yield 56%) of the title compound as a colourless amorphous substance.
Rf= 0.50 (20 : 1 = methylene chloride : methanol).
[α]D²⁵ = +65.0° (c = 0.5, CHCl₃).
Mass Spectrum (FAB) m/e 583 (M⁺+H).
¹H-Nuclear Magnetic Resonance Spectrum (CDCl₃, 270 MHz) δ (ppm):
0.80 - 0.95 (3H, multiplet);
1.20 - 1.40 (28H, multiplet);
1.37 (3H, singlet);
1.42 (3H, singlet);
1.60 - 1.80 (2H, multiplet);
2.05 (3H, singlet);
4.10 - 4.30 (3H, multiplet);
4.40 (1H, multiplet);
4.72 (1H, doubled doublet of doublets, J = 1.3, 5.3 & 47.0 Hz);
4.90 (1H, doublet of doublets, J = 2.4 & 9.3 Hz);
4.92 (1H, doubled doublet of doublets, J = 1.4, 10.0 & 28.0 Hz);
5.92 (1H, doublet, J = 7.2 Hz);
5.94 (1H, doublet, J = 2.6 Hz).

### 26(ii) Hexadecyl 5-acetamido-4-(N,N'-bis-t-butoxycarbonylguanidino)-8,9-O-isopropylidene-2,3,4,5,7-pentadeoxy-7-fluoro-D-glycero-D-galacto-non-2-enopyranosoate

240 mg (0.41 mmol) of hexadecyl 5-acetamido-4-azido-8,9-O-isopropylidene-2,3,4,5,7-pentadeoxy-7-fluoro-D-glycero-D-galacto-non-2-enopyranosoate [prepared as described in step (i) above] were dissolved in 10 ml of methanol, and 60 mg of a Lindlar catalyst were added to the resulting solution. The mixture was then stirred under a hydrogen atmosphere for 2 hours. At the end of this time, the catalyst was separated by filtration, and the solvent was removed by distillation under reduced pressure. The resulting residue was dissolved in 4 ml of dimethylformamide, and 145 mg (0.53 mmol) of N,N-di-t-butoxycarbonylthiourea, 106 mg (1.05 mmol) of triethylamine and 142 mg (0.53 mmol) of mercuric chloride were added to the resulting solution. The mixture was then stirred at room temperature for 1 hour. At the end of this time, the solid was separated by filtration, and the filtrate was poured into a 2-layer solution of 15 ml of ethyl acetate and 10 ml of a saturated aqueous solution of sodium hydrogencarbonate. The organic layer was washed with a saturated aqueous solution of sodium chloride, and dried over anhydrous sodium sulphate, after which the solvent was removed by distillation under reduced pressure. The resulting residue was purified by silica gel column chromatography, using a 1 : 2 by volume mixture of ethyl acetate and hexane as the eluent, to obtain 220 mg (yield 67%) of the title compound as a colourless viscous substance.
Rf= 0.40 (20 : 1 = methylene chloride : methanol).
[α]_{D}²⁵ = -11.6° (c = 0.06, CHCl₃).
Mass Spectrum (FAB) m/e 799 (M⁺+H).
¹H-Nuclear Magnetic Resonance Spectrum (CDCl₃, 270 MHz) δ (ppm):
0.80 - 0.95 (3H, multiplet);
1.20 - 1.40 (28H, multiplet);
1.37 (3H, singlet);
1.41 (3H, singlet);
1.49 (9H, singlet);
1.50 (9H, singlet);
1.60 - 1.80 (2H, multiplet);
1.97 (3H, singlet);
4.10 - 4.30 (4H, multiplet);
4.40 - 4.65 (2H, multiplet);
5.20 (1H, doubled doublet of doublets, J = 2.4, 7.5 & 7.5 Hz);
5.83 (1H, doublet, J = 2.4Hz);
5.65 (1H, doublet, J = 7.0 Hz);
8.60 (1H, doublet, J = 8.5 Hz).

### 26(iii) Hexadecyl 5-acetamido-4-guanidino-2,3,4,5,7-pentadeoxy-7-fluoro-D-glycero-D-galacto-non-2-enopyranosoate trifluoroacetic acid salt

200 mg (0.25 mmol) of hexadecyl 5-acetamido-4-(N,N'-bis-t-butoxycarbonylguanidino)-8,9-O-isopropylidene-2,3,4,5,7-pentadeoxy-7-fluoro-D-glycero-D-galacto-non-2-enopyranosoate [prepared as described in step (i) above] were dissolved in 10 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 8 : 1 by volume mixture of isopropanol, ethyl acetate and water as the eluent, to obtain 130 mg (yield 77%) of the title compound as a colourless solid.
Rf= 0.55 (5 : 1 : 1 = t-butanol : acetic acid : water).
[α]_{D}²⁵ = +14.7° (c = 0.1, CH₃OH).
Mass Spectrum (FAB) m/e 559 (M⁺+H).
¹H-Nuclear Magnetic Resonance Spectrum (D₂O, 270 MHz) δ (ppm):
0.70 - 0.85 (3H, multiplet);
1.10 - 1.30 (28H, multiplet);
1.60 - 1.80 (2H, multiplet);
2.00 (3H, singlet);
3.60 - 3.85 (2H, multiplet);
4.00 - 4.30 (3H, multiplet);
4.50 - 4.80 (2H, multiplet);
5.88 (1H, singlet).

### EXAMPLE 27

### Octadecyl 5-acetamido-4-guanidino-2,3,4,5,7-pentadeoxy-7-fluoro-D-glycero-D-galacto-non-2-enopyranosoate trifluoroacetic acid salt (Compound No. 1-7)

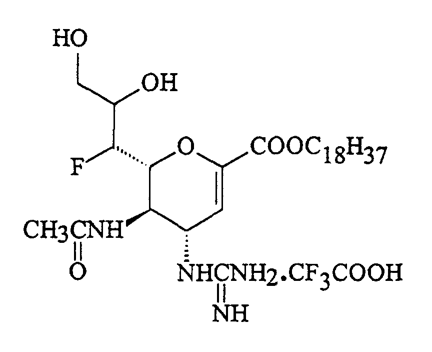

### 27(i) Octadecyl 5-acetamido-4-azido-8,9-O-isopropylidene-2,3,4,5,7-pentadeoxy-7-fluoro-D-glycero-D-galacto-non-2-enopyranosoate

45 mg (0.12 mmol) of methyl 5-acetamido-4-azido-8,9-O-isopropylidene-2,3,4,5,7-pentadeoxy-7-fluoro-D-glycero-D-galacto-non-2-enopyranosoate [prepared as described in Example 7(i)] were dissolved in 4 ml of a 6 : 1 by volume mixture of methanol and water, and 0.13 ml of a 1 M aqueous solution of potassium hydroxide was added to the resulting solution. The mixture was then stirred at room temperature for 2 hours. At the end of this time, the solvent was removed by distillation under reduced pressure, and the residue was dried under reduced pressure at room temperature for 2 hours to obtain a pale yellow solid. This solid was dissolved in 2 ml of acetonitrile, and 32 mg (0.12 mmol) of 18-crown-6 and 121 mg (0.36 mmol) of octadecyl bromide were added to the resulting solution. The mixture was then stirred at 80°C for 30 minutes. The resulting residue was poured into a 2-layer solution of 7 ml of ethyl acetate and 5 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 2 : 1 by volume mixture of hexane and ethyl acetate as the eluent, to obtain 42 mg (yield 58%) of the title compound as a colourless amorphous substance.
Rf= 0.50 (20 : 1 = methylene chloride : methanol).
[α]_{D}²⁵ = +76° (c = 0.05, CHCl₃).
Mass Spectrum (FAB) m/e 611 (M⁺+H).
¹H-Nuclear Magnetic Resonance Spectrum (CDCl₃, 270 MHz) δ (ppm):
0.85 - 0.95 (3H, multiplet);
1.20 - 1.40 (32H, multiplet);
1.37 (3H, singlet);
1.42 (3H, singlet);
1.60 - 1.80 (2H, multiplet);
2.05 (3H, singlet);
4.10 - 4.30 (3H, multiplet);
4.40 (1H, multiplet);
4.72 (1H, doubled doublet of doublets, J = 1.3, 5.3 & 47.0 Hz);
4.90 (1H, doublet of doublets, J = 2.6 & 9.5 Hz);
4.92 (1H, doubled doublet of doublets, J = 1.4, 10.0 & 28.0 Hz);
5.94 (1H, doublet, J = 7.2 Hz);
5.91 (1H, doublet, J = 2.6 Hz).

### 27(ii) Octadecyl 5-acetamido-4-(N,N'-bis-t-butoxycarbonylguanidino)-8,9-O-isopropylidene-2,3,4,5,7-pentadeoxy-7-fluoro-D-glycero-D-galacto-non-2-enopyranosoate

40 mg (0.066 mmol) of octadecyl 5-acetamido-4-azido-8,9-O-isopropylidene-2,3,4,5,7-pentadeoxy-7-fluoro-D-glycero-D-galacto-non-2-enopyranosoate [prepared as described in step (i) above] were dissolved in 3 ml of methanol, and 10 mg of a Lindlar catalyst were added thereto. The mixture was then stirred under a hydrogen atmosphere for 2 hours. At the end of this time, the catalyst was separated by filtration, and the solvent was removed by distillation under reduced pressure. The resulting residue was dissolved in 2 ml of dimethylformamide, and then 25 mg (0.09 mmol) of N,N'-di-t-butoxycarbonylthiourea, 18 mg (0.18 mmol) of triethylamine and 25 mg (0.09 mmol) of mercuric chloride were added to the resulting solution. The mixture was then stirred at room temperature for 1 hour. At the end of this time, the solid produced was separated by filtration. The filtrate was poured into a 2-layer solution of 5 ml of ethyl acetate and 3 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 1 : 1 by volume mixture of hexane and ethyl acetate as the eluent, to obtain 39 mg (yield 71%) of the title compound as a colourless viscous substance.
Rf= 0.40 (20 : 1 = methylene chloride : methanol).
[α]_{D}²⁵ = -7.4° (c = 0.10, CHCl₃).
Mass Spectrum (FAB) m/e 827 (M⁺+H).
¹H-Nuclear Magnetic Resonance Spectrum (CDCl₃, 270 MHz) δ (ppm):
0.80 - 0.95 (3H, multiplet);
1.20 - 1.40 (32H, multiplet);
1.37 (3H, singlet);
1.41 (3H, singlet);
1.49 (9H, singlet);
1.50 (9H, singlet);
1.60 - 1.80 (2H, multiplet);
1.97 (3H, singlet);
4.10 - 4.30 (4H, multiplet);
4.40 - 4.65 (2H, multiplet);
5.20 (1H, doubled doublet of doublets, J = 2.4, 7.5 & 7.5 Hz);
5.83 (1H, doublet, J = 2.3 Hz);
5.65 (1H, doublet, J = 7.0 Hz);
8.60 (1H, doublet, J = 8.5 Hz).

### 27(iii) Octadecyl 5-acetamido-4-guanidino-2,3,4,5,7-pentadeoxy-7-fluoro-D-glycero-D-galacto-non-2-enopyranosoate trifluoroacetic acid salt

30 mg (0.04 mmol) of octadecyl 5-acetamido-4-(N,N'-bis-t-butoxycarbonylguanidino)-8,9-O-isopropylidene-2,3,4,5,7-pentadeoxy-7-fluoro-D glycero-D-galacto-non-2-enopyranosoate [prepared as described in step (ii) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 8 : 1 by volume mixture of isopropanol, ethyl acetate and water as the eluent, to obtain 20 mg (yield 79%) of the title compound as a colourless solid.
Rf= 0.55 (5 : 1 : 1 = t-butanol : acetic acid : water).
[α]D²⁵ = +14.0° (c = 0.10, CH₃OH).
Mass Spectrum (FAB) m/e 587 (M⁺+H).
¹H-Nuclear Magnetic Resonance Spectrum (D₂O, 270 MHz) δ (ppm):
0.70 - 0.85 (3H, multiplet);
1.10 - 1.30 (32H, multiplet);
1.60 - 1.80 (2H, multiplet);
2.00 (3H, singlet);
3.60 - 3.85 (2H, multiplet);
4.00 - 4.30 (3H, multiplet);
4.50 - 4.80 (2H, multiplet);
5.88 (1H, singlet).

### EXAMPLE 28

### 5-Acetamido-4-guanidino-2,3,4,5,7-pentadeoxy-7-methoxy-D-glycero-D-galacto-non-2-enopyranosoic acid trifluoroacetic acid salt (Compound No. 163-1)

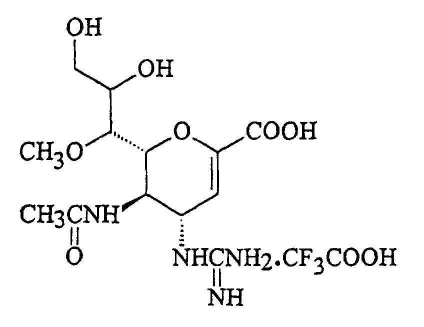

### 28(i) Benzyl N-acetyl-3,6-di-O-benzyl-4-O-methyl-α-D-glucosamine

4.48 g (9.12 mmol) of benzyl N-acetyl-3,6-di-O-benzyl-α-D-glucosamine [prepared according to the procedure described in Carbohydrate Res. 83, 163 - 169 (1980)] were dissolved in 50 ml of dimethylformamide, and 1.0 g (22.5 mmol) of sodium hydride was added to the resulting solution. The mixture was then stirred at room temperature for 30 minutes, after which 1.42 g (10.0 mmol) of methyl iodide were added at 0°C, and the mixture was stirred at room temperature for a further 5 hours. At the end of this time, the reaction mixture was poured into a 2-layer solution of 100 ml of ethyl acetate and 50 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 4 : 1 by volume mixture of methylene chloride and ethyl acetate as the eluent, to obtain 3.6 g (yield 78%) of the title compound as a colourless solid.
Rf= 0.55 (20 : 1 = methylene chloride : methanol).
[α]_{D}²⁵ = +126° (c = 1.15, CHCl₃).
Mass Spectrum (FAB) m/e 506 (M⁺+H).
¹H-Nuclear Magnetic Resonance Spectrum (CDCl₃, 270 MHz) δ (ppm):
1.80 (3H, singlet);
3.48 (3H, singlet);
3.40 - 3.80 (5H, multiplet);
4.23 (1H, doubled doublet of doublets, J = 3.5, 10.0 & 10.0 Hz);
4.40 - 4.90 (7H, multiplet);
5.25 (1H, doublet, J = 10.0 Hz);
7.20 - 7.40 (15H, multiplet).

### 28(ii) N-Acetyl-4-O-methyl-α-D-glucosamine

3.6 g (7.12 mmol) of benzyl N-acetyl-3,6-di-O-benzyl-4-O-methyl-α-D-glucosamine [prepared as described in step (i) above] were dissolved in 40 ml of acetic acid, and 5.0 g of palladium-on-carbon were added to the resulting solution. The mixture was then stirred under a hydrogen atmosphere at 3 atmospheres. for 30 hours. At the end of this time, the catalyst was separated by filtration, and the solvent was removed by distillation under reduced pressure. The resulting residue was purified by silica gel column chromatography, using a gradient elution method, with mixtures of methylene chloride and methanol ranging from 10 : 1 to 5 : 1 by volume as the eluent, to obtain 1.42 g (yield 83%) of the title compound as a colourless solid.
Rf= 0.35 (5 : 1 = methylene chloride : methanol).
[α]D²⁵ = +72.2° (c = 2.9, CH₃OH).
Mass Spectrum (FAB) m/e 236 (M⁺+H).
¹H-Nuclear Magnetic Resonance Spectrum (CD₃OD, 270 MHz) δ (ppm):
1.98 (3H, singlet);
3.57 (3H, singlet);
3.60 - 3.80 (6H, multiplet);
5.05 (1H, doublet, J = 3.2 Hz).

### 28(iii) 5-Acetamido-3,5-dideoxy-7-O-methyl-D-glycero-D-galacto-2-nonuropyranosoic acid

5.0 g (21.3 mmol) of N-acetyl-4-O-methyl-α-D-glucosamine [prepared as described in step (ii) above] were dissolved in distilled water, and 100 mg of sodium azide and 5.0 g (45.5 mmol) of sodium pyruvate were added to the resulting solution. The pH of the reaction mixture was then adjusted to a value of 10 to 11 by the addition of a 1 N aqueous solution of sodium hydroxide, after which 25 mg (660U) of N-acetyl-neuraminic acid aldolase (produced by TOYOBO K.K.) were added to the mixture, and the mixture was stirred at 20°C for 3 days. At the end of this time, the reaction mixture was desalted using a Dowex 50x8 (H⁺) resin (Dowex is a trade mark) and purified by column chromatography through Dowex 1 (HCOOH), eluted with a 1.0 M aqueous solution of formic acid, to obtain 1.8 g (yield 26%) of the title compound as a colourless viscous substance.
Rf= 0.30 (4 : 1 : 1 = isopropanol : acetic acid : water).
[α]_{D}²⁵ = -19.7° (c = 1.2, H₂O).
Mass Spectrum (FAB) m/e 324 (M⁺+H).
¹H-Nuclear Magnetic Resonance Spectrum (D₂O, 270 MHz) δ (ppm):
1.80 (1H, doublet of doublets, J = 12.0 & 12.0 Hz);
2.00 (3H, singlet);
2.10 (1H, doublet of doublets, J = 4.5 & 12.0 Hz);
3.48 (3H, singlet);
3.50 - 3.80 (7H, multiplet).

### 28(iv) Methyl 5-acetamido-3,5-dideoxy-7-O-methyl-D-glycero-D-galacto-2-nonuropyranosoate

680 mg (2.10 mmol) of 5-acetamido-3,5-dideoxy-7-O-methyl-D-glycero-D-galacto-2-nonuropyranosoic acid [prepared as described in step (iii) above] were dissolved in 20 ml of methanol, and 500 mg of a Dowex 50x8 (H⁺) cation exchange resin (Dowex is a trade mark) were added to the resulting solution. The mixture was then stirred at room temperature for 15 hours. At the end of this time, the cation exchange resin was separated by filtration, and the solvent was removed by distillation under reduced pressure. The resulting residue was purified by silica gel column chromatography, using a 5 : 1 by volume mixture of methylene chloride and methanol as the eluent, to obtain 340 mg (yield 48%) of the title compound as a colourless amorphous substance.
Rf= 0.15 (5 : 1 = methylene chloride : methanol).
[α]_{D}²⁵ = +30.2° (c = 1.1, CH₃OH).
Mass Spectrum (FAB) m/e 338 (M⁺+H).
¹H-Nuclear Magnetic Resonance Spectrum (CD₃OD, 270 MHz) δ (ppm):
1.90 (1H, doublet of doublets, J = 12.0 & 14.0 Hz);
2.00 (3H, singlet);
2.16 (1H, doublet of doublets, J = 4.5 & 12.0 Hz);
3.40 (1H, multiplet);
3.45 (3H, singlet);
3.50 - 3.80 (3H, multiplet);
3.77 (3H, singlet);
3.80 - 4.00 (2H, multiplet);
4.20 (1H, doublet of doublets, J = 1.5 & 9.5 Hz).

### 28(v) Methyl 5-acetamido-4,8,9-tri-O-acetyl-2,3,5-trideoxy-7-O-methyl-D-glycero-D-galacto-non-2-enopyranosoate

686 mg (2.03 mmol) of methyl 5-acetamido-3,5-dideoxy-7-O-methyl-D-glycero-D-galacto-2-nonuropyranosoate [prepared as described in step (iv) above] were dissolved in a mixture of 10 ml of pyridine and 10 ml of acetic anhydride, and the mixture was stirred at room temperature for 15 hours. At the end of this time, the solvent was removed by distillation under reduced pressure, and the residue was dissolved in 20 ml of a 4 M solution of hydrogen chloride in dioxane, after which it was stirred at room temperature for 15 hours. At the end of this time, the solution was dissolved in benzene, 182 mg (1.20 mmol) of 1,8-diazabicyclo[5.4.0]-7-undecene were added to the resulting solution, and the mixture was stirred at room temperature for 2 hours. The reaction mixture was then poured into a 2-layer solution of 30 ml of methylene chloride and 15 ml of a saturated aqueous ammonium chloride solution, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 310 mg (yield 34%) of the title compound as a colourless amorphous substance.
Rf= 0.30 (20 : 1 = methylene chloride : methanol).
[α]_{D}²⁵ = +206° (c = 0.15, CHCl₃).
Mass Spectrum (FAB) m/e 446 (M⁺+H).
¹H-Nuclear Magnetic Resonance Spectrum (CDCl₃, 270 MHz) δ (ppm):
2.00 (3H, singlet);
2.06 (3H, singlet);
2.08 (3H, singlet);
2.10 (3H, singlet);
3.50 (3H, singlet);
3.65 (1H, doublet of doublets, J = 3.0 & 3.0 Hz);
3.80 (3H, singlet);
4.20 - 4.35 (2H, multiplet);
4.45 (1H, multiplet);
4.70 (1H, doublet of doublets, J = 3.0 & 12.0 Hz);
5.35 (1H, multiplet);
5.50 (1H, doublet of doublets, J = 3.0 & 7.0 Hz);
5.55 (1H, doublet, J = 8.0 Hz);
6.00 (1H, doublet, J = 2.8 Hz).

### 28(vi) Methyl 5-acetamido-4-azido-8,9-di-O-acetyl-2,3,4,5,7-pentadeoxy-7-methoxy-D-glycero-D-galacto-non-2-enopyranosoate

303 mg (0.67 mmol) of methyl 5-acetamido-4,8,9-tri-O-acetyl-2,3,5-trideoxy-7-O-methyl-D-glycero-D-galacto-non-2-enopyranosoate [prepared as described in step (v) above] were dissolved in 10 ml of anhydrous methylene chloride, and 22 mg (0.67 mmol) of methanol were added to the resulting solution. 956 mg (6.74 mmol) of a boron trifluoride-diethyl ether complex were added to the resulting mixture under a nitrogen atmosphere, and the mixture was stirred at room temperature for 24 hours. At the end of this time, the reaction mixture was poured into a mixture of 20 ml of water, 10 g of ice, 5 g of solid sodium hydrogencarbonate and 20 ml of ethyl acetate, and the mixture was vigorously stirred for 10 minutes. The organic layer was then separated, washed with 10 ml of a saturated aqueous solution of sodium chloride, and dried over anhydrous sodium sulphate, after which the solvent was removed by distillation under reduced pressure. The resulting residue (270 mg) was dissolved in 10 ml of dimethylformamide, and 270 mg of a cation exchange resin [Dowex 50x8 (H⁺)-Dowex is a trade mark] and 90 mg (1.37 mmol) of sodium azide were added to the resulting solution. The mixture was then stirred at 90°C for 4 hours. At the end of this time, the Dowex 50x8 (H⁺) resin was separated by filtration, and the solvent was removed by distillation under reduced pressure. The resulting residue was dissolved in a 2-layer solution of 20 ml of ethyl acetate and 10 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 250 mg (yield 70%) of the title compound as a colourless viscous substance.
Rf= 0.30 (20 : 1 = methylene chloride : methanol).
[α]D²⁵ = +97.2° (c = 0.25, CHCl₃).
Mass Spectrum (FAB) m/e 429 (M⁺+H).
¹H-Nuclear Magnetic Resonance Spectrum (CDCl₃, 270 MHz) δ (ppm):
2.06 (6H, singlet);
2.10 (3H, singlet);
3.50 (3H, singlet);
3.65 (1H, doublet of doublets, J = 1.8 & 4.0 Hz);
3.80 (3H, singlet);
4.25 (1H, doublet of doublets, J = 5.0 & 12.0 Hz);
4.45 - 4.55 (2H, multiplet);
4.75 (1H, doublet of doublets, J = 3.2 & 12.0 Hz);
5.35 (1H, multiplet);
5.52 (1H, doublet, J = 8.2 Hz);
5.98 (1H, doublet, J = 2.7 Hz).

### 28(vii) Methyl 5-acetamido-4-(N,N'-bis-t-butoxycarbonylguanidino)-8,9-di-O-acetyl-2,3,4,5,7-pentadeoxy-7-methoxy-D-glycero-D-galacto-non-2-enopyranosoate

80 mg (0.42 mmol) of methyl 5 acetamido-4-azido-8,9-di-O-acetyl-2,3,4,5,7-pentadeoxy-7-methoxy-D-glycero-D-galacto-non-2-enopyranosoate [prepared as described in step (vi) above] were dissolved in 10 ml of methanol, and 130 mg of a Lindlar catalyst were added to the resulting solution. The mixture was then stirred under a hydrogen atmosphere for 2 hours. At the end of this time, the catalyst was separated by filtration, and the solvent was removed by distillation under reduced pressure. The resulting residue (120 mg) was dissolved in 10 ml of dimethylformamide, and 100 mg (0.37 mmol) of N,N'-di-t-butoxycarbonylthiourea, 75 mg (0.74 mmol) of triethylamine and 100 mg (0.37 mmol) of mercuric chloride were added to the resulting solution. The mixture was then stirred at room temperature for 1 hour. At the end of this time, the solid was separated by filtration, and the filtrate was poured into a 2-layer solution of 10 ml of ethyl acetate and 50 ml of a saturated aqueous solution of sodium hydrogencarbonate. The organic layer was separated, washed with a saturated aqueous solution of sodium chloride and dried over anhydrous sodium sulphate, after which the solvent was removed by distillation under reduced pressure. The resulting residue was purified by silica gel column chromatography, using a 2 : 1 by volume mixture of hexane and ethyl acetate as the eluent, to obtain 180 mg (yield 64 %) of the title compound as a colourless amorphous substance.
Rf= 0.50 (20 : 1 = methylene chloride : methanol).
[α]_{D}²⁵=+2.8° (c = 0.65, CHCl₃).
Mass Spectrum (FAB) m/e 645 (M⁺+H).
1H-Nuclear Magnetic Resonance Spectrum (CDCl₃, 270 MHz) δ (ppm):
1.49 (9H, singlet);
1.50 (9H, singlet);
1.97 (3H, singlet);
2.05 (3H, singlet);
2.07 (3H, singlet);
3.52 (3H, singlet);
3.55 (1H, multiplet);
3.78 (3H, singlet);
4.10 (1H, doublet of doublets, J = 1.0 & 10.5 Hz);
4.25 - 4.40 (2H, multiplet);
4.82 (1H, doublet of doublets, J = 3.2 & 12.0 Hz);
5.12 (1H, doubleted doublet of doublets, J = 2.5 & 11.0 & 11.0 Hz);
5.30 (1H, multiplet);
5.85 (1H, doublet, J = 2.3 Hz);
6.35 (1H, doublet, J = 8.8 Hz).

### 28(viii) 5-Acetamido-4-guanidino-2,3,4,5,7-pentadeoxy-7-methoxy-D-glycero-D-galacto-non-2-enopyranosoic acid trifluoroacetic acid salt

53 mg (0.083 mmol;) of methyl 5 acetamido-4-(N,N'-bis-t-butoxycarbonylguanidino)-8,9-di-O-acetyl-2,3,4,5,7-pentadeoxy-7-methoxy-D-glycero-D-galacto-non-2-enopyranosoate [prepared as described in step (vii) above] were dissolved in 2 ml of methanol, and 0.2 ml of a 0.1 N methanolic solution of sodium methoxide was added to the resulting solution. The mixture was then stirred at room temperature for 1 hour. At the end of this time, the reaction mixture was neutralized with a 4 M solution of hydrogen chloride in dioxane, and the solvent was removed by distillation under reduced pressure. The resulting residue was dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was dissolved in 1 ml of distilled water. 72 µl of a 1N aqueous solution of sodium hydroxide were then added to the reaction mixture, which was then stirred at room temperature for 1 hour. At the end of this time, the reaction mixture was neutralized with a Dowex 50x8 (H⁺) resin (Dowex is a trade mark), and then the water was removed by distillation. The resulting residue was purified by silica gel column chromatography, using a 5 : 1 : 1: by volume mixture of isopropanol, ethyl acetate and water as the eluent, to obtain 24 mg (yield 85%) of the title compound as a colourless solid.
Rf= 0.30 (4 : 1 : 1 = isopropanol: acetic acid : water).
[α]_{D}²⁵=-9.3 °(c = 0.1, CH₃OH).
Mass Spectrum (FAB) m/e 347 (M⁺+H).
1H-Nuclear Magnetic Resonance Spectrum (CDCl₃, 270 MHz) δ (ppm):
2.00 (3H, singlet);
3.37 (3H, singlet);
3.55 (1H, doublet, J = 8.5 Hz);
3.65 (1H, doublet of doublets, J = 5.0 & 12 Hz);
3.90 (3H, multiplet);
4.20 (1H, doublet of doublets, J = 10 & 10 Hz);
4.40 - 4.50(2H, multiplet);
5.85 (1H, doublet, J = 1.8 Hz).

### EXAMPLE 29

### 5-Acetamido-4-guanidino-9-O-tetradecanoyl-2,3,4,5,7-pentadeoxy-7-methoxy-D-glycero-D-galacto-non-2-enopyranosoic acid trifluoroacetic acid salt (Compound No. 163-41)

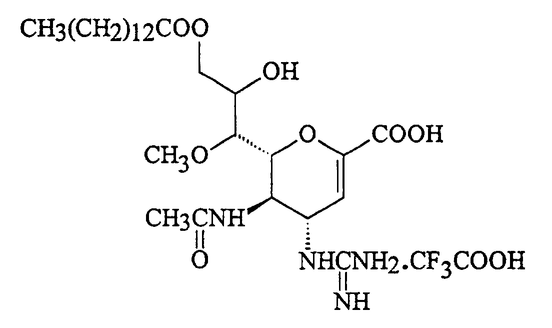

### 29(i) Diphenylmethyl 5-acetamido-4-(N,N'-bis-t-butoxycarbonylguanidino)-2,3,4,5,7-pentadeoxy-7-methoxy-D-glycero-D-galacto-non-2-enopyranosoate

87 mg (0.14 mmol) of methyl 5 acetamido-4-(N,N'-bis-t-butoxycarbonylguanidino)-8,9-di-O-acetyl-2,3,4,5,7-pentadeoxy-7-methoxy-D-glycero-D-galacto-non-2-enopyranosoate [prepared as described in Example 28(vii)] were dissolved in 5 ml of methanol, and 2 ml of a 0.4 N methanolic solution of sodium methoxide were added to the resulting solution. The mixture was then stirred at room temperature for 1 hour. At the end of this time, the reaction mixture was neutralized with a 4 M solution of hydrogen chloride in dioxane, and the solvent was removed by distillation under reduced pressure. The resulting residue was dissolved in 4 ml of distilled water and 0.2 ml of a 1 N aqueous solution of sodium hydroxide was added to the resulting solution. The mixture was then stirred at room temperature for 1 hour. At the end of this time, the reaction mixture was neutralized with a 4 M solution of hydrogen chloride in dioxane, and then the water was removed by distillation, after which the residue was dissolved in a 1 : 1 by volume mixture of methanol and methylene chloride, and 47 mg (0.24 mmol) of diphenyldiazomethane and 50 mg (0.38 mmol) of a boron trifluoride-diethyl ether complex were added to the resulting solution. The mixture was then stirred at room temperature for 2 hours. At the end of this time, acetic acid was added, and the solvent was removed by distillation under reduced pressure. The resulting residue was purified by silica gel column chromatography, using a 20 : 1 by volume mixture of methylene chloride and methanol as the eluent, to obtain 29 mg (yield 30%) of the title compound as a colourless amorphous substance.
Rf= 0.3 (20 : 1 = methylene chloride : methanol).
[α]D²⁵ = +22.0° (c = 0.4, CHCl₃).
Mass Spectrum (FAB) m/e 713 (M⁺+H).
¹H-Nuclear Magnetic Resonance Spectrum (CD₃OD, 270 MHz) δ (ppm):
1.46 (9H, singlet);
1.52 (9H, singlet);
1.95 (3H, singlet);
3.48 (1H, doublet, J = 9.0 Hz);
3.68 (1H, doublet of doublets, J = 4.0 & 12 Hz);
3.85 (1H, doublet of doublets, J = 2.5 & 12 Hz);
3.95 (1H, multiplet);
4.32 (1H, doublet of doublets, J = 10 & 10 Hz);
4.43 (1H, doublet, J = 10 Hz);
5.05 (1H, doublet of doublets, J = 2.2 & 10 Hz);
6.02 (1H, doublet, J = 2.2 Hz);
6.90 (1H, singlet);
7.20 - 7.50 (10H, multiplet).

### 29(ii) Diphenylmethyl 5-acetamido-4-(N,N'-bis-t-butoxycarbonylguanidino)-9-O-tetradecanoyl-2,3,4,5,7-pentadeoxy-7-methoxy-D-glycero-D-galacto-non-2-enopyranosoate

28 mg (0.039 mmol) of diphenylmethyl 5-acetamido-4-(N,N'-bis-t-butoxycarbonylguanidino)-2,3,4,5,7-pentadeoxy-7-methoxy-D-glycero-D-galacto-non-2-enopyranosoate [prepared as described in step (i) above] were dissolved in 2 ml of methylene chloride, and 6 mg (0.06 mmol) of triethylamine and 8 mg (0.05 mmol) of tetradecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 22 mg (yield 61%) of the title compound as a colourless amorphous substance.
Rf= 0.45 (20 : 1 = methylene chloride : methanol).
[α]_{D}²⁵ = +40.5° (c = 0.2, CHCl₃).
Mass Spectrum (FAB) m/e 923 (M⁺+H).
¹H-Nuclear Magnetic Resonance Spectrum (CDCl₃, 270 MHz) δ (ppm):
0.85 (3H, multiplet);
1.20 - 1.30 (20H, multiplet);
1.48 (9H, singlet);
1.50 (9H, singlet);
1.55 - 1.70 (2H, multiplet);
1.98 (3H, singlet);
2.33 (2H, triplet, J = 7.5 Hz);
3.40 (1H, multiplet);
3.48 (3H, singlet);
4.10 - 4.50 (5H, multiplet);
5.18 (1H, doubled doublet of doublets, J = 2.4, 9.0 & 9.0 Hz);
5.95 (1H, doublet, J = 2.3 Hz);
6.18 (1H, doublet, J= 8.7 Hz);
6.92 (1H, singlet);
7.20 - 7.50 (10H, multiplet);
8.55 (1H, doublet, J = 8.7 Hz).

### 29(iii) 5-Acetamido-4-guanidino-9-O-tetradecanoyl-2,3,4,5,7-pentadeoxy-7-methoxy-D-glycero-D-galacto-non-2-enopyranosoic acid trifluoroacetic acid salt

22 mg (0.024 mmol) of diphenylmethyl 5-acetamido-4-(N,N'-bis-t-butoxycarbonylguanidino)-9-O-tetradecanoyl-2,3,4,5,7-pentadeoxy-7-methoxy-D-glycero-D-galacto-non-2-enopyranosoate [prepared as described in step (ii) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 10 mg (yield 74%) of the title compound as a colourless solid.
Rf= 0.4 (2 : 5 : 1 = isopropanol : ethyl acetate : water).
[α]_{D}²⁵ = +24.0° (c = 0.1, CH₃OH).
Mass Spectrum (FAB) m/e 557 (M⁺+H).
¹H-Nuclear Magnetic Resonance Spectrum (CD₃OD, 270 MHz) δ (ppm):
0.90 (3H, multiplet);
1.25 - 1.40 (20H, multiplet);
1.60 - 1.70 (2H, multiplet);
2.00 (3H, singlet);
2.37 (2H, triplet, J = 7.5 Hz);
3.38 (3H, singlet);
3.45 (1H, multiplet);
4.10 - 4.40 (6H, multiplet);
5.53 (1H, doublet, J = 1.8 Hz).

### EXAMPLE 30

### 5-Acetamido-4-guanidino-2,3,4,5,7-pentadeoxy-7-ethoxy-D-glycero-D-galacto-non-2-enoic acid trifluoroacetic acid salt (Compound No. 199-1)

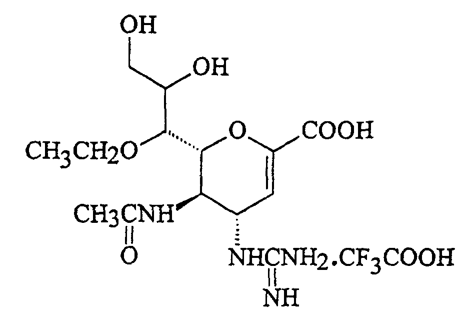

### 30(i) Benzyl N-acetyl-3,6-di-O-benzyl-4-O-ethyl-α-D-glucosamine

8.35 g (16.8 mmol) of benzyl N-acetyl-3,6-di-O-benzyl-α-D-glucosamine [prepared according to the procedure described in Carbohydrate Res. 83, 163 - 169 (1980)] were dissolved in 80 ml of dimethylformamide, and 1.84 g (42.2 mmol) sodium hydride were added to the resulting solution. The mixture was then stirred at room temperature for 30 minutes, after which 2.86 g (18.5 mmol) of ethyl iodide were added to the mixture at 0°C, and the mixture was stirred at room temperature for 5 hours. At the end of this time, the reaction mixture was poured into a 2-layer solution of 200 ml of ethyl acetate and 100 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 4 : 1 by volume mixture of methylene chloride and ethyl acetate as the eluent, to obtain 7.0 g (yield 80%) of the title compound as a colourless solid.
Rf= 0.55 (20 : 1 = methylene chloride : methanol).
[α]_{D}²⁵ = +123° (c = 2.3, CHCl₃).
Mass Spectrum (FAB) m/e 520 (M⁺+H).
¹H-Nuclear Magnetic Resonance Spectrum (CDCl₃, 270 MHz) δ (ppm):
1.10 (3H, triplet, J = 7.0 Hz);
1.80 (3H, singlet);
3.40 - 3.90 (7H, multiplet);
4.21 (1H, doubled doublet of doublets, J = 3.5, 10.0 & 10.0 Hz);
4.40 - 4.90 (7H, multiplet);
5.22 (1H, doublet, J = 10.0 Hz);
7.20 - 7.40 (15H, multiplet).

### 30(ii) N-Acetyl-4-O-ethyl-α-D-glucosamine

7.0 g (13.4 mmol) of benzyl N-acetyl-3,6-di-O-benzyl-4-O-ethyl-α-D-glucosamine [prepared as described in step (i) above] were dissolved in 60 ml of acetic acid, and 10 g of palladium-on-carbon were added to the resulting solution. The mixture was then stirred under hydrogen atmosphere at 3 atmospheres pressure for 30 hours. At the end of this time, the catalyst was separated by filtration, and the solvent was removed by distillation under reduced pressure. The resulting residue was purified by silica gel column chromatography, using a gradient elution method, with mixtures of methylene chloride and methanol ranging from 10 : 1 to 5 : 1 by volume as the eluent, to obtain 2.4 g (yield 81%) of the title compound as a colourless solid.
Rf= 0.35 (5 : 1 = methylene chloride : methanol).
[α]_{D}²⁵ = +66.1° (c = 2.1, CH₃OH).
Mass Spectrum (FAB) m/e 250 (M⁺+H).
¹H-Nuclear Magnetic Resonance Spectrum (D₂O, 270 MHz) δ (ppm):
1.15 (3H, triplet, J = 7.0 Hz);
2.00 (3H, singlet);
3.33 (2H, quartet, J = 7.0 Hz);
3.60 - 3.90 (6H, multiplet);
5.15 (1H, singlet).

### 30(iii) 5-Acetamido-3,5-dideoxy-7-O-ethyl-D-glycero-D-galacto-2-nonuropyranosoic acid

5.0 g (20.1 mmol) of N-acetyl-4-O-ethyl-α-D-glucosamine [prepared as described in step (iii) above] were dissolved in distilled water, and 100 mg of sodium azide and 5.0 g (45.5 mmol) of sodium pyruvate were added to the resulting solution. The pH of the reaction mixture was adjusted to a value of 10 to 11 using a 1 N aqueous solution of sodium hydroxide, after which 25 mg (660U) of N-acetyl-neuraminic acid aldolase (produced by TOYOBO K.K.) were added, and the mixture was stirred at 20°C for 3 days. At the end of this time, the reaction mixture was desalted using a Dowex 50x8 (H⁺) resin (Dowex is a trade mark) and purified by chromatography using a Dowex 1 (HCOOH) resin with a 1.0 M aqueous solution of formic acid as the eluent, to obtain 500 mg (yield 7.4%) of the title compound as a colourless viscous substance.
Rf= 0.20 (4 : 1 : 1 = isopropanol: acetic acid : water).
[α]_{D}²⁵ = -8.4° (c = 0.19, CH₃OH).
Mass Spectrum (FAB) m/e 338 (M⁺+H).
¹H-Nuclear Magnetic Resonance Spectrum (D₂O, 270 MHz) δ (ppm):
1.05 (3H, triplet, J = 7.0 Hz);
1.80 (1H, doublet of doublets, J = 12.0 & 12.0 Hz);
2.00 (3H, singlet);
2.20 (1H, doublet of doublets, J = 4.5 & 12.0 Hz);
3.50 - 3.80 (7H, multiplet).

### 30(iv) Methyl 5-acetamido-3,5-dideoxy-7-O-ethyl-D-glycero-D-galacto-2-nonuropyranosoate

1.9 g (5.64 mmol) of 5-acetamido-3,5-dideoxy-7-O-ethyl-D-glycero-D-galacto-2-nonuropyranosoic acid [prepared as described in step (iii) above] were dissolved in 60 ml of methanol, and 600 mg of a cation exchange resin [Dowex 50x8 (H⁺) - Dowex is a trade mark] were added to the resulting solution. The mixture was then stirred at room temperature for 15 hours. At the end of this time, the Dowex 50x8 (H⁺) was separated by filtration, and the solvent was removed by distillation under reduced pressure. The resulting residue was purified by silica gel column chromatography, using a 5 : 1 by volume mixture of methylene chloride and methanol as the eluent, to obtain 1.4 g (yield 71%) of the title compound as an amorphous substance.
Rf= 0.3 (5 : 1 = methylene chloride : methanol).
[α]_{D}²⁵ = -7.5° (c = 0.10, CHCl₃).
Mass Spectrum (FAB) m/e 352 (M⁺+H).
¹H-Nuclear Magnetic Resonance Spectrum (CD₃OD, 270 MHz) δ (ppm):
1.17 (3H, triplet, J = 7.0 Hz);
1.95 (1H, doublet of doublets, J = 12.0 & 14.0 Hz);
2.00 (3H, singlet);
2.16 (1H, doublet of doublets, J = 4.5 & 12.0 Hz);
3.42 (1H, doublet of doublets, J = 1.5 & 8.0 Hz);
3.60 (2H, quartet, J = 7.0 Hz);
3.77 (3H, singlet);
3.70 - 4.00 (4H, multiplet);
4.15 (1H, doublet of doublets, J = 1.5 & 9.5 Hz).

### 30(v) Methyl 5-acetamido-4,8,9,-tri-O-acetyl-2,3,5,7-tetradeoxy-7-ethoxy-D-glycero-D-galacto-non-2-enopyranosoate

1.4 g (3.99 mmol) of methyl 5-acetamido-3,5-dideoxy-7-O-ethyl-D-glycero-D-galacto-2-nonuropyranosoate [prepared as described in step (iv) above] were dissolved in a mixture of 20 ml of pyridine and 10 ml of acetic anhydride, and the mixture was stirred at room temperature for 15 hours. At the end of this time, the solvent was removed by distillation under reduced pressure, and the residue was dissolved in 50 ml of a 4 M solution of hydrogen chloride in dioxane. The mixture was then stirred at room temperature for 15 hours. At the end of this time, the solvent was removed by distillation under reduced pressure, and the residue was dissolved in benzene. 952 mg (6.30 mmol) of 1,8-diazabicyclo[5.4.0]-7-undecene were then added to the reaction mixture, and the mixture was stirred at room temperature for 2 hours. At the end of this time, the reaction mixture was poured into a 2-layer solution of 30 ml of methylene chloride and 15 ml of a saturated aqueous ammonium chloride solution, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 1.1 g (yield 60%) of the title compound as a colourless amorphous substance.
Rf= 0.30 (20 : 1 = methylene chloride : methanol).
[α]D²⁵ = +24.1° (c = 0.5, CHCl₃).
Mass Spectrum (FAB) m/e 460 (M⁺+H).
¹H-Nuclear Magnetic Resonance Spectrum (CDCl₃, 270 MHz) δ (ppm):
1.20 (3H, triplet, J = 7.0 Hz);
1.98 (3H, singlet);
2.05 (3H, singlet);
2.08 (3H, singlet);
3.50 - 3.80 (3H, multiplet);
3.80 (3H, singlet);
4.20 - 4.35 (2H, multiplet);
4.45 (1H, multiplet);
4.70 (1H, doublet of doublets, J = 3.2 & 12.0 Hz);
5.33 (1H, multiplet);
5.47 (1H, doublet of doublets, J = 3.3 & 7.0 Hz);
5.51 (1H, doublet, J = 7.0 Hz);
6.00 (1H, doublet, J = 3.3 Hz).

### 30(vi) Methyl 5-acetamido-4-azido-8,9-di-O-acetyl-2,3,4,5,7-pentadeoxy-7-ethoxy-D-glycero-D-galacto-non-2-enopyranosoate

1000 mg (2.18 mmol) of methyl 5-acetamido-4,8,9,-tri-O-acetyl-2,3,5,7-tetradeoxy-7-ethoxy-D-glycero-D-galacto-non-2-enopyranosoate [prepared as described in step (v) above] were dissolved in 20 ml of anhydrous methylene chloride, and 25 mg (0.78 mmol) of methanol were added to the resulting solution, after which 3.1 g (21.8 mmol) of a boron trifluoride-diethyl ether complex were added under a nitrogen atmosphere, and the mixture was stirred at room temperature for 24 hours. At the end of this time, the reaction mixture was poured into a mixture of 50 ml of water, 10 g of ice, 10 g of solid sodium hydrogencarbonate and 50 ml of ethyl acetate, and the mixture was vigorously stirred for 10 minutes. The organic layer was then washed with 10 ml of a saturated aqueous solution of sodium chloride and dried over anhydrous sodium sulphate, after which the solvent was removed by distillation under reduced pressure. The resulting residue (800 mg) was dissolved in 10 ml of dimethylformamide, and 800 mg of a cation exchange resin [Dowex 50x8 (H⁺)-Dowex is a trade mark] and 400 mg (6.15 mmol) of sodium azide were added. The mixture was then stirred at 90°C for 4 hours. At the end of this time, the Dowex 50x8 (H⁺) was separated by filtration, and the solvent was removed by distillation under reduced pressure. The resulting residue was dissolved in a 2-layer solution of 30 ml of ethyl acetate and 20 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 600 mg (yield 63%) of the title compound as a colourless viscous substance.
Rf= 0.30 (20 : 1 = methylene chloride : methanol).
[α]_{D}²⁵ = +72.6° (c = 0.05, CHCl₃).
Mass Spectrum (FAB) m/e 443 (M⁺+H).
¹H-Nuclear Magnetic Resonance Spectrum (CDCl₃, 270 MHz) δ (ppm):
1.25 (3H, triplet, J = 7.0 Hz);
2.09 (3H, singlet);
2.10 (3H, singlet);
2.13 (3H, singlet);
3.50 - 3.80 (3H, multiplet);
3.80 (3H, singlet);
4.05 (1H, multiplet);
4.25 (1H, doublet of doublets, J = 5.0 & 12.0 Hz);
4.45 - 4.55 (2H, multiplet);
4.80 (1H, doublet of doublets, J = 3.2 & 12.0 Hz);
5.35 (1H, multiplet);
5.51 (1H, doublet, J = 8.2 Hz);
6.00 (1H, doublet, J = 2.7 Hz).

### 30(vii) Methyl 5-acetamido-4-(N,N'-bis-t-butoxycarbonylguanidino)-8,9-di-O-acetyl-2,3,4,5,7-pentadeoxy-7-ethoxy-D-glycero-D-galacto-non-2-enopyranosoate

580 mg (1.31 mmol) of methyl 5-acetamido-4-azido-8,9-di-O-acetyl-2,3,4,5,7-pentadeoxy-7-ethoxy-D-glycero-D-galacto-non-2-enopyranosoate [prepared as described in step (vi) above] were dissolved in 10 ml of methanol, and 270 mg of a Lindlar catalyst were added to the resulting solution. The mixture was then stirred under a hydrogen atmosphere for 2 hours. At the end of this time, the catalyst was separated by filtration, and the solvent was removed by distillation under reduced pressure. The resulting residue (400 mg) was dissolved in 10 ml of dimethylformamide, and 408 mg (1.48 mmol) of N,N-di-t-butoxycarbonylthiourea, 300 mg(2.95 mmol) of triethylamine and 402 mg (1.48 mmol) of mercuric chloride were added to the resulting solution. The mixture was then stirred at room temperature for 1 hour. At the end of this time, the solid was separated by filtration and the filtrate was poured into a 2-layer solution of 20 ml of ethyl acetate and 10 ml of a saturated aqueous solution of sodium hydrogencarbonate. The organic layer was separated, washed with a saturated aqueous solution of sodium chloride and dried over anhydrous sodium sulphate, after which the solvent was removed by distillation under reduced pressure. The resulting residue was purified by silica gel column chromatography, using a 2 : 1 by volume mixture of hexane and ethyl acetate as the eluent, to obtain 530 mg (yield 61%) of the title compound as a colourless amorphous substance.
Rf= 0.40 (20 : 1 = methylene chloride : methanol).
[α]_{D}²⁵ = +2.8° (c = 0.05, CHCl₃).
Mass Spectrum (FAB) m/e 659 (M⁺+H).
¹H-Nuclear Magnetic Resonance Spectrum (CDCl₃, 270 MHz) δ (ppm):
1.20 (3H, triplet, J = 7.0 Hz);
1.49 (9H, singlet);
1.50 (9H, singlet);
1.95 (3H, singlet);
2.06 (3H, singlet);
2.09 (3H, singlet);
3.50 - 3.80 (3H, multiplet);
3.80 (3H, singlet);
4.10 (1H, doublet of doublets, J = 1.0 & 10.5 Hz);
4.25 - 4.40 (2H, multiplet);
4.80 (1H, doublet of doublets, J = 3.2 & 12.0 Hz);
5.10 (1H, doubled doublet of doublets, J = 2.5, 11.0 & 11.0 Hz);
5.30 (1H, multiplet);
5.83 (1H, daublet, J = 2.3 Hz);
6.20 (1H, doublet, J = 8.8 Hz).

### 30(viii) 5-Acetamido-4-guanidino-2,3,4,5,7-pentadeoxy-7-ethoxy-D-glycero-D-galacto-non-2-enopyranosoic acid trifluoroacetic acid salt

50 mg (0.076 mmol) of methyl 5-acetamido-4-(N,N,'-bis-t-butoxycarbonylguanidino)-8,9-di-O-acetyl-2,3,4,5,7-pentadeoxy-7-ethoxy-D-glycero-D-galacto-non-2-enopyranosoate [prepared as described in step (vii) above] were dissolved in 2 ml of methanol, and 0.2 ml of a 0.1 N methanolic solution of sodium methoxide was added to the resulting solution. The mixture was then stirred at room temperature for 1 hour. At the end of this time, the reaction mixture was neutralized with a 4 M solution of hydrogen chloride in dioxane, and the solvent was removed by distillation under reduced pressure. The resulting residue was dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was dissolved in 1 ml of distilled water. 110 µl of a 1 N aqueous solution of sodium hydroxide were then added to the reaction mixture, which was then stirred at room temperature for 1 hour. At the end of this time, the reaction mixture was neutralized with a Dowex 50x8 (H⁺) resin (Dowex is a trade mark), and then the water was removed by distillation. The resulting residue was purified by silica gel column chromatography, using a 5 : 1 : 1 by volume mixture of isopropanol, ethyl acetate and water as the eluent, to obtain 27 mg (yield 75%) of the title compound as a colourless solid.
Rf= 0.30 (4 : 1 : 1 = isopropanol : acetic acid : water).
[α]_{D}²⁵ = +32.5° (c = 0.1, CH₃OH).
Mass Spectrum (FAB) m/e 361 (M⁺+H).
¹H-Nuclear Magnetic Resonance Spectrum (D₂O, 270 MHz) δ (ppm):
1.05 (3H, triplet, J = 7.0 Hz);
1.97 (3H, singlet);
3.40 (1H, multiplet);
3.50 - 3.65 (3H, multiplet);
3.80 (1H, doublet of doublets, J = 5.0 & 12 Hz);
3.90 (1H, multiplet);
4.15 (1H, doublet of doublets, J = 10 & 10 Hz);
4.40 - 4.50 (2H, multiplet);
5.55 (1H, doublet, J = 1.8 Hz).

### EXAMPLE 31

### 5-Acetamido-4-guanidino-9-O-octanoyl-2,3,4,5,7-pentadeoxy-7-ethoxy-D-glycero-D-galacto-non-2-enopyranosoic acid trifluoroacetic acid salt (Compound No. 199-38)

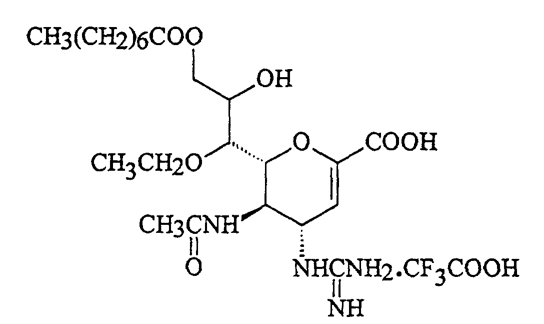

### 31(i) Diphenylmethyl 5-acetamido-4-(N,N'-bis-t-butoxycarbonylguanidino)-2,3,4,5,7-pentadeoxy-7-ethoxy-D-glycero-D-galacto-non-2-enopyranosoate

495 mg (0.75 mmol) of methyl 5-acetamido-4-(N,N'-bis-t-butoxycarbonylguanidino)-8,9-di-O-acetyl-2,3,4,5,7-pentadeoxy-7-ethoxy-D-glycero-D-galacto-non-2-enopyranosoate [prepared as described in Example 30(vii)] were dissolved in 10 ml of methanol, and 2 ml of a 0.1 N methanolic solution of sodium methoxide were added to the resulting solution. The mixture was then stirred at room temperature for 1 hour. At the end of this time, the reaction mixture was neutralized with a 4 M solution of hydrogen chloride in dioxane, and the solvent was removed by distillation under reduced pressure. The resulting residue was dissolved in 10 ml of distilled water, and 1.0 ml of a 1 N aqueous solution of sodium hydroxide was added thereto. The mixture was then stirred at room temperature for 1 hour. At the end of this time, the reaction mixture was neutralized with a 4 M solution of hydrogen chloride in dioxane, and then the water was removed by distillation. The resulting residue was dissolved in a 6 : 1 by volume mixture of methanol and methylene chloride, and 500 mg (2.60 mmol) of diphenyldiazomethane and 50 mg (0.38 mmol) of a boron trifluoride-diethyl ether complex were added to the resulting solution. The mixture was then stirred at room temperature for 2 hours, after which acetic acid was added thereto, and the solvent was removed by distillation under reduced pressure. The resulting residue was purified by silica gel column chromatography, using a 20 : 1 by volume mixture of methylene chloride and methanol as the eluent, to obtain 450 mg (yield 82%) of the title compound as a colourless amorphous substance.
Rf= 0.25 (10 : 1 = methylene chloride : methanol).
[α]_{D}²⁵ = -10.7° (c = 0.13, CHCl₃).
Mass Spectrum (FAB) m/e 727 (M⁺+H).
¹H-Nuclear Magnetic Resonance Spectrum (CDCl₃, 270 MHz) δ (ppm):
1.20 (3H, triplet, J = 7.0 Hz);
1.48 (9H, singlet);
1.50 (9H, singlet);
1.95 (3H, singlet);
3.60 - 4.10 (6H, multiplet);
4.30 - 4.50 (2H, multiplet);
5.10 (1H, multiplet);
5.95 (1H, doublet, J = 2.7 Hz);
6.55 (1H, doublet, J = 8.0 Hz);
6.95 (1H, singlet);
7.20 - 7.50 (10H, multiplet);
8.50 (1H, doublet, J = 8.5 Hz).

### 31(ii) Diphenylmethyl 5-acetamido-4-(N,N'-bis-t-butoxycarbonylguanidino)-9-O-octanoyl-2,3,4,5,7-pentadeoxy-7-ethoxy-D-glycero-D-galacto-non-2-enopyranosoate

30 mg (0.041 mmol) of diphenylmethyl 5-acetamido-4-(N,N'-bis-t-butoxycarbonylguanidino)-2,3,4,5,7-pentadeoxy-7-ethoxy-D-glycero-D-galacto-non-2-enopyranosoate [prepared as described in step (i) above] were dissolved in 2 ml of methylene chloride, and 6 mg (0.06 mmol) of triethylamine and 8 mg (0.05 mmol) of octanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 24 mg (yield 69%) of the title compound as a colourless amorphous substance.
Rf= 0.30 (20 : 1 = methylene chloride : methanol).
[α]_{D}²⁵ = -14.0° (c = 0.05, CHCl₃).
Mass Spectrum (FAB) m/e 854 (M⁺+H).
¹H-Nuclear Magnetic Resonance Spectrum (CDCl₃, 270 MHz) δ (ppm):
0.85 (3H, multiplet);
1.20 (3H, triplet, J = 7.0 Hz);
1.20 - 1.30 (8H, multiplet);
1.48 (9H, singlet);
1.50 (9H, singlet);
1.55-1.70 (2H, multiplet);
1.95 (3H, singlet);
2.33 (2H, triplet, J = 7.5 Hz);
3.50 (1H, triplet, J = 5.5 Hz);
3.59 (2H, quartet, J = 7.0 Hz);
4.10 - 4.50 (5H, multiplet);
5.10 (1H, doubled doublet of doublets, J = 2.4, 9.0 & 9.0 Hz);
5.95 (1H, doublet, J = 2.3 Hz);
6.10 (1H, doublet, J = 8.7 Hz);
6.94 (1H, singlet);
7.20 - 7.50 (10H, multiplet);
8.55 (1H, doublet, J = 8.7 Hz).

### 31(iii) 5-Acetamido-4-guanidino-9-O-octanoyl-2,3,4,5,7-pentadeoxy-7-ethoxy-D-glycero-D-galacto-non-2-enopyranosoic acid trifluoroacetic acid salt

20 mg (0.023 mmol) of diphenylmethyl 5-acetamido-4-(N,N'-bis-t-butoxycarbonylguanidino)-9-O-octanoyl-2,3,4,5,7-pentadeoxy-7-ethoxy-D-glycero-D-galacto-non-2-enopyranosoate [prepared as described in step (ii) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 10 mg (yield 73%) of the title compound as a colourless solid.
Rf= 0.3 (2 : 5 : 1 = isopropanol: ethyl acetate : water).
[α]_{D}²⁵ = +55° (c = 0.10, CH₃OH).
Mass Spectrum (FAB) m/e 487 (M⁺+H).
¹H-Nuclear Magnetic Resonance Spectrum (CD₃OD, 270 MHz) δ (ppm):
0.90 (3H, multiplet);
1.15 (3H, triplet, J = 7.0 Hz);
1.20 - 1.40 (8H, multiplet);
1.55 - 1.70 (2H, multiplet);
2.00 (3H, singlet);
2.35 (2H, triplet, J = 7.5 Hz);
3.45 - 3.60 (3H, multiplet);
4.10 - 4.40 (6H, multiplet);
5.53 (1H, doublet, J = 1.8 Hz).

### EXAMPLE 32

### 5-Acetamido-4-guanidino-9-O-dodecanoyl-2,3,4,5,7-pentadeoxy-7-ethoxy-D-glycero-D-galacto-non-2-enopyranosoic acid trifluoroacetic acid salt (Compound No. 199-40)

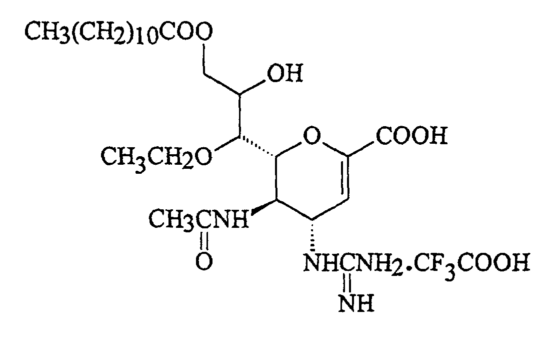

### 32(i) Diphenylmethyl 5-acetamido-4-(N,N'-bis-t-butoxycarbonylguanidino)-9-O-dodecanoyl-2,3,4,5,7-pentadeoxy-7-ethoxy-D-glycero-D-galacto-non-2-enopyranosoate

30 mg (0.041 mmol) of diphenylmethyl 5-acetamido-4-(N,N'-bis-t-butoxycarbonylguanidino)-2,3,4,5,7-pentadeoxy-7-ethoxy-D-glycero-D-galacto-non-2-enopyranosoate [prepared as described in Example 31(i)] were dissolved in 2 ml of methylene chloride, and 6 mg (0.06 mmol) of triethylamine and 11 mg (0.05 mmol) of dodecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 29 mg (yield 78%) of the title compound as a colourless amorphous substance.
Rf= 0.35 (20 : 1 = methylene chloride : methanol).
[α]D²⁵ = -9.0° (c = 0.1, CHCl₃).
Mass Spectrum (FAB) m/e 910 (M⁺+H).
¹H-Nuclear Magnetic Resonance Spectrum (CDCl₃, 270 MHz) δ (ppm):
0.85 (3H, multiplet);
1.20 (3H, triplet, J = 7.0 Hz);
1.20 - 1.30 (16H, multiplet);
1.48 (9H, singlet);
1.50 (9H, singlet);
1.55 - 1.70 (2H, multiplet);
1.95 (3H, singlet);
2.33 (2H, triplet, J = 7.5 Hz);
3.50 (1H, triplet, J = 5.5 Hz);
3.59 (2H, quartet, J = 7.0 Hz);
4.10 - 4.50 (5H, multiplet);
5.10 (1H, doubled doublet of doublets, J = 2.4, 9.0 & 9.0 Hz);
5.95 (1H, doublet, J = 2.3 Hz);
6.10 (1H, doublet, J = 8.7 Hz);
6.94 (1H, singlet);
7.20 - 7.50 (10H, multiplet);
8.55 (1H, doublet, J = 8.7 Hz).

### 32(ii) 5-Acetamido-4-guanidino-9-O-dodecanoyl-2,3,4,5,7-pentadeoxy-7-ethoxy-D-glycero-D-galacto-non-2-enopyranosoic acid trifluoroacetic acid salt

28 mg (0.03 mmol) of diphenylmethyl 5-acetamido-4-(N,N'-bis-t-butoxycarbonylguanidino)-9-O-dodecanoyl-2,3,4,5,7-pentadeoxy-7-ethoxy-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 12 mg (yield 61%) of the title compound as a colourless solid.
Rf= 0.3 (2 : 5 : 1 = isopropanol: ethyl acetate : water).
[α]_{D}²⁵ = +50° (c = 0.05, CH₃OH).
Mass Spectrum (FAB) m/e 543 (M⁺+H).
¹H-Nuclear Magnetic Resonance Spectrum (CD₃OD, 270 MHz) δ (ppm):
0.90 (3H, multiplet);
1.15 (3H, triplet, J = 7.0 Hz);
1.20 - 1.40 (16H, multiplet);
1.55 - 1.70 (2H, multiplet);
2.00 (3H, singlet);
2.35 (2H, triplet, J = 7.5 Hz);
3.45 - 3.60 (3H, multiplet);
4.10 - 4.40 (6H, multiplet);
5.53 (1H, doublet, J J = 1.8 Hz).

### EXAMPLE 33

### 5-Acetamido-4-guanidino-9-O-tetradecanoyl-2,3,4,5,7-pentadeoxy-7-ethoxy-D-glycero-D-galacto-non-2-enopyranosoic acid trifluoroacetic acid salt (Compound No. 199-41)

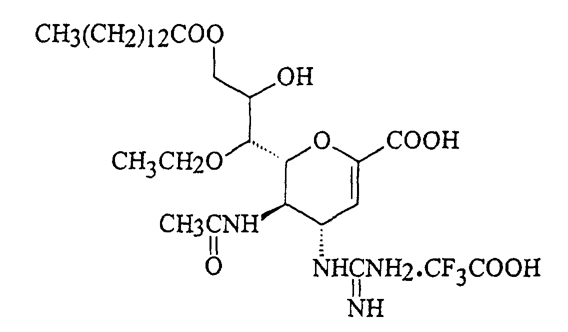

### 33(i) Diphenylmethyl 5-acetamido-4-(N,N'-bis-t-butoxycarbonylguanidino)-9-O-tetradecanoyl-2,3,4,5,7-pentadeoxy-7-ethoxy-D-glycero-D-galacto-non-2-enopyranosoate

30 mg (0.041 mmol) of diphenylmethyl 5-acetamido-4-(N,N'-bis-t-butoxycarbonylguanidino)-2,3,4,5,7-pentadeoxy-7-ethoxy-D-glycero-D-galacto-non-2-enopyranosoate [prepared as described in Example 31(i)] were dissolved in 2 ml of methylene chloride, and 6 mg (0.06 mmol) of triethylamine and 12 mg (0.05 mmol) of tetradecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 30 mg (yield 78%) of the title compound as a colourless amorphous substance.
Rf = 0.35 (20 : 1 = methylene chloride : methanol).
[α]_{D}²⁵ = -8.0° (c = 0.1, CHCl₃).
Mass Spectrum (FAB) m/e 938 (M⁺+H).
¹H-Nuclear Magnetic Resonance Spectrum (CDCl₃, 270 MHz) δ (ppm):
0.85 (3H, multiplet);
1.20 (3H, triplet, J = 7.0 Hz);
1.20 - 1.30 (20H, multiplet);
1.48 (9H, singlet);
1.50 (9H, singlet);
1.55 - 1.70 (2H, multiplet);
1.95 (3H, singlet);
2.33 (2H, triplet, J = 7.5 Hz);
3.50 (1H, triplet, J = 5.5 Hz);
3.59 (2H, quartet, J = 7.0 Hz);
4.10 - 4.50 (5H, multiplet);
5.10 (1H, doubled doublet of doublets, J = 2.4, 9.0 & 9.0 Hz);
5.95 (1H, doublet, J = 2.3 Hz);
6.10 (1H, doublet, J = 8.7 Hz);
6.94 (1H, singlet);
7.20 - 7.50 (10H, multiplet);
8.55 (1H, doublet, J = 8.7 Hz).

### 33(ii) 5-Acetamido-4-guanidino-9-O-tetradecanoyl-2,3,4,5,7-pentadeoxy-7-ethoxy-D-glycero-D-galacto-non-2-enopyranosoic acid trifluoroacetic acid salt

28 mg (0.03 mmol) of diphenylmethyl 5-acetamido-4-(N,N'-bis-t-butoxycarbonylguanidino)-9-O-tetradecanoyl-2,3,4,5,7-pentadeoxy-7-ethoxy-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 13 mg (yield 74%) of the title compound as a colourless solid.
Rf= 0.3 (2 : 5 : 1 = isopropanol : ethyl acetate : water).
[α]_{D}²⁵ = +38.0° (c = 0.05, CH₃OH).
Mass Spectrum (FAB) m/e 571 (M⁺+H).
¹H-Nuclear Magnetic Resonance Spectrum (CD₃OD, 270 MHz) δ (ppm):
0.90 (3H, multiplet);
1.15 (3H, triplet, J = 7.0 Hz);
1.20 - 1.40 (20H, multiplet);
1.55 - 1.70 (2H, multiplet);
2.00 (3H, singlet);
2.35 (2H, triplet, J = 7.5 Hz);
3.45 - 3.60 (3H, multiplet);
4.10 - 4.40 (6H, multiplet);
5.53 (1H, doublet, J = 1.8 Hz).

### EXAMPLE 34

### 5-Acetamido-4-guanidino-9-O-hexadecanoyl-2,3,4,5,7-pentadeoxy-7-ethoxy-D-glycero-D-galacto-non-2-enopyranosoic acid trifluoroacetic acid salt (Compound No. 199-42)

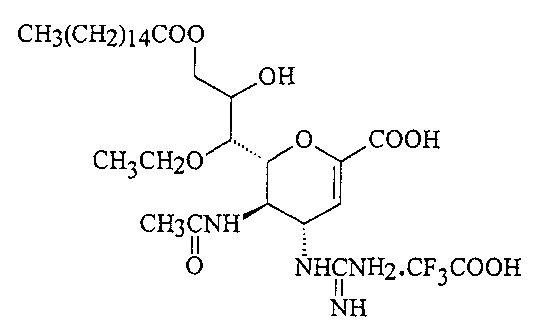

### 34(i) Diphenylmethyl 5-acetamido-4-(N,N'-bis-t-butoxycarbonylguanidino)-9-O-hexadecanoyl-2,3,4,5,7-pentadeoxy-7-ethoxy-D-glycero-D-galacto-non-2-enopyranosoate

30 mg (0.041 mmol) of diphenylmethyl 5-acetamido-4-(N,N'-bis-t-butoxycarbonylguanidino)-2,3,4,5,7-pentadeoxy-1-ethoxy-D-glycero-D-galacto-non-2-enopyranosoate [prepared as described in Example 31(i)] were dissolved in 2 ml of methylene chloride, and 6 mg (0.06 mmol) of triethylamine and 14 mg (0.05 mmol) of hexadecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 31 mg (yield 78%) of the title compound as a colourless amorphous substance.
Rf= 0.35 (20 : 1 = methylene chloride : methanol).
[α]_{D}²⁵ = -7.5° (c = 0.1, CHCl₃).
Mass Spectrum (FAB) m/e 966 (M⁺+H).
¹H-Nuclear Magnetic Resonance Spectrum (CDCl₃, 270 MHz) δ (ppm):
0.85 (3H, multiplet);
1.20 (3H, triplet, J = 7.0 Hz);
1.20 - 1.30 (24H, multiplet);
1.48 (9H, singlet);
1.50 (9H, singlet);
1.55 - 1.70 (2H, multiplet);
1.95 (3H, singlet);
2.33 (2H, triplet, J = 7.5 Hz);
3.50 (1H, triplet, J = 5.5 Hz);
3.59 (2H, quartet, J = 7.0 Hz);
4.10 - 4.50 (5H, multiplet);
5.10 (1H, doubled doublet of doublets, J = 2.4, 9.0 & 9.0 Hz);
5.95 (1H, doublet, J = 2.3 Hz);
6.10 (1H, doublet, J = 8.7 Hz);
6.94 (1H, singlet);
7.20 - 7.50 (10H, multiplet);
8.55 (1H, doublet, J = 8.7 Hz).

### 34(ii) 5-Acetamido-4-guanidino-9-O-hexadecanoyl-2,3,4,5,7-pentadeoxy-7-ethoxy-D-glycero-D-galacto-non-2-enopyranosoic acid trifluoroacetic acid salt

28 mg (0.029 mmol) of diphenylmethyl 5-acetamido-4-(N,N'-bis-t-butoxycarbonylguanidino)-9-O-hexadecanoyl-2,3,4,5,7-pentadeoxy-7-ethoxy-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 12 mg (yield 58%) of the title compound as a colourless solid.
Rf= 0.3 (2 : 5 : 1 = isopropanol: ethyl acetate : water).
[α]_{D}²⁵ = +30.0° (c = 0.1, CH₃OH).
Mass Spectrum (FAB) m/e 599 (M⁺+H).
¹H-Nuclear Magnetic Resonance Spectrum (CD₃OD, 270 MHz) δ (ppm):
0.90 (3H, multiplet);
1.15 (3H, triplet, J = 7.0 Hz);
1.20 - 1.40 (24H, multiplet);
1.55-1.70 (2H, multiplet);
2.00 (3H, singlet);
2.35 (2H, triplet, J = 7.5 Hz);
3.45 - 3.60 (3H, multiplet);
4.10 - 4.40 (6H, multiplet);
5.53 (1H, doublet, J = 1.8 Hz).

### EXAMPLE 35

### 5-Acetamido-4-guanidino-9-O-octanoyl-2,3,4,5,7-pentadeoxy-7-methoxy-D-glycero-D-galacto-non-2-enopyranosoic acid trifluoroacetic acid salt (Compound No. 163-38)

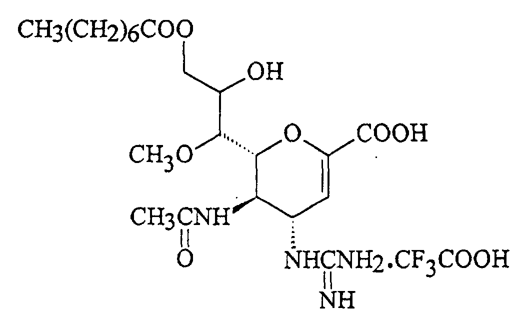

### 35(i) Diphenylmethyl 5-acetamido-4-(N,N'-bis-t-butoxycarbonylguanidino)-9-O-octanoyl-2,3,4,5,7-pentadeoxy-7-methoxy-D-glycero-D-galacto-non-2-enopyranosoate

57 mg (0.08 mmol) of diphenylmethyl 5-acetamido-4-(N,N'-bis-t-butoxycarbonylguanidino)-2,3,4,5,7-pentadeoxy-7-methoxy-D-glycero-D-galacto-non-2-enopyranosoate [prepared as described in Example 29(i)] were dissolved in 2 ml of methylene chloride, and 11 mg (0.10 mmol) of triethylamine and 16 mg (0.09 mmol) of octanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 38 mg (yield 58%) of the title compound as a colourless amorphous substance.
Rf = 0.5 (10 : 1 = methylene chloride : methanol).
[α]_{D}²⁵ = +2.7° (c = 0.11, CHCl₃).
Mass Spectrum (FAB) m/e 839 (M⁺+H).
¹H-Nuclear Magnetic Resonance Spectrum (CDCl₃, 270 MHz) δ (ppm):
0.85 (3H, multiplet);
1.20 - 1.30 (8H, multiplet);
1.48 (9H, singlet);
1.50 (9H, singlet);
1.55 - 1.70 (2H, multiplet);
1.98 (3H, singlet);
2.33 (2H, triplet, J = 7.5 Hz);
3.40 (1H, multiplet);
3.48 (3H, singlet);
4.10 - 4.50 (5H, multiplet);
5.18 (1H, doubled doublet of doublets, J = 2.4, 9.0 & 9.0 Hz);
5.95 (1H, doublet, J = 2.3 Hz);
6.18 (1H, doublet, J = 8.7 Hz);
6.92 (1H, singlet);
7.20 - 7.50 (10H, multiplet);
8.55 (1H, doublet, J = 8.7 Hz).

### 35(ii) 5-Acetamido-4-guanidino-9-O-octanoyl-2,3,4,5,7-pentadeoxy-7-methoxy-D-glycero-D-galacto-non-2-enopyranosoic acid trifluoroacetic acid salt

31 mg (0.037 mmol) of diphenylmethyl 5-acetamido-4-(N,N'-bis-t-butoxycarbonylguanidino)-9-O-octanoyl-2,3,4,5,7-pentadeoxy-7-methoxy-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 15 mg (yield 69%) of the title compound as a colourless solid.
Rf= 0.34 (2 : 5 : 1 = isopropanol : ethyl acetate : water).
[α]D²⁵ = +38.2° (c = 0.11, CH₃OH)
Mass Spectrum (FAB) m/e 473 (M⁺+H)
¹H-Nuclear Magnetic Resonance Spectrum (CD₃OD, 270 MHz) δ (ppm):
0.90 (3H, multiplet);
1.25 - 1.40 (8H, multiplet);
1.60 - 1.70 (2H, multiplet);
2.00 (3H, singlet);
2.37 (2H, triplet, J = 7.5 Hz);
3.38 (3H, singlet);
3.45 (1H, multiplet);
4.10 - 4.40 (6H, multiplet);
5.53 (1H, doublet, J = 1.8 Hz).

### EXAMPLE 36

### 5-Acetamido-4-guanidino-9-O-decanoyl-2,3,4,5,7-pentadeoxy-7-methoxy-D-glycero-D-galacto-non-2-enopyranosoic acid trifluoroacetic acid salt (Compound No. 163-39)

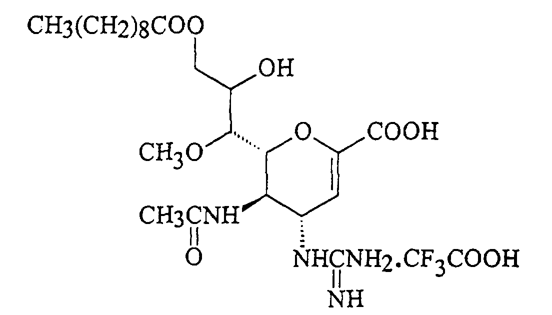

### 36(i) Diphenylmethyl 5-acetamido-4-(N,N'-bis-t-butoxycarbonylguanidino)-9-O-decanoyl-2,3,4,5,7-pentadeoxy-7-methoxy-D-glycero-D-galacto-non-2-enopyranosoate

56 mg (0.08 mmol) of diphenylmethyl 5-acetamido-4-(N,N'-bis-t-butoxycarbonylguanidino)-2,3,4,5,7-pentadeoxy-7-methoxy-D-glycero-D-galacto-non-2-enopyranosoate [prepared as described in Example 29(i)] were dissolved in 2 ml of methylene chloride, and 11 mg (0.10 mmol) of triethylamine and 18 mg (0.09 mmol) of decanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 40 mg (yield 50%) of the title compound as a colourless amorphous substance.
Rf= 0.5 (10 : 1 = methylene chloride : methanol).
[α]_{D}²⁵ = +13.9° (c = 0.11, CHCl₃)
Mass Spectrum (FAB) m/e 867 (M⁺+H)
¹H-Nuclear Magnetic Resonance Spectrum (CDCl₃, 270 MHz) δ (ppm):
0.85 (3H, multiplet);
1.20 - 1.30 (12H, multiplet);
1.48 (9H, singlet);
1.50 (9H, singlet);
1.55 - 1.70 (2H, multiplet);
1.98 (3H, singlet);
2.33 (2H, triplet, J = 7.5 Hz);
3.40 (1H, multiplet);
3.48 (3H, singlet);
4.10 - 4.50 (5H, multiplet);
5.18 (1H, doubled doublet of doublets, J = 2.4, 9.0 & 9.0 Hz);
5.95 (1H, doublet, J = 2.3 Hz);
6.18 (1H, doublet, J = 8.7 Hz);
6.92 (1H, singlet);
7.20 - 7.50 (10H, multiplet);
8.55 (1H, doublet, J = 8.7 Hz).

### 36(ii) 5-Acetamido-4-guanidino-9-O-decanoyl-2,3,4,5,7-pentadeoxy-7-methoxy-D-glycero-D-galacto-non-2-enopyranosoic acid trifluoroacetic acid salt

33 mg (0.04 mmol) of diphenylmethyl 5-acetamido-4-(N,N'-bis-t-butoxycarbonylguanidino)-9-O-decanoyl-2,3,4,5,7-pentadeoxy-7-methoxy-D-glycero-D-galacto-non-2-enopyranosoate [prepared as described in Example 36(i)] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 20 mg (yield 85%) of the title compound as a colourless solid.
Rf= 0.34 (2 : 5 : 1 = isopropanol : ethyl acetate : water).
[α]_{D}²⁵ = +33.3° (c = 0.11, CH₃OH).
Mass Spectrum (FAB) m/e 501 (M⁺+H)
¹H-Nuclear Magnetic Resonance Spectrum (CD₃OD, 270 MHz) δ (ppm):
0.90 (3H, multiplet);
1.25 - 1.40 (12H, multiplet);
1.60 - 1.70 (2H, multiplet);
2.00 (3H, singlet);
2.37 (2H, triplet, J = 7.5 Hz);
3.38 (3H, singlet);
3.45 (1H, multiplet);
4.10 - 4.40 (6H, multiplet);
5.53 (1H, doublet, J = 1.8 Hz).

### EXAMPLE 37

### 5-Acetamido-4-guanidino-9-O-dodecanoyl-2,3,4,5,7-pentadeoxy-7-methoxy-D-glycero-D-galacto-non-2-enopyranosoic acid trifluoroacetic acid salt (Compound No. 163-40)

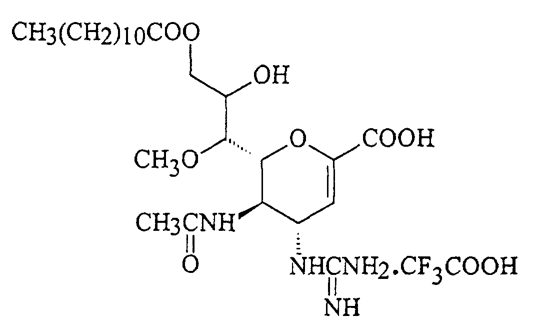

### 37(i) Diphenylmethyl 5-acetamido-4-(N,N'-bis-t-butoxycarbonylguanidino)-9-O-dodecanoyl-2,3,4,5,7-pentadeoxy-7-methoxy-D-glycero-D-galacto-non-2-enopyranosoate

51 mg (0.07 mmol) of diphenylmethyl 5-acetamido-4-(N,N'-bis-t-butoxycarbonylguanidino)-2,3,4,5,7-pentadeoxy-7-methoxy-D-glycero-D-galacto-non-2-enopyranosoate [prepared as described in Example 29(i)] were dissolved in 2 ml of methylene chloride, and 9 mg (0.09 mmol) of triethylamine and 19 mg (0.09 mmol) of dodecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 39 mg (yield 61%) of the title compound as a colourless amorphous substance.
Rf= 0.6 (20 : 1 = methylene chloride : methanol).
[α]_{D}²⁵ = +2.4° (c = 0.13, CHCl₃)
Mass Spectrum (FAB) m/e 895 (M⁺+H)
¹H-Nuclear Magnetic Resonance Spectrum (CDCl₃, 270 MHz) δ (ppm):
0.85 (3H, multiplet);
1.20 - 1.30 (16H, multiplet);
1.48 (9H, singlet);
1.50 (9H, singlet);
1.55 - 1.70 (2H, multiplet);
1.98 (3H, singlet);
2.33 (2H, triplet, J = 7.5 Hz);
3.40 (1H, multiplet);
3.48 (3H, singlet);
4.10 - 4.50 (5H, multiplet);
5.18 (1H, doubled doublet of doublets, J = 2.4, 9.0 & 9.0 Hz);
5.95 (1H, doublet, J = 2.3 Hz);
6.18 (1H, doublet, J = 8.7 Hz);
6.92 (1H, singlet);
7.20 - 7.50 (10H, multiplet);
8.55 (1H, doublet, J = 8.7 Hz).

### 37(ii) 5-Acetamido-4-guanidino-9-O-dodecanoyl-2,3,4,5,7-pentadeoxy-7-methoxy-D-glycero-D-galacto-non-2-enopyranosoic acid trifluoroacetic acid salt

31 mg (0.035 mmol) of diphenylmethyl 5-acetamido-4-(N,N'-bis-t-butoxycarbonylguanidino)-9-O-dodecanoyl-2,3,4,5,7-pentadeoxy-7-methoxy-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 22 mg (yield 98 %) of the title compound as a colourless solid.
Rf= 0.31 (2 : 5 : 1 = isopropanol : ethyl acetate : water).
[α]_{D}²⁵ = +25.8° (c = 0.16, CH₃OH).
Mass Spectrum (FAB) m/e 529 (M⁺+H).
¹H-Nuclear Magnetic Resonance Spectrum (CD₃OD, 270 MHz) δ (ppm):
0.90 (3H, multiplet);
1.25 - 1.40 (16H, multiplet);
1.60 - 1.70 (2H, multiplet);
2.00 (3H, singlet);
2.37 (2H, triplet, J = 7.5 Hz);
3.38 (3H, singlet);
3.45 (1H, multiplet);
4.10 - 4.40 (6H, multiplet);
5.53 (1H, doublet, J = 1.8 Hz).

### EXAMPLE 38

### 5-Acetamido-4-guanidino-9-O-hexadecanoyl-2,3,4,5,7-pentadeoxy-7-methoxy-D-glycero-D-galacto-non-2-enopyranosoic acid trifluoroacetic acid salt (Compound No. 163-42)

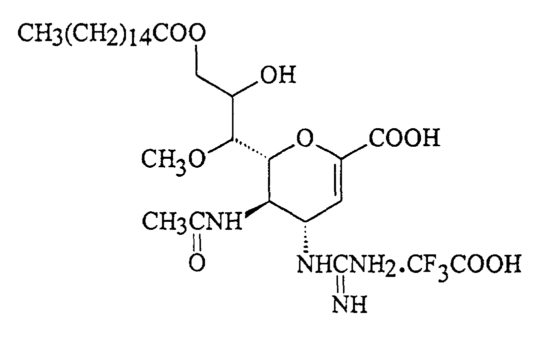

### 38(i) Diphenylmethyl 5-acetamido-4-(N,N'-bis-t-butoxycarbonylguanidino)-9-O-hexadecanoyl-2,3,4,5,7-pentadeoxy-7-methoxy-D-glycero-D-galacto-non-2-enopyranosoate

50 mg (0.07 mmol) of diphenylmethyl 5-acetamido-4-(N,N'-bis-t-butoxycarbonylguanidino)-2,3,4,5,7-pentadeoxy-7-methoxy-D-glycero-D-galacto-non-2-enopyranosoate [prepared as described in Example 29(i)] were dissolved in 2 ml of methylene chloride, and 9 mg (0.09 mmol) of triethylamine and 23 mg (0.084 mmol) of hexadecanoyl chloride were added to the resulting solution, whilst ice-cooling. The mixture was then stirred at 0°C for 1 hour. At the end of this time, the reaction mixture was poured into a 2-layer solution of 5 ml of ethyl acetate and 3 ml of a saturated aqueous solution of sodium hydrogencarbonate, and the organic layer was separated and washed with a saturated aqueous solution of sodium chloride. It was then dried over anhydrous sodium sulphate, and the solvent was removed by distillation under reduced pressure. The resulting residue was purified by silica gel column chromatography, using a 50 : 1 by volume mixture of methylene chloride and methanol as the eluent, to obtain 40 mg (yield 60%) of the title compound as a colourless amorphous substance.
Rf= 0.6 (10 : 1 = methylene chloride : methanol).
[α]D²⁵ = +5.5° (c = 0.11, CHCl₃).
Mass Spectrum (FAB) m/e 951 (M⁺+H).
¹H-Nuclear Magnetic Resonance Spectrum (CDCl₃, 270 MHz) δ (ppm):
0.85 (3H, multiplet);
1.20 - 1.30 (24H, multiplet);
1.48 (9H, singlet);
1.50 (9H, singlet);
1.55 - 1.70 (2H, multiplet);
1.98 (3H, singlet);
2.33 (2H, triplet, J = 7.5 Hz);
3.40 (1H, multiplet);
3.48 (3H, singlet);
4.10 - 4.50 (5H, multiplet);
5.18 (1H, doubled doublet of doublets, J = 2.4, 9.0 & 9.0 Hz);
5.95 (1H, doublet, J = 2.3 Hz);
6.18 (1H, doublet, J = 8.7 Hz);
6.92 (1H, singlet);
7.20 - 7.50 (10H, multiplet);
8.55 (1H, doublet, J = 8.7 Hz).

### 38(ii) 5-Acetamido-4-guanidino-9-O-hexadecanoyl-2,3,4,5,7-pentadeoxy-7-methoxy-D-glycero-D-galacto-non-2-enopyranosoic acid trifluoroacetic acid salt

34 mg (0.036 mmol) of diphenylmethyl 5-acetamido-4-(N,N'-bis-t-butoxycarbonylguanidino)-9-O-hexadecanoyl-2,3,4,5,7-pentadeoxy-7-methoxy-D-glycero-D-galacto-non-2-enopyranosoate [prepared as described in step (i) above] were dissolved in 3 ml of a 3 : 1 by volume mixture of methylene chloride and trifluoroacetic acid, and the mixture was stirred at room temperature for 5 hours. At the end of this time, the solvent was removed by distillation under reduced pressure, and the residue was purified by silica gel column chromatography, using a 2 : 5 : 1 by volume mixture of isopropanol, ethyl acetate and water as the eluent, to obtain 23 mg (yield 92%) of the title compound as a colourless solid.
Rf= 0.47 (2 : 5 : 1 = isopropanol : ethyl acetate : water).
[α]_{D}²⁵ = +21.6° (c = 0.12, CH₃OH).
Mass Spectrum (FAB) m/e 585 (M⁺+H).
¹H-Nuclear Magnetic Resonance Spectrum (CD₃OD, 270 MHz) δ (ppm):
0.90 (3H, multiplet);
1.25 - 1.40 (24H, multiplet);
1.60 - 1.70 (2H, multiplet);
2.00 (3H, singlet);
2.37 (2H, triplet, J = 7.5 Hz);
3.38 (3H, singlet);
3.45 (1H, multiplet);
4.10 - 4.40 (6H, multiplet);
5.53 (1H, doublet, J = 1.8 Hz).

## Claims

1. Compounds of formula (I): 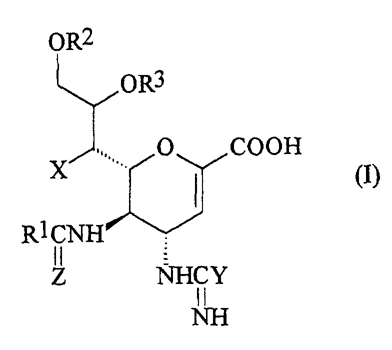 in which:
R¹ represents an alkyl group having from 1 to 4 carbon atoms;
R² and R³ are the same as or different from each other and each represents a hydrogen atom or an aliphatic acyl group having from 2 to 25 carbon atoms;
X represents a halogen atom or an alkoxy group having from 1 to 4 carbon atoms;
Y represents a group of formula R^{b}R^{c}N- or a group of formula R^{b}R^{c}N-O-, where R^{b} and R^{c} are the same as or different from each other and each represents a hydrogen atom or an alkyl group having from 1 to 4 carbon atoms; and
Z represents an oxygen atom or a sulfur atom;
and pharmaceutically acceptable salts and esters thereof.

2. A compound according to Claim 1, in which R¹ represents a methyl group.

3. A compound according to Claim 1, in which R² represents a hydrogen atom or an aliphatic carboxylic acyl group having from 6 to 25 carbon atoms.

4. A compound according to Claim 3, in which R² represents a hydrogen atom or an aliphatic carboxylic acyl group having from 8 to 16 carbon atoms.

5. A compound according to Claim 3, in which R² represents a hydrogen atom or an aliphatic carboxylic acyl group having from 8 to 16 carbon atoms and R³ represents a hydrogen atom.

6. A compound according to Claim 3, in which R² represents an octanoyl, decanoyl, dodecanoyl, myristoyl or palmitoyl group.

7. A compound according to Claim 5, in which R² represents an octanoyl, decanoyl, dodecanoyl, myristoyl or palmitoyl group and R³ represents a hydrogen atom.

8. A compound according to Claim 1, in which R³ represents a hydrogen atom or an aliphatic carboxylic acyl group having from 6 to 25 carbon atoms.

9. A compound according to Claim 1, in which R³ represents a hydrogen atom or an aliphatic carboxylic acyl group having from 8 to 16 carbon atoms.

10. A compound according to Claim 9, in which R³ represents a hydrogen atom, or an octanoyl, decanoyl, dodecanoyl, myristoyl or palmitoyl group.

11. A compound according to Claim 1, in which X represents a fluorine atom, a methoxy group or an ethoxy group.

12. A compound according to Claim 1, in which Y represents an amino group or a group of formula R^{b}R^{c}N-O-, where R^{b} and R^{c} are as defined in Claim 1.

13. A compound according to Claim 12, in which Y represents an amino group or an aminooxy group.

14. A compound according to Claim 12, in which Y represents an amino group.

15. A compound according to Claim 1, in which Z represents an oxygen atom.

16. A compound according to Claim 1, in which:
R¹ represents a methyl group;
R² represents a hydrogen atom or an aliphatic carboxylic acyl group having from 6 to 25 carbon atoms;
R³ represents a hydrogen atom or an aliphatic carboxylic acyl group having from 6 to 25 carbon atoms;
X represents a halogen atom or an alkoxy group having from 1 to 4 carbon atoms;
Y represents an amino group or a group of formula R^{b}R^{c}N-O-, where R^{b} and R^{c} are as defined above; and
Z represents an oxygen atom.

17. A compound according to Claim 1, in which:
R¹ represents a methyl group,
R² represents a hydrogen atom or an aliphatic carboxylic acyl group having from 8 to 16 carbon atoms;
R³ represents a hydrogen atom or an aliphatic carboxylic acyl group having from 8 to 16 carbon atoms;
X represents a fluorine atom, a methoxy group or an ethoxy group;
Y represents an amino group or an aminooxy group; and
Z represents an oxygen atom.

18. A compound according to Claim 1, in which:
R¹ represents a methyl group,
R² represents a hydrogen atom or an aliphatic carboxylic acyl group having from 8 to 16 carbon atoms;
R³ represents a hydrogen atom;
X represents a fluorine atom, a methoxy group or an ethoxy group;
Y represents an amino group or an aminooxy group; and
Z represents an oxygen atom.

19. A compound according to Claim 1, in which:
R¹ represents a methyl group;
R² represents an octanoyl, decanoyl, dodecanoyl, myristoyl or palmitoyl group;
R³ represents a hydrogen atom, or an octanoyl, decanoyl, dodecanoyl, myristoyl or palmitoyl group;
X represents a fluorine atom, a methoxy group or an ethoxy group;
Y represents an amino group; and
Z represents an oxygen atom.

20. A compound according to Claim 1, in which:
R¹ represents a methyl group;
R² represents an octanoyl, decanoyl, dodecanoyl, myristoyl or palmitoyl group;
R³ represents a hydrogen atom;
X represents a fluorine atom, a methoxy group or an ethoxy group;
Y represents an amino group; and
Z represents an oxygen atom.

21. The following compounds according to Claim 1:
5-acetamido-4-guanidino-2,3,4,5,7-pentadeoxy-7-fluoro-D-glycero-D-galacto-non-2-enopyranosoic acid;
5-acetamido-4-guanidino-9-O-dodecanoyl-2,3,4,5,7-pentadeoxy-7-fluoro-D-glycero-D-galacto-non-2-enopyranosoic acid;
5-acetamido-4-guanidino-9-O-myristoyl-2,3,4,5,7-pentadeoxy-7-fluoro-D-glycero-D-galacto-non-2-enopyranosoic acid;
5-acetamido-4-guanidino-9-O-palmitoyl-2,3,4,5,7-pentadeoxy-7-fluoro-D-glycero-D-galacto-non-2-enopyranosoic acid;
5-acetamido-4-guanidino-2,3,4,5,7-pentadeoxy-7-methoxy-D-glycero-D-galacto-non-2-enopyranosoic acid;
5-acetamido-4-guanidino-9-O-myristoyl-2,3,4,5,7-pentadeoxy-7-methoxy-D-glycero-D-galacto-non-2-enopyranosoic acid;
5-acetamido-4-guanidino-2,3,4,5,7-pentadeoxy-7-ethoxy-D-glycero-D-galacto-non-2-enopyranosoic acid;
5-acetamido-4-guanidino-9-O-octanoyl-2,3,4,5,7-pentadeoxy-7-ethoxy-D-glycero-D-galacto-non-2-enopyranosoic acid;
5-acetamido-4-guanidino-9-O-dodecanoyl-2,3,4,5,7-pentadeoxy-7-ethoxy-D-glycero-D-galacto-non-2-enopyranosoic acid;
5-acetamido-4-guanidino-9-O-myristoyl-2,3,4,5,7-pentadeoxy-7-ethoxy-D-glycero-D-galacto-non-2-enopyranosoic acid;
5-acetamido-4-guanidino-9-O-palmitoyl-2,3,4,5,7-pentadeoxy-7-ethoxy-D-glycero-D-galacto-non-2-enopyranosoic acid;
5-acetamido-4-guanidino-9-O-octanoyl-2,3,4,5,7-pentadeoxy-7-methoxy-D-glycero-D-galacto-non-2-enopyranosoic acid;
5-acetamido-4-guanidino-9-O-decanoyl-2,3,4,5,7-pentadeoxy-7-methoxy-D-glycero-D-galacto-non-2-enopyranosoic acid;
5-acetamido-4-guanidino-9-O-dodecanoyl-2,3,4,5,7-pentadeoxy-7-methoxy-D-glycero-D-galacto-non-2-enopyranosoic acid; and
5-acetamido-4-guanidino-9-O-hexadecanoyl-2,3,4,5,7-pentadeoxy-7-methoxy-D-glycero-D-galacto-non-2-enopyranosoic acid;
and pharmaceutically acceptable salts and esters thereof.

22. The use of a compound of formula (I) or a pharmaceutically acceptable salt or ester thereof, as claimed in any one of Claims 1 to 21, for the manufacture of a medicament for treating or preventing an infection in a mammal caused by a sialidase-bearing virus.

23. A pharmaceutical composition for the treatment or prevention of infections in a mammal caused by sialidase-bearing viruses, which composition comprises a sialidase inhibitory compound in admixture with a pharmaceutically acceptable carrier or diluent, in which the sialidase inhibitory compound is at least one compound of formula (I) or a pharmaceutically acceptable salt or ester thereof, as claimed in any one of Claims 1 to 21.

## Patentansprüche

1. Verbindungen der Formel (I): 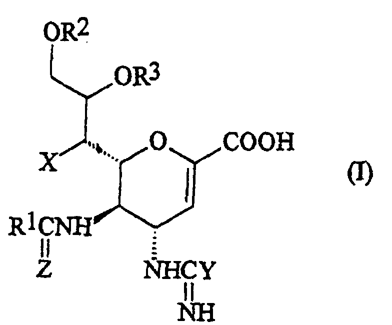 worin:
R¹ eine Alkylgruppe mit 1 bis 4 Kohlenstoffatomen darstellt,
R² und R³ gleich oder unterschiedlich sind und jeweils ein Wasserstoffatom oder eine aliphatische Acylgruppe mit 2 bis 25 Kohlenstoffatomen darstellen,
X ein Halogenatom oder eine Alkoxygruppe mit 1 bis 4 Kohlenstoffatomen darstellt,
Y ein Gruppe der Formel R^{b}R^{c}N- oder eine Gruppe der Formel R^{b}R^{c}N-O- darstellt, worin R^{b} und R^{c} gleich oder unterschiedlich sind und jeweils ein Wasserstoffatom oder eine Alkylgruppe mit 1 bis 4 Kohlenstoffatomen darstellen, und
Z ein Sauerstoffatom oder Schwefelatom ist,
und pharmazeutisch geeignete Salze und Ester davon.

2. Verbindung nach Anspruch 1, worin R¹ eine Methylgruppe darstellt.

3. Verbindung nach Anspruch 1, worin R² ein Wasserstoffatom oder eine Acylgruppe einer aliphatischen Carbonsäure mit 6 bis 25 Kohlenstoffatomen darstellt.

4. Verbindung nach Anspruch 3, worin R² ein Wasserstoffatom oder eine Acylgruppe einer aliphatischen Carbonsäure mit 8 bis 16 Kohlenstoffatomen darstellt.

5. Verbindung nach Anspruch 3, worin R² ein Wasserstoffatom oder eine Acylgruppe einer aliphatischen Carbonsäure mit 8 bis 16 Kohlenstoffatomen darstellt und R³ ein Wasserstoffatom darstellt.

6. Verbindung nach Anspruch 3, worin R² eine Octanoyl-, Decanoyl-, Dodecanoyl-, Myristoyl- oder Palmitoylgruppe darstellt.

7. Verbindung nach Anspruch 5, worin R² eine Octanoyl-, Decanoyl-, Dodecanoyl-, Myristoyl- oder Palmitoylgruppe darstellt und R³ ein Wasserstoffatom darstellt.

8. Verbindung nach Anspruch 1, worin R³ ein Wasserstoffatom oder eine Acylgruppe einer aliphatischen Carbonsäure mit 6 bis 25 Kohlenstoffatomen darstellt.

9. Verbindung nach Anspruch 1, worin R³ ein Wasserstoffatom oder eine Acylgruppe einer aliphatischen Carbonsäure mit 8 bis 16 Kohlenstoffatomen darstellt.

10. Verbindung nach Anspruch 9, worin R³ ein Wasserstoffatom oder eine Octanoyl-, Decanoyl-, Dodecanoyl-, Myristoyl- oder Palmitoylgruppe darstellt.

11. Verbindung nach Anspruch 1, worin X ein Fluoratom, eine Methoxygruppe oder eine Ethoxygruppe darstellt.

12. Verbindung nach Anspruch 1, worin Y eine Aminogruppe oder eine Gruppe der Formel R^{b}R^{c}N-O- darstellt, worin R^{b} und R^{c} wie in Anspruch 1 definiert sind.

13. Verbindung nach Anspruch 12, worin Y eine Aminogruppe oder eine Aminooxygruppe darstellt.

14. Verbindung nach Anspruch 12, worin Y eine Aminogruppe darstellt.

15. Verbindung nach Anspruch 1, worin Z ein Sauerstoffatom darstellt.

16. Verbindung nach Anspruch 1, worin:
R¹ eine Methylgruppe darstellt,
R² ein Wasserstoffatom oder eine Acylgruppe einer aliphatischen Carbonsäure mit 6 bis 25 Kohlenstoffatomen darstellt,
R³ ein Wasserstoffatom oder eine Acylgruppe einer aliphatischen Carbonsäure mit 6 bis 25 Kohlenstoffatomen darstellt,
X ein Halogenatom oder eine Alkoxygruppe mit 1 bis 4 Kohlenstoffatomen darstellt,
Y eine Aminogruppe oder eine Gruppe der Formel R^{b}R^{c}N-O- darstellt, worin R^{b} und R^{c} wie vorstehend definiert sind, und
Z ein Sauerstoffatom darstellt.

17. Verbindung nach Anspruch 1, worin:
R¹ eine Methylgruppe darstellt,
R² ein Wasserstoffatom oder eine Acylgruppe einer aliphatischen Carbonsäure mit 8 bis 16 Kohlenstoffatomen darstellt,
R³ ein Wasserstoffatom oder eine Acylgruppe einer aliphatischen Carbonsäure mit 8 bis 16 Kohlenstoffatomen darstellt,
X ein Fluoratom, eine Methoxygruppe oder eine Ethoxygruppe darstellt,
Y eine Aminogruppe oder eine Aminooxygruppe darstellt, und
Z ein Sauerstoffatom darstellt.

18. Verbindung nach Anspruch 1, worin:
R¹ eine Methylgruppe darstellt,
R² ein Wasserstoffatom oder eine Acylgruppe einer aliphatischen Carbonsäure mit 8 bis 16 Kohlenstoffatomen darstellt,
R³ ein Wasserstoffatom darstellt,
X ein Fluoratom, eine Methoxygruppe oder eine Ethoxygruppe darstellt,
Y eine Aminogruppe oder eine Aminooxygruppe darstellt, und
Z ein Sauerstoffatom darstellt.

19. Verbindung nach Anspruch 1, worin:
R¹ eine Methylgruppe darstellt,
R² eine Octanoyl-, Decanoyl-, Dodecanoyl-, Myristoyl- oder Palmitoylgruppe darstellt,
R³ ein Wasserstoffatom oder eine Octanoyl-, Decanoyl-, Dodecanoyl-, Myristoyl- oder Palmitoylgruppe darstellt,
X ein Fluoratom, eine Methoxygruppe oder eine Ethoxygruppe darstellt,
Y eine Aminogruppe darstellt, und
Z ein Sauerstoffatom darstellt.

20. Verbindung nach Anspruch 1, worin:
R¹ eine Methylgruppe darstellt.
R² eine Octanoyl-, Decanoyl-, Dodecanoyl-, Myristoyl- oder Palmitoylgruppe darstellt,
R³ ein Wasserstoffatom darstellt,
X ein Fluoratom, eine Methoxygruppe oder eine Ethoxygruppe darstellt,
Y eine Aminogruppe darstellt und
Z ein Sauerstoffatom darstellt.

21. Folgende Verbindung nach Anspruch 1:
5-Acetamido-4-guanidino-2,3,4,5,7-pentadeoxy-7-fluor-D-glycero-D-galacto-non-2-enopyranosonsäure;
5-Acetamido-4-guanidino-9-O-dodecanoyl-2,3,4,5,7-pentadeoxy-7-fluor-D-glycero-D-galacto-non-2-enopyranosonsäure;
5-Acetamido-4-guanidino-9-O-myristoyl-2,3,4,5,7-pentadeoxy-7-fluor-D-glycero-D-galacto-non-2-enopyranosonsäure;
5-Acetamido-4-guanidino-9-O-palmitoyl-2,3,4,5,7-pentadeoxy-7-fluor-D-glycero-D-galacto-non-2-enopyranosonsäure;
5-Acetamido-4-guanidino-2,3,4,5,7-pentadeoxy-7-methoxy-D-glycero-D-galacto-non-2-enopyranosonsäure;
5-Acetamido-4-guanidino-9-O-myristoyl-2,3,4,5,7-pentadeoxy-7-methoxy-D-glycero-D-galacto-non-2-enopyranosonsäure;
5-Acetamido-4-guanidino-2,3,4,5,7-pentadeoxy-7-ethoxy-D-glycero-D-galacto-non-2-enopyranosonsäure;
5-Acetamido-4-guanidino-9-O-octanoyl-2,3,4,5,7-pentadeoxy-7-ethoxy-D-glycero-D-galacto-non-2-enopyranosonsäure;
5-Acetamido-4-guanidino-9-O-dodecanoyl-2,3,4,5,7-pentadeoxy-7-ethoxy-D-glycero-D-galacto-non-2-enopyranosonsäure;
5-Acetamido-4-guanidino-9-O-myristoyl-2,3,4,5,7-pentadeoxy-7-ethoxy-D-glycero-D-galacto-non-2-enopyranosonsäure;
5-Acetamido-4-guanidino-9-O-palmitoyl-2,3,4,5,7-pentadeoxy-7-ethoxy-D-glycero-D-galacto-non-2-enopyranosonsäure;
5-Acetamido-4-guanidino-9-O-octanoyl-2,3,4,5,7-pentadeoxy-7-methoxy-D-glycero-D-galacto-non-2-enopyranosonsäure;
5-Acetamido-4-guanidino-9-O-decanoyl-2,3,4,5,7-pentadeoxy-7-methoxy-D-glycero-D-galacto-non-2-enopyranosonsäure;
5-Acetamido-4-guanidino-9-O-dodecanoyl-2,3,4,5,7-pentadeoxy-7-methoxy-D-glycero-D-galacto-non-2-enopyranosonsäure; und
5-Acetamido-4-guanidino-9-O-hexadecanoyl-2,3,4,5,7-pentadeoxy-7-methoxy-D-glycero-D-galacto-non-2-enopyranosonsäure
und pharmazeutisch geeignete Salze und Ester davon.

22. Verwendung einer Verbindung der Formel (I) oder eine pharmazeutisch geeigneten Salzes oder Esters davon nach einem der Ansprüche 1 bis 21, für die Herstellung eines Arzneimittels zur Behandlung oder Prävention einer Infektion in einem Säuger, die durch einen Sialidase tragenden Virus verursacht wird.

23. Arzneimittelzusammensetzung für die Behandlung oder Prävention von Infektionen in einem Säuger, die durch Sialidase tragende Viren verursacht werden, wobei die Zusammensetzung eine Sialidase inhibierende Verbindung im Gemisch mit einem pharmazeutisch geeigneten Träger oder Verdünnungsmittel umfaßt, wobei die Sialidase inhibierende Verbindung mindestens eine Verbindung der Formel (I) oder ein pharmazeutisch geeignetes Salz oder ein pharmazeutisch geeigneter Ester davon nach einem der Ansprüche 1 bis 21 ist.

## Revendications

1. Composés de formule (I) : 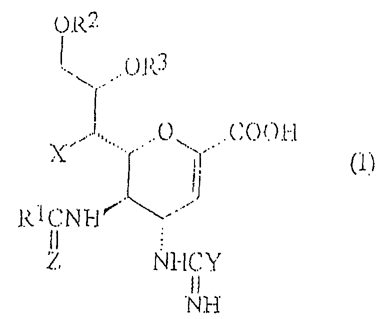 dans laquelle :
R¹ représente un groupe alkyle comportant de 1 à 4 atomes de carbone ;
R² et R³ sont identiques ou différents l'un de l'autre, et représentent chacun un atome d'hydrogène ou un groupe acyle aliphatique comportant de 2 à 25 atomes de carbone ;
X représente un atome d'halogène ou un groupe alkoxy comportant de 1 à 4 atomes de carbone ;
Y représente un groupe de formule R^{b}R^{c}N- ou un groupe de formule R^{b}R^{c}N-O-, R^{b} et R^{c} étant identiques ou différents l'un de l'autre, et ils représentent chacun un atome d'hydrogène ou un groupe alkyle comportant de 1 à 4 atomes de carbone ; et
Z représente un atome d'oxygène ou un atome de soufre ;
et leurs sels et esters pharmaceutiquement acceptables.

2. Composé selon la revendication 1, dans lequel R¹ représente un groupe méthyle.

3. Composé selon la revendication 1, dans lequel R² représente un atome d'hydrogène ou un groupe acyle d'acide carboxylique aliphatique comportant de 6 à 25 atomes de carbone.

4. Composé selon la revendication 3, dans lequel R² représente un atome d'hydrogène ou un groupe acyle d'acide carboxylique aliphatique comportant de 8 à 16 atomes de carbone.

5. Composé selon la revendication 3, dans lequel R² représente un atome d'hydrogène ou un groupe acyle d'acide carboxylique aliphatique comportant de 8 à 16 atomes de carbone, et R³ représente un atome d'hydrogène.

6. Procédé selon la revendication 3, dans lequel R² représente un groupe octanoyle, décanoyle, dodécanoyle, myristoyle ou palmitoyle.

7. Composé selon la revendication 5, dans lequel R² représente un groupe octanoyle, décanoyle, dodécanoyle, myristoyle ou palmitoyle, et R³ représente un atome d'hydrogène.

8. Composé selon la revendication 1, dans lequel R³ représente un atome d'hydrogène ou un groupe acyle d'acide carboxylique aliphatique comportant de 6 à 25 atomes de carbone.

9. Composé selon la revendication 1, dans lequel R³ représente un atome d'hydrogène ou un groupe acyle d'acide carboxylique aliphatique comportant de 8 à 16 atomes de carbone.

10. Composé selon la revendication 2, dans lequel R³ représente un atome d'hydrogène, ou un groupe octanoyle, décanoyle, dodécanoyle, myristoyle ou palmitoyle.

11. Composé selon la revendication 1, dans lequel X représente un atome de fluor, un groupe méthoxy ou éthoxy.

12. Composé selon la revendication 1, dans lequel Y représente un groupe amino ou un groupe de formule R^{b}R^{c}N-O- dans laquelle R^{b} et R^{c} sont tels que définis dans la revendication 1.

13. Composé selon la revendication 12, dans lequel Y représente un groupe amino ou aminoxy.

14. Composé selon la revendication 12, dans lequel Y représente un groupe amino.

15. Composé selon la revendication 1, dans lequel Z représente un atome d'oxygène.

16. Composé selon la revendication 1, dans lequel :
R¹ représente un groupe méthyle ;
R² représente un atome d'hydrogène, ou un groupe acyle d'acide carboxylique aromatique comportant de 6 à 25 atomes de carbone ;
R³ représente un atome d'hydrogène ou un groupe acyle d'acide carboxylique aliphatique comportant de 6 à 25 atomes de carbone ;
X représente un atome d'halogène ou un groupe alkoxy comportant de 1 à 4 atomes de carbone ;
Y représente un groupe amino de formule R^{b}R^{c}N-O- dans laquelle R^{b} et R^{c} sont tels que définis ci-dessus ; et
Z représente un atome d'oxygène.

17. Composé selon la revendication 1, dans lequel :
R¹ représente un groupe méthyle ;
R² représente un atome d'hydrogène, ou un groupe acyle d'acide carboxylique aliphatique comportant de 8 à 16 atomes de carbone ;
R³ représente un atome d'hydrogène ou un groupe acyle d'acide carboxylique aliphatique comportant de 8 à 16 atomes de carbone ;
X représente un atome de fluor, un groupe méthoxy ou un groupe éthoxy ;
Y représente un groupe amino ou un groupe aminoxy ; et
Z représente un atome d'oxygène.

18. Composé selon la revendication 1, dans lequel :
R¹ représente un groupe méthyle ;
R² représente un atome d'hydrogène ou un groupe acyle d'acide carboxylique aliphatique comportant de 8 à 16 atomes de carbone ;
R³ représente un atome d'hydrogène ;
X représente un atome de fluor, un groupe méthoxy ou un groupe éthoxy ;
Y représente un groupe amino ou un groupe aminoxy ; et
Z représente un atome d'oxygène.

19. Composé selon la revendication 1, dans lequel :
R¹ représente un groupe méthyle ;
R² représente un groupe octanoyle, décanoyle, dodécanoyle, myristoyle ou palmitoyle ;
R³ représente un atome d'hydrogène, ou un groupe octanoyle, décanoyle, dodécanoyle, myristoyle ou palmitoyle ;
X représente un atome de fluor, un groupe méthoxy ou un groupe éthoxy ;
Y représente un groupe amino ; et
Z représente un atome d'oxygène.

20. Composé selon la revendication 1, dans lequel :
R¹ représente un groupe méthyle ;
R² représente un octanoyle, décanoyle, dodécanoyle, myristoyle ou palmitoyle ;
R³ représente un atome d'hydrogène ;
X représente un atome de fluor, un groupe méthoxy ou un groupe éthoxy ;
Y représente un groupe amino ; et
Z représente un atome d'oxygène.

21. Les composés suivants selon la revendication 1 :
l'acide 5-acétamido-4-guanidino-2,3,4,5,7-penta-désoxy-7-fluoro-D-glycéro-D-galacto-non-2-énopyranosoïque ;
l'acide 5-acétamido-4-guanidino-9-O-dodécanoyl-2,3,4,5,7-pentadésoxy-7-fluoro-D-glycéro-D-galacto-non-2-énopyranosoïque ;
l'acide 5-acétamido-4-guanidino-9-O-myristoyl-2,3,4,5,7-pentadésoxy-7-fluoro-D-glycéro-D-galacto-non-2-énopyranosoïque ;
l'acide 5-acétamido-4-guanidino-9-O-palmitoyl-2,3,4,5,7-pentadésoxy-7-fluoro-D-glycéro-D-galacto-non-2-énopyranosoique ;
l'acide 5-acétamido-4-guanidino-2,3,4,5,7-pentadésoxy-7-méthoxy-D-glycéro-D-galacto-non-2-énopyranosoïque ;
l'acide 5-acétamido-4-guanidino-9-O-myristoyl-2,3,4,5,7-pentadésoxy-7-méthoxy-D-glycéro-D-galacto-non-2-énopyranosoïque ;
l'acide 5-acétamido-4-guanidino-2,3,4,5,7-pentadésoxy-7-éthoxy-D-glycéro-D-galacto-non-2-énopyranosoïque ;
l'acide 5-acétamido-4-guanidino-9-O-octanoyl-2,3,4,5,7-pentadésoxy-7-éthoxy-D-glycéro-D-galacto-non-2-énopyranosoïque ;
l'acide 5-acétamido-4-guanidino-9-O-dodécanoyl-2,3,4,5,7-pentadésoxy-7-éthoxy-D-glycéro-D-galacto-non-2-énopyranosoïque ;
l'acide 5-acétamido-4-guanidino-9-O-myristoyl-2,3,4,5,7-pentadésoxy-7-éthoxy-D-glycéro-D-galacto-non-2-énopyranosoïque ;
l'acide 5-acétamido-4-guanidino-9-O-palmitoyl-2,3,4,5,7-pentadésoxy-7-éthoxy-D-glycéro-D-galacto-non-2-énopyranosoïque ;
l'acide 5-acétamido-4-guanidino-9-O-octanoyl-2,3,4,5,7-pentadésoxy-7-méthoxy-D-glycéro-D-galacto-non-2-énopyranosoïque ;
l'acide 5-acétamido-4-guanidino-9-O-dodécanoyl-2,3,4,5,7-pentadésoxy-7-méthoxy-D-glycéro-D-galacto-non-2-énopyranosoique ;
l'acide 5-acétamido-4-guanidino-9-O-dodécanoyl-2,3,4,5,7-pentadésoxy-7-méthoxy-D-glycéro-D-galacto-non-2-énopyranosoïque; et
l'acide 5-acétamido-4-guanidino-9-O-hexa-décanoyl-2,3,4,5,7-pentadésoxy-7-méthoxy-D-glycéro-D-galacto-non-2-énopyranosoïque ;
de même que leurs sels et leurs esters pharmaceutiquement acceptables.

22. Utilisation d'un composé de formule (I), ou d'un sel ou d'un ester pharmaceutiquement acceptable de celui-ci, tel que revendiqué dans l'une quelconque des revendications 1 à 21, pour la préparation d'un médicament destiné au traitement ou à la prévention chez un mammifère d'une infection provoquée par un virus véhiculant la sialidase.

23. Composition pharmaceutique, pour le traitement ou la prévention chez un mammifère d'infections dues à des virus véhiculant la sialidase, cette composition comprenant un composant inhibiteur de la sialidase en mélange avec un véhicule ou un diluant pharmaceutiquement acceptable, le composant inhibiteur de la sialidase comprenant au moins un composé de formule (I), ou un sel ou un ester de celui-ci, tel que revendiqué dans l'une quelconque des revendications 1 à 21.
